# EUROPÄISCHE PATENTSCHRIFT

(11) **EP 2 033 688 B1**
(45) Veröffentlichungstag und Bekanntmachung des Hinweises auf die Patenterteilung: **17.10.2012**
(21) Anmeldenummer: 08162309.2
(22) Anmeldetag: 13.08.2008
(51) Int. Cl.: A61Q 11/00, A61K 8/42, A61K 8/49, C07C 233/56, C07D 295/185, A23G 4/06, A23G 3/36

(54) **Oxalsäurederivate und deren Verwendung als physiologische Kühlwirkstoffe**
Oxalic acid derivatives and their use as physiological cooling agents
Dérivés d'acide oxalique et leur utilisation en tant que matières de refroidissement physiologiques

(30) Priorität: 20.08.2007 US 956726 P
(43) Veröffentlichungstag der Anmeldung: 11.03.2009
(73) Patentinhaber: Symrise AG, 37603 Holzminden (DE)
(72) Erfinder: Surburg, Horst, 37603 Holzminden (DE); Looft, Jan, 37603 Holzminden (DE); Oertling, Heiko, 37603 Holzminden (DE); Vössing, Tobias, 37688 Beverungen (DE)
(74) Vertreter: Eisenführ, Speiser & Partner

(56) Entgegenhaltungen:
- EP-A2- 1 493 336
- BELOKON' ET AL: "Enantioselective and diastereoselective syntheses of cyanohydrin carbonates" TETRAHEDRON, ELSEVIER SCIENCE PUBLISHERS, AMSTERDAM, NL, Bd. 63, Nr. 39, 16. August 2007 (2007-08-16), Seiten 9724-9740, XP022201064 ISSN: 0040-4020

## Beschreibung

Die Erfindung betrifft bestimmte Oxalsäurederivate und deren Mischungen, die eine physiologische Kühlwirkung auf der Haut und/oder einer Schleimhaut bewirken können. Sie betrifft auch Mischungen und Zubereitungen, die die Oxalsäurederivate in ausreichender Menge enthalten, so dass eine Kühlwirkung auf der Haut und/oder Schleimhäuten erzeugt wird. Sie betrifft außerdem die Verwendung der genannten Verbindungen als Kühlsubstanz bzw. zur Herstellung eines Arzneimittels sowie ein Verfahren zum Erreichen einer physiologischen Kühlwirkung auf der Haut und/oder Schleimhäuten.

Physiologische Kühlwirkstoffe werden regelmäßig eingesetzt, um einen kühlen sensorischen Eindruck auf der Haut bzw. Schleimhaut, zum Beispiel auf der Schleimhaut im Mund-, Nasen- und/oder Rachenraum hervorzurufen, wobei allerdings tatsächlich keine physikalische Abkühlung wie zum Beispiel bei der Verdunstung von Lösungsmitteln stattfindet. Als physiologische Kühlwirkstoffe können sowohl Einzelkomponenten als auch Mischungen verwendet werden.

Der bekannteste Kühlwirkstoff ist L-Menthol, der aber einige Nachteile besitzt, z. B. starker Geruchseindruck, eine hohe Flüchtigkeit und in höheren Konzentrationen einen bitteren und/oder scharfen Eigengeschmack. Der Einsatz von L-Menthol kann somit in bestimmten Aromakompositionen, insbesondere solchen, die nicht in Richtung (Pfeffer)Minz-Aroma gehen, unerwünscht sein.

Es wurden schon früher Untersuchungen durchgeführt, die auf starke Kühlwirkstoffe ohne Aromaeffekt hinzielen. So wurden z.B. Milchsäureester von Menthol(en) gemäß DE 2 608 226 und gemischte Carbonate mit Menthol(en) und Polyolen gemäß DE 4 226 043 und Menthonketale gemäß EP 0 507 190 beschrieben.

Menthylmonoester von Disäuren nach US 5,725,865 und US 5,843,466 sind zwar interessante natürlich vorkommende Alternativen, können aber in sensorischen Tests nicht die Stärke der vorher beschriebenen Kühlwirkstoffe erreichen.

In J. Soc. Cosmet. Chem. 1978, 29, 185-200 wurden die Ergebnisse einer Studie an ca. 1200 Verbindungen präsentiert, bei der die Verbindungen L-Menthancarbonsäure-N-ethylamid ("WS3") und insbesondere *N*^{α}-(L-Menthancarbonyl)-glycinethylester ("WS5") als die stärksten Kühlwirkstoffe gefunden wurden. Letzterer hat bei starker Wirkung aber den Nachteil, hydrolyseempfindlich zu sein und dabei die entsprechende freie Säure *N*^{α}-(L-Menthancarbonyl)-glycin zu bilden, die selbst nur noch eine sehr schwache Kühlwirkung zeigt. Trotz der beschriebenen ausführlichen Untersuchungen ist eine systematische Vorhersage zu den Eigenschaften von potentiellen Kühlwirkstoffen, insbesondere zu deren Bitterkeit und/oder deren anderen trigeminalen Effekten nicht möglich und auch nicht beschrieben. So sind auch viele unter die Klasse der Menthancarbonsäureamide fallende Moleküle zwar stark kühlend, zeigen jedoch häufig gleichzeitig ausgeprägt bittere Noten (z.B. die Menthancarbonsäure-N-(alkyloxyalkyl)amide nach JP 2004059474) oder sind zusätzlich stark reizend (WS5: N-[[5-Methyl-2-(1-methylethyl)cyclohexyl]carbonyl]glycinethylester, US 2005/0222256).

*N*^{α}-(Menthancarbonyl)alkyloxyalkylamide wurden in JP 2004059474 beschrieben. Diese haben bei starker Kühlwirkung und hoher Hydrolysestabilität jedoch den Nachteil, stark bitter zu sein, und sie sind somit in Nahrungsmitteln und auch in der Gesichtspflege dienenden kosmetischen Produkten nicht einsetzbar.

Des Weiteren sind Menthylglyoxylate und ihre Hydrate in JP 2005343795 als Kühlsubstanzen beschrieben worden.

In EP 1 493 336 werden Monomenthylesterderivate von Bernsteinsäure und die entsprechenden höheren Homologen als Kühlsubstanzen beschrieben. Bei einer Verkostung im direkten Vergleich mit Verbindungen der Formel (I) durch ein Expertenpanel wurde deutlich, dass die beschriebenen Oxamate den Monomenthylestern sensorisch überlegen sind, da sie keinen bitteren, scharfen Beigeschmack bei vergleichbarer Kühlwirkung besitzen.

(t) - Mentyloxyamid wurde in Tetrahedron 2007, 63, 9724-9740 vorberchrieben.

Es war die primäre Aufgabe der vorliegenden Erfindung, neue Verbindungen bzw. Mischungen von Verbindungen anzugeben, die eine starke physiologische Kühlwirkung besitzen und dabei in Nahrungs- und/oder Genussmitteln und/oder Mundpflegeprodukten und/oder (oralen) pharmazeutischen Zubereitungen und/oder kosmetischen Zubereitungen als Kühlsubstanzen (Kühlwirkstoffe) eingesetzt werden können. Die anzugebenden Verbindungen bzw. Mischungen von Verbindungen sollten vorzugsweise einen möglichst schwachen Eigengeschmack zeigen, insbesondere wenig oder gar nicht bitter schmecken sowie möglichst nicht reizend sein.

Diese primäre Aufgabe wird erfindungsgemäß gelöst durch (a) eine Verbindung der Formel (I) sowie durch (b) eine Mischung umfassend eine, zwei oder mehr Verbindungen der Formel (I) sowie durch (c) eine Mischung bestehend aus zwei oder mehr Verbindungen der Formel (I) 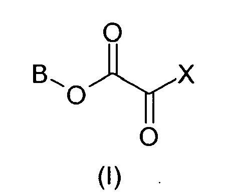 wobei jeweils gilt:
B bedeutet einen Kohlenwasserstoffrest ausgewählt aus der folgenden Gruppe: 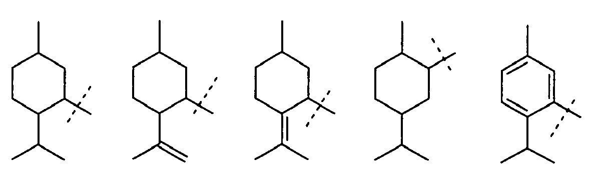 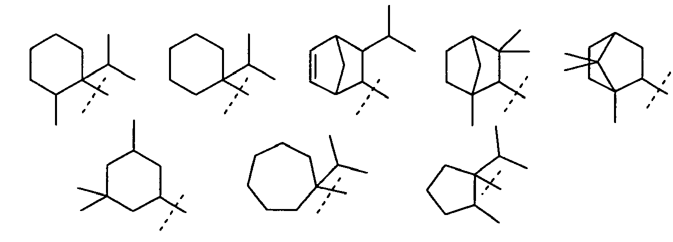 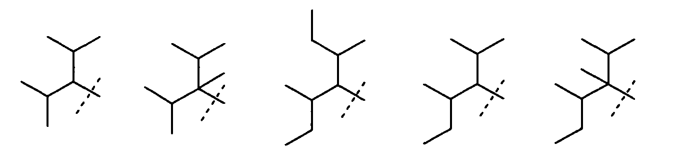 worin die gestrichelte Linie die Bindung markiert, welche den Kohlenwasserstoffrest B mit dem in Formel (I) benachbarten Sauerstoffatom verknüpft,
X bedeutet NR¹R² oder SR³,
   wobei jeweils unabhängig voneinander
   in NR¹R² die Reste R¹ und R² ausgewählt sind und
   in SR³ der Rest R³ ausgewählt ist
   aus der Gruppe bestehend aus:
   Wasserstoff
   und
   organischer Rest mit
   1 bis 12 C-Atomen der vorzugsweise
   ausgewählt ist aus der Gruppe bestehend aus substituiertem oder nicht substituiertem Alkyl, Heteroalkyl, Cycloalkyl, Heterocycloalkyl, Cycloalkylalkyl, Alkenyl, Cycloalkenyl, Cycloalkenylalkyl, Alkinyl, Cycloalkylalkinyl, Aryl, Heteroaryl, Arylalkyl, Cycloalkylaryl, Cycloalkenylaryl, Cycloalkylheteroaryl, Heterocycloalkylaryl, Heterocycloalkenylaryl, Heterocycloalkenylheteroaryl und Heteroarylalkyl,
   wobei gegebenenfalls in NR¹R² die Reste R¹ und R² unter Fortfall je eines Wasserstoffatomes und unter Ausbildung eines Ringes kovalent miteinander verbunden sind,
   mit der Maßgabe, dass im obigen Falle (a) die Verbindung der Formel (I) und im Falle (b) zumindest eine Verbindung der Formel (I) nicht ausgewählt ist aus der Gruppe bestehend aus: 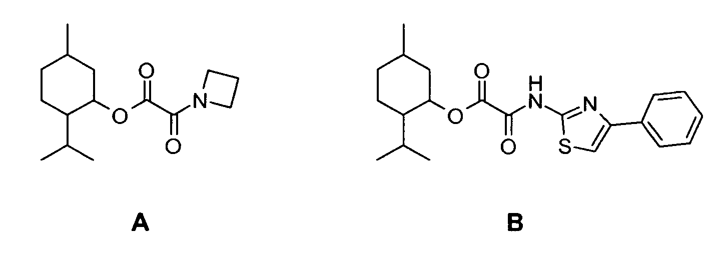 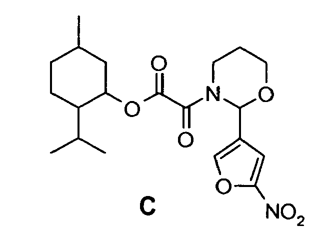
und D (+) -Mentyloxamid

Vorzugsweise sind die Verbindungen A, B und C in einer erfindungsgemäßen Mischung nicht enthalten.

Eine bevorzugte Ausführungsform der vorliegenden Erfindung ist eine (a) Verbindung der Formel (I) oder (b) Mischung umfassend eine, zwei oder mehr Verbindungen der Formel (I) oder (c) Mischung bestehend aus zwei oder mehr Verbindungen der Formel (I) wie vorstehend beschrieben, wobei im Falle (a) die Verbindung bzw. im Falle (b) oder (c) zumindest eine der Verbindungen in der Mischung ausgewählt ist aus der Gruppe bestehend aus Verbindungen der Formel (I), wobei gilt:
X bedeutet NR¹R² oder SR³,
   wobei jeweils unabhängig voneinander
   in NR¹R² die Reste R¹ und R² ausgewählt sind und
   in SR³ der Rest R³ ausgewählt ist
   aus der Gruppe bestehend aus:
   Wasserstoff
   und
   organischer Rest mit
   1 bis 12 C-Atomen und
   gegebenenfalls bis zu 3 Heteroatomen, die jeweils unabhängig voneinander ausgewählt sind aus der Gruppe bestehend aus N, S und O, wobei gegebenenfalls vorhandene Heteroatome aus der Gruppe bestehend aus O und S keine kovalente Bindung zu anderen Heteroatomen aus dieser Gruppe besitzen,
wobei gegebenenfalls eine Anzahl von an C gebundenen Wasserstoffatomen durch Fluor ersetzt ist,
wobei der organische Rest jeweils inklusive aller gegebenenfalls vorhandenen Substituenten nicht mehr Heteroatome aus der Gruppe bestehend aus N, S und O als C-Atome enthält und ausgewählt ist aus der Gruppe bestehend aus substituiertem oder nicht substituiertem
C₁-C₁₀-Alkyl, C₁-C₁₀-Heteroalkyl, C₂-C₁₀-Heterocycloalkyl, C₃-C₁₀-Cycloalkyl, C₄-C₁₀-Cycloalkylalkyl, C₂-C₁₀-Alkenyl, C₃-C₁₀-Cycloalkenyl, C₄-C₁₀-Cycloalkenylalkyl, C₂-C₁₀-Alkinyl, C₅-C₁₀-Cycloalkylalkinyl, C₃-C₁₀-Aryl, C₂-C₁₀-Heteroaryl, C₄-C₁₀-Arylalkyl, C₈-C₁₀-Cycloalkylaryl, C₈-C₁₀-Cycloalkenylaryl, C₅-C₁₀-Cycloalkylheteroaryl, C₈-C₁₀-Heterocycloalkylaryl, C₈-C₁₀-Heterocycloalkenylaryl, C₈-C₁₀-Heterocycloalkenylheteroaryl und C₃-C₁₀-Heteroarylalkyl,
und bevorzugt ausgewählt ist aus der Gruppe bestehend aus substituiertem oder nicht substituiertem
C₁-C₈-Alkyl, C₂-C₈-Heteroalkyl, C₁-C₈-Heterocycloalkyl, C₃-C₈-Cycloalkyl, C₄-C₈-Cycloalkylalkyl, C₂-C₈-Alkenyl, C₃-C₈-Cycloalkenyl, C₄-C₈-Cycloalkenylalkyl, C₃-C₆-Aryl, C₂-C₆-Heteroaryl, C₄-C₈-Arylalkyl,
wobei gegebenenfalls in NR¹R² die Reste R¹ und R² unter Fortfall je eines Wasserstoffatomes und unter Ausbildung eines Ringes kovalent miteinander verbunden sind.

Vorzugsweise ist im Falle (b) oder (c) jede in der Mischung vorhandene Verbindung der Formel (I) ausgewählt aus der besagten Gruppe.

Eine besonders bevorzugte Ausführungsform der vorliegenden Erfindung ist eine (a) Verbindung der Formel (I) oder (b) Mischung umfassend eine, zwei oder mehr Verbindungen der Formel (I) oder (c) Mischung bestehend aus zwei oder mehr Verbindungen der Formel (I) nach einer der vorstehend beschriebenen Ausführungsformen, wobei im Falle (a) die Verbindung bzw. im Falle (b) oder (c) zumindest eine der Verbindungen in der Mischung ausgewählt ist aus der Gruppe bestehend aus Verbindungen der Formel (I), wobei gilt:
X bedeutet NR¹R²,
   wobei jeweils unabhängig voneinander
   in NR¹R² die Reste R¹ und R² ausgewählt sind aus der Gruppe bestehend aus:
   Wasserstoff
   und
   organischer Rest mit
   1 bis 12 C-Atomen und
   gegebenenfalls bis zu 3 Heteroatomen, die jeweils unabhängig voneinander ausgewählt sind aus der Gruppe bestehend aus N, S und O, wobei gegebenenfalls vorhandene Heteroatome aus der Gruppe bestehend aus O und S keine kovalente Bindung zu anderen Heteroatomen aus dieser Gruppe besitzen,
wobei gegebenenfalls eine Anzahl von an C gebundenen Wasserstoffatomen durch Fluor ersetzt ist,
wobei der organische Rest jeweils inklusive aller gegebenenfalls vorhandenen Substituenten nicht mehr Heteroatome aus der Gruppe bestehend aus N, S und O als C-Atome enthält und ausgewählt ist aus der Gruppe bestehend aus substituiertem oder nicht substituiertem
C₁-C₄-Alkyl, C₂-C₅-Heteroalkyl, C₃-C₆-Cycloalkyl, C₃-C₆-Aryl, C₂-C₆-Heteroaryl, C₄-C₈-Arylalkyl
und bevorzugt ausgewählt ist aus der Gruppe bestehend aus

Methyl, Ethyl, n-Propyl, n-Butyl, iso-Butyl, sec.-Butyl, tert.-Butyl, 2-Hydroxyethyl, 3-Hydroxypropyl, Methoxymethyl, 2-Methoxyethyl, 3-Methoxypropyl, 2-Ethoxyethyl, 3-Ethoxypropyl, Cyclopropyl, 2,2-Dimethylcyclopropyl, Cyclopentyl, Cyclohexyl, Phenyl, p-Toluyl, p-Anisyl, 2-Furfuryl, 2-Pyrrolidyl, 2-Pyridinyl und Benzyl,
wobei in NR¹R² die Reste R¹ und R² unter Fortfall je eines Wasserstoffatomes und unter Ausbildung eines Ringes kovalent miteinander verbunden sind.

Die ausgeprägteste Kühlwirkung zeigten erfindungsgemäße Verbindungen, in denen X eine Gruppe NHR² bedeutet, deshalb ist eine bevorzugte Ausführungsform der vorliegenden Erfindung eine (a) Verbindung der Formel (I) oder (b) Mischung umfassend eine, zwei oder mehr Verbindungen der Formel (I) oder (c) Mischung bestehend aus zwei oder mehr Verbindungen der Formel (I) nach einem der vorhergehenden Ansprüche,
wobei im Falle (a) die Verbindung bzw. im Falle (b) oder (c) zumindest eine der Verbindungen in der Mischung ausgewählt ist aus der Gruppe bestehend aus Verbindungen der Formel (I), wobei gilt:
X bedeutet NHR¹
wobei R¹ die oben angegebene Bedeutung hat.

Vorzugsweise ist im Falle (b) oder (c) jede in der Mischung vorhandene Verbindung der Formel (I) ausgewählt aus der besagten Gruppe.

Eine wegen der hervorragenden Kühlwirkung bevorzugte Ausführungsform der vorliegenden Erfindung ist eine (a) Verbindung der Formel (I) oder (b) Mischung umfassend eine, zwei oder mehr Verbindungen der Formel (I) oder (c) Mischung bestehend aus zwei oder mehr Verbindungen der Formel (I) nach einer der vorstehend beschriebenen Ausführungsformen, wobei im Falle (a) die Verbindung bzw. im Falle (b) oder (c) zumindest eine der Verbindungen in der Mischung ausgewählt ist aus der Gruppe bestehend aus Verbindungen der Formel (I), wobei gilt:
B bedeutet einen Kohlenwasserstoffrest ausgewählt aus der Gruppe bestehend aus: 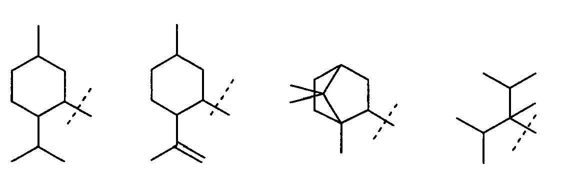
wobei es besonders bevorzugt ist, wenn B einen Kohlenwasserstoffrest ausgewählt aus der folgenden Gruppe bedeutet:
L-Menthyl, D-Menthyl oder rac-Menthyl.

Vorzugsweise ist im Falle (b) oder (c) jede in der Mischung vorhandene Verbindung der Formel (I) ausgewählt aus der besagten Gruppe.

Eine weiterhin bevorzugte Ausführungsform der vorliegenden Erfindung ist eine (a) Verbindung der Formel (I) oder (b) Mischung umfassend eine, zwei oder mehr Verbindungen der Formel (I) oder (c) Mischung bestehend aus zwei oder mehr Verbindungen der Formel (I) nach einer der vorstehend beschriebenen Ausführungsformen, wobei im Falle (a) die Verbindung bzw. im Falle (b) oder (c) zumindest eine der Verbindungen in der Mischung ausgewählt ist aus der Gruppe bestehend aus Verbindungen der Formel (I), wobei einzelne, vorzugsweise jedoch sämtliche gegebenenfalls vorhandenen Substituenten der gegebenenfalls vorhandenen Reste R¹, R² und R³ unabhängig voneinander ausgewählt sind aus der Gruppe bestehend aus:

C₁-C₅-Alkyl, C₃-C₈-Cycloalkyl, C₂-C₄-Alkenyl, C₂-C₄-Alkinyl, Propinyl, C₁-C₄-Perfluoralkyl, Hydroxy, C₁-C₈-Alkoxy, C₃-C₈-Cycloalkoxy, C₁-C₄-Acyl, C₁-C₄-Alkoxy-carbonyl, C₁-C₄-Acyloxy oder C₂-C₇-Heteroalkyl
und bevorzugt ausgewählt sind aus der Gruppe bestehend aus:
Methyl, Ethyl, n-Propyl, iso-Propyl, n-Butyl, iso- Butyl, tert.- Butyl, Cyclopropyl, Cyclopentyl, Cyclohexyl, Cyclooctyl, Ethenyl, Propenyl, Ethinyl, Propinyl, Trifluormethyl, Methoxy, Ethoxy, iso-Propoxy, n-Butoxy, iso-Butoxy, tert.-Butoxy, C₃-Cycloalkoxy, C₅-Cycloalkoxy, C₆-Cycloalkoxy, C₈-Cycloalkoxy, -[-O-CH₂-CH₂-]ᵥ-Q oder -[-O-CH₂-CHMe-]ᵥ-Q, wobei Q = OH oder CH₃ ist und wobei v = 1 oder 2 bedeuten kann, Acetyl, CO₂Me, CO₂Et, CO₂iso-Pr, CO₂tert.-Bu, Acetyloxy.

Vorzugsweise ist im Falle (b) oder (c) jede in der Mischung vorhandene Verbindung der Formel (I) ausgewählt aus der besagten Gruppe.

Eine weiterhin bevorzugte Ausführungsform der vorliegenden Erfindung ist eine (a) Verbindung der Formel (I) oder (b) Mischung umfassend eine, zwei oder mehr Verbindungen der Formel (I) oder (c) Mischung bestehend aus zwei oder mehr Verbindungen der Formel (I) nach einer der vorstehend beschriebenen Ausführungsformen, wobei im Falle (a) die Verbindung bzw. im Falle (b) oder (c) zumindest eine der Verbindungen in der Mischung ausgewählt ist aus der Gruppe bestehend aus Verbindungen der Formel (I), wobei die Reste R¹ und R² der Gruppe NR¹R² unter Fortfall je eines Wasserstoffatoms kovalent miteinander verbunden sind und einen maximal 7-gliedrigen Ring bilden, und vorzugweise eine Gruppe NR¹R² bilden, die ausgewählt ist aus der Gruppe der 6 oder 7-gliedrigen Ringe und besonders bevorzugt ausgesucht ist aus der Gruppe bestehend aus Piperidinyl und Morpholinyl.

Weniger bevorzugt ist die Auswahl von Vierringen, insbesondere, wenn sie unsubstituiert sind, und 5-Ringen, insbesondere, wenn sie (i) genau ein Stickstoffatom und genau ein Sauerstoffatom oder (ii) genau ein Stickstoffatom und genau ein Schwefelatom im Ring enthalten.

Eine günstige Kombination von guter physiologischer Kühlwirkung, schwachem Eigengeschmack sowie geringer Reizwirkung besitzt eine (a) Verbindung der Formel (I) oder (b) Mischung umfassend eine, zwei oder mehr Verbindungen der Formel (I) oder (c) Mischung bestehend aus zwei oder mehr Verbindungen der Formel (I) nach einer der vorstehend beschriebenen Ausführungsformen, wobei im Falle (a) die Verbindung bzw. im Falle (b) oder (c) zumindest eine der Verbindungen in der Mischung ausgewählt ist aus der Gruppe bestehend aus Verbindungen der Formel (I), wobei gilt:
B bedeutet einen Kohlenwasserstoffrest ausgewählt aus der Gruppe bestehend aus:
   L-Menthyl, D-Menthyl oder rac-Menthyl
   X bedeutet NR¹R²
wobei jeweils unabhängig voneinander
die Reste R¹ und R² ausgewählt sind aus der Gruppe bestehend aus:
Wasserstoff
und
organischer Rest mit
1 bis 12 C-Atomen und
gegebenenfalls bis zu 3 Heteroatomen, die jeweils unabhängig voneinander ausgewählt sind aus der Gruppe bestehend aus N, S und O, wobei gegebenenfalls vorhandene Heteroatome aus der Gruppe bestehend aus O und S keine kovalente Bindung zu anderen Heteroatomen aus dieser Gruppe besitzen,
wobei gegebenenfalls eine Anzahl von an C gebundenen Wasserstoffatomen durch Fluor ersetzt ist,
wobei der organische Rest jeweils inklusive aller gegebenenfalls vorhandenen Substituenten nicht mehr Heteroatome aus der Gruppe bestehend aus N, S und O als C-Atome enthält und ausgewählt ist aus der Gruppe bestehend aus substituiertem oder nicht substituiertem
C₁-C₄-Alkyl, C₂-C₅-Heteroalkyl, C₃-C₆-Cycloalkyl, C₃-C₆-Aryl, C₂-C₆-Heteroaryl, C₄-C₈-Arylalkyl
wobei in NR¹R² gegebenenfalls die Reste R¹ und R² auch unter Fortfall je eines Wasserstoffatomes kovalent miteinander verbunden sind, wobei der dadurch ausgebildete Ring maximal 7-gliedrig ist,
wobei die gegebenenfalls vorhandenen Substituenten der gegebenenfalls vorhandenen Reste R¹, R² und R³ unabhängig voneinander ausgewählt werden aus der Gruppe bestehend aus:
Methyl, Ethyl, n-Propyl, iso-Propyl, n-Butyl, iso- Butyl, tert.- Butyl, Cyclopropyl, Cyclopentyl, Cyclohexyl, Cyclooctyl, Ethenyl, Propenyl, Ethinyl, Propinyl, Trifluormethyl, Methoxy, Ethoxy, iso-Propoxy, n-Butoxy, iso-Butoxy, tert.-Butoxy, C₃-Cycloalkoxy, C₅-Cycloalkoxy, C₆-Cycloalkoxy, C₈-Cycloalkoxy, -[-O-CH₂-CH₂-]ᵥ-Q oder -[-O-CH₂-CHMe-]ᵥ-Q, wobei Q = OH oder CH₃ ist und wobei v = 1 oder 2 bedeuten kann, Acetyl, CO₂Me, CO₂Et, CO₂iso-Pr, CO₂tert.-Bu, Acetyloxy.
Eine besonders günstige Kombination von guter physiologischer Kühlwirkung, eher geringem schwachen Eigengeschmack sowie geringer Reizwirkung besitzt eine (a) Verbindung der Formel (I) oder (b) Mischung umfassend eine, zwei oder mehr Verbindungen der Formel (I) oder (c) Mischung bestehend aus zwei oder mehr Verbindungen der Formel (I) nach einer der vorstehend beschriebenen Ausführungsformen, wobei im Falle (a) die Verbindung bzw. im Falle (b) oder (c) zumindest eine der Verbindungen in der Mischung ausgewählt ist aus der Gruppe bestehend aus
Menthyloxamat
Menthyl-N-methyloxamat
Menthyl-N,N-dimethyloxamat
Menthyl-N-ethyloxamat
Menthyl-N,N-diethyloxamat
Menthyl-N-propyloxamat
Menthyl-N,N-dipropyloxamat
Menthyl-N-isopropyloxamat
Menthyl-N,N-diisopropyloxamat
Menthyl-N-cyclopropyloxamat
Menthyl-N-butyloxamat
Morpholin-4-yl-oxo-essigsäure-(1R,2S,5R)-2-isopropyl-5-methyl-cyclohexylester
Menthyl-N-(2-methoxyethyl)-oxamat
Menthyl-N-(3-methoxypropyl)-oxamat
Menthyl-N-(2-hydroxyethyl)-oxamat
Menthyl-N-(3-hydroxypropyl)-oxamat.
Vorzugsweise ist im Falle (b) oder (c) jede in der Mischung vorhandene Verbindung der Formel (I) ausgewählt aus der besagten Gruppe.

Ebenfalls ganz besonders bevorzugt ist eine (a) Verbindung der Formel (I) oder (b) Mischung umfassend eine, zwei oder mehr Verbindungen der Formel (I) oder (c) Mischung bestehend aus zwei oder mehr Verbindungen der Formel (I) nach einer der vorstehend beschriebenen Ausführungsformen, wobei im Falle (a) die Verbindung bzw. im Falle (b) oder (c) zumindest eine der Verbindungen in der Mischung ausgewählt ist aus der Gruppe bestehend aus
L-Menthyl-N-methyloxamat,
D-Menthyl-N-methyloxamat,
rac-Menthyl-N-methyloxamat,
L-Menthyl-N-ethyloxamat,
D-Menthyl-N-ethyloxamat,
rac-Menthyl-N-ethyloxamat.

Vorzugsweise ist im Falle (b) oder (c) jede in der Mischung vorhandene Verbindung der Formel (I) ausgewählt aus der besagten Gruppe.

L-Menthyloxamate sind am meisten bevorzugt, da sie die dieser Erfindung zugrunde liegende Aufgabe am besten lösen.

Einzelne Verbindungen der Formel (I) und Ihre Synthese sind in der Literatur beschrieben worden, nicht jedoch eine physiologische Kühlwirkung oder entsprechende Verwendungen oder Verfahren. Als Beispiel für eine Synthese von Oxamaten wird auf DE 1 002 318 und DE 2 413 966 verwiesen.

Folgende Verbindungen A, B und C, die unter die oben definierte Formel (I) fallen, sind bereits beschrieben worden, und zwar **A** in EP 0 350 693, Verbindung **B** in DE 3027 527 A1 und US 4,407,810 , Verbindung **C** in Liebigs Ann. Chem. 765, 1972, 78 und (+) - Mentyloxamid in Tetrahedron 2007, 63 9724-9740. Eine Verbindung **E,** welche strukturell noch entfernt verwandt ist, ist in WO 01/70687 beschrieben.

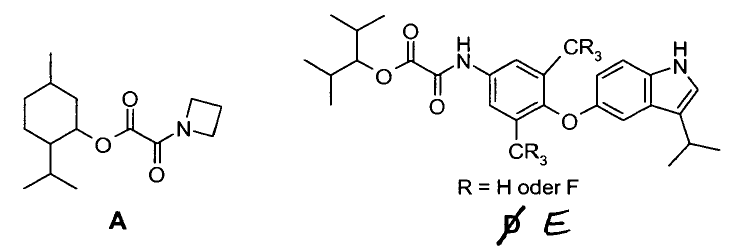

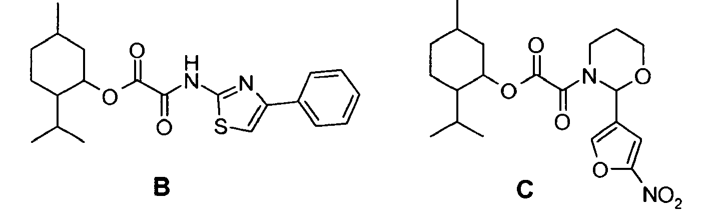

Ebenfalls strukturell noch verwandte Verbindungen beschreibt die Offenlegungsschrift DE 2 413 966, wo Oxamidsäure-Derivate der Formel ANHCOCO₂R⁵ beschrieben werden, worin R⁵ eine Gruppe wie Niedrigalkyl, Cycloalkyl, Aralkyl etc. und insbesondere auch Cyclohexyl bedeutet und die Reste A Pyridyl, Pyrazinyl, Pyrimidyl oder Phenyl sind.

Die Verbindungen A, B, C und D sind als solche nicht Gegenstand der vorliegenden Erfindung, ebenso wenig wie Mischungen, die lediglich eine der Verbindungen A, B, C oder D, jedoch keine weiteren Verbindungen der Formel (I) mit den oben angegebenen Bedeutungen von B und X umfassen. Insgesamt sind die in EP 0 350 693, DE 3027 527 A1, US 4,407,810, Liebigs Ann. Chem. 765, 1972, 78, Tetrahedron 2007, 63, 9724-9740 offenbarten Verbindungen der Formel (I), und die in DE 2 413 966 und WO 01/70687 offenbarten strukturell noch verwandten Verbindungen (die dort offenbarten Mischungen, welche diese Verbindungen umfassen, sowie die dort für diese Verbindungen offenbarten Herstellungsvorschriften nicht Gegenstand der Erfindung bzw. für die Zwecke der vorliegenden Erfindung nicht bevorzugt. Insbesondere sind Verbindungen, in denen -NR¹R² substiutiertes oder nicht substituiertes Azetidin oder NHR¹ bedeutet, wobei R¹ substituiertes 2-Thiazolyl ist, nicht bevorzugt. Oxamidsäure-Derivate der Formel ANHCOCO₂R⁵ gemäß DE 2 413 966 mit R⁵ = Cyclohexyl sind ebenso wenig Gegenstand der vorliegenden Erfindung wie die entsprechenden Verbindungen in denen R⁵ nicht substituiertes Cycloalkyl ist, gleiches gilt für Mischungen, die lediglich solche Verbindungen, jedoch keine Verbindungen der Formel (I) mit den oben angegebenen erfindungsgemäßen Bedeutungen von B und X umfassen.

Die Erfindung basiert auf der überraschenden Erkenntnis, dass die erfindungsgemäßen Verbindungen, Oxalsäurederivate der Formeln (I) sowie deren Mischungen ein starkes und langanhaltendes Gefühl der Kälte auf der Haut oder Schleimhaut verursachen, insbesondere auf den Schleimhäuten des Mund-, Nasen- und Rachenraumes. Dabei zeigen die besagten Verbindungen keine anderen trigeminalen Effekte wie Schärfe, Tingling oder Betäubung und sind nicht bitter. Gleichzeitig sind die erfindungsgemäßen Verbindungen im Rahmen der üblichen Formulierungen und Zubereitungsbedingungen im Bereich von pH 1 bis pH 12, insbesondere im Bereich von pH 4 bis pH 9, bezogen auf wasserhaltige Zubereitungen, hydrolysestabil, so dass die erfindungsgemäßen Verbindungen und Mischungen in Zubereitungen lange haltbar sind, so dass auch die jeweilige Zubereitung selbst lange haltbar ist.

Die Erfindung betrifft auch eine Mischung (b) wie vorstehend beschrieben, bestehend aus oder umfassend
(1) eine, zwei oder mehr Verbindungen der Formel (I) sowie
(2) einen oder mehrere weitere (d.h. nicht unter die Formel (I) fallende) Stoffe mit physiologischer Kühlwirkung, wobei der weitere Stoff bzw. einer, mehrere oder sämtliche der weiteren Stoffe (i) einen geschmacklichen Effekt verursachen oder (ii) keinen geschmacklichen Effekt verursachen, und/oder
(3) einen oder mehrere Aromastoffe ohne physiologische Kühlwirkung und/oder
(4) einen oder mehrere trigeminal oder mundwässernd wirksame Stoffe ohne physiologische Kühlwirkung.

Besonders bevorzugt ist eine erfindungsgemäße Mischung, umfassend als Bestandteil (2) einen oder mehrere weitere Stoffe mit physiologischer Kühlwirkung, wobei diese keinen geschmacklichen Effekt und keine Aromawirkung verursachen, sondern lediglich eine Kühlwirkung (im Wesentlichen) ohne weiteren sensorischen Effekt. Hierdurch wird vermieden, dass das Aromaprofil der erfindungsgemäßen Mischung beispielsweise in Richtung "Minze" (Pfefferminze) gebunden wird.

Ganz besonders bevorzugt ist eine erfindungsgemäße Mischung umfassend als Bestandteil (3) einen oder mehrere Aromastoffe ohne physiologische Kühlwirkung und/oder als Bestandteil (4) eine oder mehrere Verbindungen, der oder die unabhängig voneinander oder gemeinsam zusätzlich einen geschmacksmodulierenden Effekt und/oder einen trigiminalen und/oder einen mundwässernden Reiz verursachen, wobei der trigiminale Reiz bevorzugt keine physiologische Kühlwirkung darstellt. Insbesondere besitzen solche erfindungsgemäße Mischungen, die die letztgenannten Bestandteile (3) und (4) gleichzeitig enthalten eine angenehme Kühlwirkung und ein ausgeglichenes sensorisches Profil bei gleichzeitig hohem Impact, d.h. hohem geschmacklichen Ersteindruck.

Der eine bzw. die mehreren weiteren Stoffe mit physiologischer Kühlwirkung, die als Bestandteil (2) in einer erfindungsgemäßen Mischung eingesetzt werden können, werden dabei vorzugsweise ausgewählt aus der folgenden Liste: Menthol und Mentholderivate (z.B. L-Menthol, D-Menthol, racemisches Menthol, Isomenthol, Neoisomenthol, Neomenthol) Menthylether (z.B. (I-Menthoxy)-1,2-propandiol, (I-Menthoxy)-2-methyl-1,2-propandiol, I-Menthyl-methylether), Menthylester (z.B. Menthylformiat, Menthylacetat, Menthylisobutyrat, Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat oder deren Mischungen), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), Menthancarbonsäureamide (dabei vorzugsweise Menthancarbonsäure-N-ethylamid [WS3], N^{α}-(Menthancarbonyl)glycinethylester [WS5] oder Menthancarbonsäure-N-(4-methoxyphenyl)amid [WS12], wie in US 4,150,052 beschrieben, Menthancarbonsäure-N-(4-cyanophenyl)amid oder Menthancarbonsäure-N-(4-cyanomethylphenyl)amid wie in WO 2005/049553 beschrieben, Menthancarbonsäure-N-(alkoxyalkyl)amide), Menthon und Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid [WS23]), Isopulegol oder seine Ester (I-(-)-Isopulegol, I-(-)-Isopulegolacetat), Menthanderivate (z.B. p-Menthan-3,8-diol), Cubebol oder synthetische oder natürliche Mischungen, enthaltend Cubebol, Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, wie in WO 2004/026840 beschrieben).

Der oder die mehreren weiteren Stoffe mit physiologischer Kühlwirkung, die als Bestandteil (2) einer erfindungsgemäßen Mischung eingesetzt werden können, sind insbesondere vorzugsweise Stoffe, welche zumindest im Wesentlichen eine physiologische Kühlwirkung verursachen, ohne gleichzeitig eine geschmackliche Wirkung zu verursachen. Solche bevorzugten Stoffe sind: Menthylether (z.B. (I-Menthoxy)-1,2-propandiol, (I-Menthoxy)-2-methyl-1,2-propandiol), polarere Menthylester (z.B. Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), nicht erfindungsgemäße Menthancarbonsäureamide (z.B. Menthancarbonsäure-N-ethylamid [WS3], N^{α}-(Menthancarbonyl)glycinethylester [WS5], Menthancarbonsäure-N-(4-cyanophenyl)amid, Menthancarbonsäure-N-(alkoxyalkyl)amide), Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid), Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, die in WO 2004/026840 beschrieben sind).

Als Aromastoffe eignen sich sowohl komplexe natürliche Rohstoffe wie aus Pflanzen gewonnene Extrakte und etherische Öle, bzw. daraus gewonnene Fraktionen und einheitliche Stoffe, als auch einheitliche synthetisch oder biotechnologisch gewonnene Aromastoffe.
Beispiele für natürliche Rohstoffe sind z.B.:
Pfefferminzöle, Krauseminzöle, Mentha-Arvensis-Öle, Anisöle, Nelkenöle, Citrusöle, Zimtrindenöle, Wintergrünöle, Cassiaöle, Davanaöle, Fichtennadelöle, Eucalyptusöle, Fenchelöle, Galbanumöle, Ingweröle, Kamillenöle, Kümmelöle, Rosenöle, Geraniumöle, Salbeiöle, Scharfgarbenöle, Sternanisöle, Thymianöle, Wacholderbeeröle, Rosmarinöle, Angelikawurzelöle, und die Fraktionen dieser Öle. Beispiele für einheitliche Aromastoffe sind z.B.:
Anethol, Menthol, Menthon, Isomenthon, Menthylacetat, Menthofuran, Menthylmethylether, Mintlacton, Eucalyptol, Limonen, Eugenol, Pinen, Sabinenhydrat, 3-Octanol, Carvon, gamma-Octalacton, gamma-Nonalacton, Germacren-D, Viridiflorol, 1,3E,5Z-Undecatrien, Isopulegol, Piperiton, 2-Butanon, Ethylformiat, 3-Octylacetat, Isoamylisovalerianat, Hexanol, Hexanal, cis-3-Hexenol, Linalool, alpha-Terpineol, cis und trans Carvylacetat, p-Cymol, Thymol, 4,8-Dimethyl-3,7-nonadien-2-on, Damascenon, Damascone, Rosenoxid, Dimethylsulfid, Fenchol, Acetaldehyddiethylacetal, cis-4-Heptenal, Isobutyraldehyd, Isovaleraldehyd, cis-Jasmon, Anisaldehyd, Methylsalicylat, Myrtenylacetat, 8-Ocimenylacetat, 2-Phenylethylalkohol, 2-Phenylethylisobutyrat, 2-Phenylethylisovalerat, Zimtaldehyd, Geraniol, Nerol. Bei chiralen Verbindungen können die genannten Aromastoffe als Racemat, als einzelnes Enantiomer oder als enantiomerenangereicherte Mischungen vorliegen.

Beispiele für weitere Geschmackstoffe, die vorteilhaft mit den erfindungsgemäßen Verbindungen der Formel (I) kombiniert werden können, sind z.B. Stoffe mit einer physiologischen Kühlwirkung, d.h. Stoffe, die auf der Haut oder Schleimhaut eine Kältempfindung hervorrufen. Solche Kühlwirkstoffe sind z.B. I-Menthol, I-Isopulegol, Menthonacetale (z.B. Menthonglycerinacetal), Menthylester, Ester aus Menthol und Hydroxycarbonsäuren mit 2 bis 6 C-Atomen (z.B. Menthyllactat), substituierte Menthan-3-carbonsäureamide (z.B. Menthan-3-carbonsäure-N-ethylamid), verzweigte Alkancarbonsäureamide (z.B. 2-Isopropyl-N,2,3-trimethylbutanamid), 3,3,5-Trimethylcyclohexanol, 3-Menthoxy-1,2-propandiol, 3-Menthoxy-2-methyl-1,2-propandiol, 2-Menthoxyethanol, 2-Menthoxypropanol, 3-Menthoxypropanol, 4-Menthoxybutanol, 2-Hydroxyethylmenthylcarbonat, 2-Hydroxypropylmenthylcarbonat, Glycerinmenthylcarbonat, N-Acetylglycinmenthylester, Menthylhydroxycarbonsäureester (z.B. Menthyl-3-hydroxybutyrat), Menthan-3,8-diol, Menthyl-2-methoxyacetat, Menthyl-2-(2-methoxyethoxy)acetat, Menthylmonosuccinat, 2-Mercaptocyclodecanon, Menthyl-2-pyrrolidin-5-oncarboxylat. Bevorzugte Stoffe mit einer physiologischen Kühlwirkung sind Menthylester, Menthonacetale, Menthan-3-carbonsäureamide und verzweigte Alkancarbonsäureamide.

Vorteilhaft ist auch die Verwendung der erfindungsgemäßen Verbindungen der Formel (I) in Verbindung mit Stoffen, die einen scharfen Geschmack oder eine Wärme- oder Hitzempfindung auf Haut und Schleimhäuten oder ein Prickel-, bzw. Kribbelgefühl im Mund- und Rachenraum hervorrufen, wie z.B. Paprikapulver, Chili-Pfeffer-Pulver, Extrakte aus Paprika, Extrakte aus Pfeffer, Extrakte aus Chili-Pfeffer, Extrakte aus Ingwerwurzeln, Extrakte aus Paradieskörnern (Aframomum melegueta), Extrakte aus Parakresse (Jambu-Oleoresin; Spilanthes acmella, bzw. Spilanthes oleracea), Extrakte aus Japanischem Pfeffer (Zanthoxylum piperitum), Extrakte aus Kaempferia galanga, Extrakte aus Alpinia galanga, Extrakte aus Wasserpfeffer (Polygonium hydropiper), Capsaicin, Dihydrocapsaicin, Gingerol, Paradol, Shogaol, Piperin, Saanshool-I, Saanshool-II, Sanshoamid, Spilanthol, Carbonsäure-N-Vanillylamide, insbesonders Nonansäure-N-vanillylamid, 2-Nonensäureamide, insbesonders 2-Nonensäure-N-isobutylamid, 2-Nonensäure-N-4-hydroxy-3-methoxyphenylamid, Alkylether von 4-Hydroxy-3-methoxybenzylalkohol, insbesonders 4-Hydroxy-3-methoxybenzyl-n-butylether, Alkylether von 3-Hydroxy-4-methoxybenzylalkohol, Alkylether von 3,4-Dimethoxybenzylalkohol, Alkylether von 3-Ethoxy-4-hydroxybenzylalkohol, Alkylether von 3,4-Methylendioxybenzylalkohol, Acetale von Vanillin, Acetale von Ethylvanillin, Acetale von Isovanillin, (4-Hydroxy-3-methoxyphenyl)essigsäureamide, insbesonders (4-Hydroxy-3-methoxyphenyl)essigsäure-N-n-octylamid, Allylisothiocyanat, Nicotinaldehyd, Methylnicotinat, Propylnicotinat, 2-Butoxyethylnicotinat, Benzylnicotinat, 1-Acetoxychavicol.

Bevorzugte Aromastoffe ohne physiologische Kühlwirkung sind die Aromastoffe, die zu ihrem eigentlichen geruchlichen Aromawert auch einen Geschmackseindruck, einen geschmacksmodulierenden Effekt oder einen trigeminalen, aber nicht-kühlenden oder auch einen mundwässernden Reiz verursachen. Bevorzugte Geschmackseindrücke sind süß, umami, bitter, salzig und sauer; bevorzugte geschmacksmodulierende Effekte sind bitter-maskierende, umami-verstärkende, süß-verstärkende, salz-verstärkende und sauer-maskierende Effekte; bevorzugte trigeminale Reize sind Schärfe, Wärme, Kribbeln und Stechen.

Bevorzugte modulierende Aroma- und/oder Geschmackstoffe werden vorzugsweise ausgewählt aus der Gruppe bestehend aus Adenosin-5'-monophosphat, Cytidin-5'-monophosphat, Inosin-5'-monophosphat, und deren pharmazeutisch akzeptablen Salzen; Lactisolen; 2,4-Dihydroxybenzoesäure; 3-Hydroxybenzoesäure; Natriumsalzen, vorzugsweise Natriumchlorid, Natriumlactat, Natriumcitrat, Natriumacetat, Natriumgluconoat; Hydroxyflavanonen, wie zum Beispiel Eriodictyol, Homoeriodictyol, und deren Natriumsalzen; Hydroxybenzoesäureamiden, wie zum Beispiel 2,4-Dihydroxybenzoesäurevanillylamid, 2,4-Dihydroxybenzoesäure-*N*-(4-hydroxy-3-methoxybenzyl)amid, 2,4,6-Trihydroxybenzoesäure-*N*-(4-hydroxy-3-methoxybenzyl)amid, 2-Hydroxy-benzoesäure-*N*-4-(hydroxy-3-methoxybenzyl)amid, 4-Hydroxybenzoesäure-*N-*(4-hydroxy-3-methoxybenzyl)amid, 2,4-Dihydroxybenzoesäure-*N*-(4-hydroxy-3-methoxybenzyl)amid-Mononatriumsalz, 2,4-Dihydroxybenzoesäure-*N*-2-(4-hydroxy-3-methoxyphenyl)ethylamid, 2,4-Dihydroxybenzoesäure-*N*-(4-hydroxy-3-ethoxybenzyl)amid, 2,4-Dihydroxybenzoesäure-*N*-(3,4-dihydroxybenzyl)amid und 2-Hyd roxy-5-methoxy-N-[2-(4-hydroxy-3-methoxyphenyl)ethyl]amid; 4-Hydroxybenzoesäurevanillylamid (insbesondere solche wie beschrieben in WO 2006/024587); Hydroxydeoxybenzoinen, wie zum Beispiel 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon) (insbesondere solche wie beschrieben in WO 2006/106023); Hydroxyphenylalkadionen, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527); Diacetyltrimeren (insbesondere solche wie beschrieben in WO 2006/058893); γ-Aminobuttersäure wie beschrieben in WO 2005/096841), Divanillin wie beschrieben in WO 2004/078302 und Hesperetin gemäß WO 2007/014879.

Ebenfalls besonders bevorzugte Aromastoffe ohne physiologische Kühlwirkung sind speichelflussfördernde Stoffe wie Pellitorine nach WO 04/000787 oder US 2004/0241312 und Alkamide nach DE 103 51 422.

Die Erfindung betrifft auch entsprechende Verfahren zur Herstellung von erfindungsgemäßen Verbindungen oder zur Herstellung von erfindungsgemäßen Mischungen von Verbindungen.

Die Synthese der erfindungsgemäßen Verbindungen der Formel (E) (Verbindung der Formel (I) mit X=NR¹R²) bzw. die Synthese entsprechender Mischungen wird vorzugsweise durch Umsetzung des entsprechenden Alkohols B-OH (wobei B die oben genannte Bedeutung hat) mit (a) Oxalylchlorid und anschließend mit einem entsprechenden Amin HNR¹R² oder (b) Dialkyloxalat und anschließend mit einem entsprechenden Amin HNR¹R² oder (c) Alkylchlorooxoacetat und anschließend mit einem entsprechenden Amin HNR¹R² oder (d) dem entsprechenden Aminooxoacetylchlorid erreicht.

Die Synthese erfindungsgemäßer Verbindungen der Formel (I) mit X=SR³ erfolgt analog. 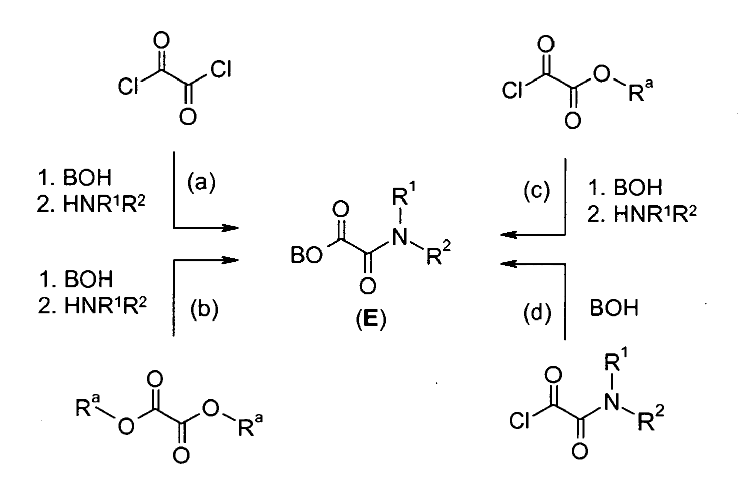 Dabei bedeuten die Reste R^{a} jeweils unabhängig voneinander einen Alkylrest, vorzugsweise einen C₁-C₄-Alkylrest, bevorzugt Methyl oder Ethyl.

Ein weiteres Verfahren zur Herstellung einer erfindungsgemäßen Verbindung nach Formel (I), umfasst die Schritte:
(1) Bereitstellen eines gemischten Oxalsäureesters der Formel 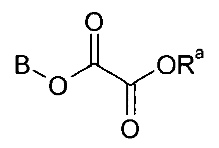 wobei R^{a} einen Alkylrest, vorzugsweise C₁-C₄-Alkylrest, bevorzugt Methyl oder Ethyl, bedeutet, und B die oben angegebene Bedeutung hat,
   oder eines Oxalsäurehalogenids, vorzugsweise Oxalsäurechlorids, der Formel 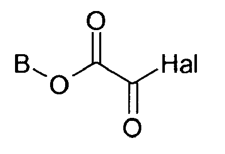 wobei Hal Halogenid bedeutet, vorzugsweise Cl oder Br, insbesondere bevorzugt Cl, und
   wobei B die oben angegebene Bedeutung hat,
(2) Bereitstellen eines Amins HNR¹R² oder Thiols HSR³ oder eines entsprechenden Salzes, wobei R¹, R² und R³ die oben angegebene Bedeutung haben,
(3) Umsetzen der bereitgestellten Verbindungen miteinander, so dass eine erfindungsgemäße Verbindung entsteht.

Ein besonders bevorzugter Syntheseweg kann anhand des nachfolgenden Reaktionsschemas verdeutlicht werden, welches zu einer Verbindung der Formel (la) führt, wobei R¹ und R² jeweils die oben angegebene Bedeutung haben. Ein entsprechender Syntheseweg ist bevorzugt für die Herstellung einer Verbindung der Formel (la).

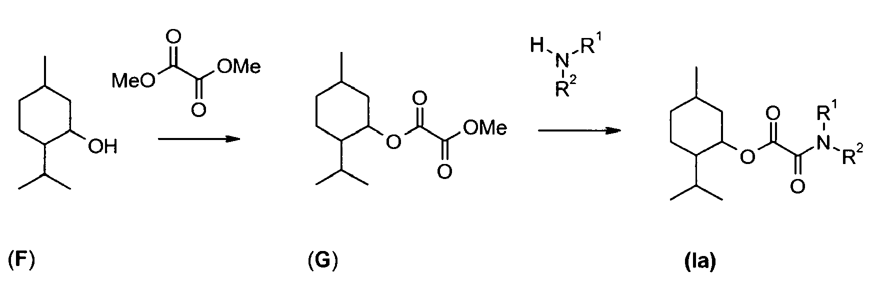

Dabei kann der Alkohol - hier Menthol (F) - beispielsweise nach bekannten Verfahren mittels Dimethyloxalat und eines Katalysators in das entsprechende gemischte Oxalat (G) überführt werden.
Optional kann diese Umsetzung in Gegenwart von anderen (Hilfs)Stoffen oder Additiven erfolgen, z.B. in Gegenwart von
(i) einem oder mehreren Lösungsmitteln bzw. Verdünnungsmitteln (z.B.Toluol, 1,4-Dioxan, Dichlormethan, Tetrahydrofuran, andere Ether, Chloroform, Ethylacetat, Aceton, Alkanen, Cycloalkanen, Alkoholen) und/oder
(ii) Phasentransferkatalysatoren (z.B. peralkylierte/perarylierte Ammoniumsalze oder Phosphoniumsalze, Kronenether).

Die Rohprodukte der Synthese werden vorzugsweise durch physikalische, gegebenenfalls auch enantioselektive oder enantiospezifische Trennmethoden, z.B. Extraktion, Verteilungsmethoden, Kristallisation, Destillation, Chromatographie, Sublimation, Wasserdampfdestillation, Umkehrosmose, Permeation oder dergleichen aufgereinigt bzw. angereichert, wobei die Trennmethode vorzugsweise so gewählt ist, dass nach der Trennoperation die Stereochemie der erfindungsgemäßen Verbindungen der Formel (I) (soweit die Formel (I) die Stereochemie festlegt) zu einem Anteil von 90 - 100 Mol-%, bevorzugt 95 - 100 Mol-%, entspricht, bezogen auf die Gesamtmenge der in dem aufgereinigten Produkt vorliegenden Verbindungen der Formel (I).

Die Erfindung betrifft auch der Ernährung, der Mundhygiene oder dem Genuss dienende oder pharmazeutische oder kosmetische Zubereitungen, die eine zum Erreichen einer physiologischen Kühlwirkung auf der Haut und/oder Schleimhaut ausreichende Menge (1) einer erfindungsgemäßen Verbindung oder einer erfindungsgemäßen Mischung (vorzugsweise in einer als bevorzugt angegebenen Ausgestaltung) oder (2) (ii) einer erfindungsgemäßen Mischung (vorzugsweise in einer als bevorzugt angegebenen Ausgestaltung) umfassen. Insbesondere soll die eingesetzte Menge der Verbindung, der Mischung zum Erreichen einer physiologischen Kühlwirkung auf der Schleimhaut im Mund-, Nasen- und/oder Rachenraum ausreichen.

Bevorzugte erfindungsgemäße Zubereitungen umfassen übliche Grund-, Hilfs- und Zusatzstoffe für der Ernährung, der Mundhygiene oder dem Genuss dienende oder pharmazeutische oder kosmetische Zubereitungen. Bevorzugte erfindungsgemäße Zubereitungen enthalten 0,0001 Gew.-% bis 20 Gew.-%, bevorzugt 0,0001 bis 10 Gew.-%, besonders bevorzugt 0,001 Gew.-% bis 0,5 Gew.-% an Verbindungen der Formel (I), bezogen auf das Gesamtgewicht der Zubereitung. Weitere Bestandteile, insbesondere Verbindungen der Formel (I) sowie Bestandteile (2) (weitere Stoffe mit physiologischer Kühlwirkung), (3) (Aromastoffe ohne physiologische Kühlwirkung) und/oder (4) (trigeminal oder mundwässernd wirksame Stoffe ohne physiologische Kühlwirkung) (wie oben beschrieben) sowie weitere übliche Grund-, Hilfs- und Zusatzstoffe können in Mengen von 0,0000001 bis 99,99 Gew.-%, vorzugsweise 10 bis 80 Gew.-%, bezogen auf das Gesamtgewicht der Zubereitung, enthalten sein. Ferner können die erfindungsgemäßen Zubereitungen Wasser in einer Menge bis zu 99,99 Gew.-%, vorzugsweise 5 bis 80 Gew.-%, bezogen auf das Gesamtgewicht der Zubereitung, enthalten.

Der Ernährung oder dem Genuss dienende Zubereitungen sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Liköre, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, eingekochte Gemüse), Knabberartikel (z.B. gebackene oder frittierte Kartoffelchips oder Kartoffelteigprodukte, Extrudate auf Mais- oder Erdnussbasis), Produkte auf Fett- und Ölbasis oder Emulsionen derselben (z.B. Mayonnaise, Remoulade, Dressings), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nicht überzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Der Mundhygiene dienende Zubereitungen sind insbesondere Zahnpflegemittel wie Zahnpasten, Zahngele, Zahncremes, Zahnpulver, Mundwässer, Zahncreme und Mundwasser als 2-in-1 Produkt, Zahnseide, (zuckerfreie) Kaugummis und andere Mundpflegemittel.

Zahnpflegemittel (als Basis für der Mundpflege dienende Zubereitungen), welche die erfindungsgemäße Verbindungen, Mischungen enthalten, umfassen im allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Natrium-Cyclamat, Sucralose, Acesulfam-K oder Zuckeralkohole, Geschmackskorrigenzien für unangenehme Geschmackseindrücke wie z.B Hydroxyflavanone nach US 2002/0188019, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für der Mundpflege dienende Zubereitungen), die erfindungsgemäße Verbindungen, Mischungen enthalten, umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkohole, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Im Stand der Technik sind zahlreiche unterschiedliche Kaugummibasen bekannt, wobei zwischen sogenannten "chewing gum" - oder um "bubble gum" - Basen zu unterscheiden ist, wobei letztere weicher sind, damit sich damit auch Kaugummiblasen bilden lassen. Gängige Kaugummibasen umfassen neben traditionell eingesetzten natürlichen Harzen oder dem Naturlatex Chicle heute zumeist Elastomere wie Polyvinylacetate (PVA), Polyethylene, (nieder- oder mittelmolekulare) Polyisobutene (PIB), Polybutadiene, Isobuten-Isopren Copolymere (Butyl Rubber), Polyvinylethylether (PVE), Polyvinylbutylether, Copolymere von Vinylestern und Vinylethern, Styrol-Butadien-Copolymere (Styrol-Butadien-Rubber, SBR) oder Vinyl-Elastomere, z.B. auf Basis Vinylacetat/Vinyllaurat, Vinylacetat/Vinylstaerat oder Ethylen/Vinylacetat, sowie Mischungen der genannten Elastomere, wie beispielsweise in EP 0 242 325, US 4,518,615, US 5,093,136, US 5,266,336 US 5,601,858 oder US 6,986,709 beschrieben. Daneben umfassen Kaugummibasen weitere Bestandteile wie beispielsweise (mineralische) Füllstoffe, Weichmacher, Emulgatoren, Antioxidantien, Wachse, Fette oder fette Öle, wie beispielsweise gehärtete (hydrierte) pflanzliche oder tierische Fette, Mono-, Di- oder Triglyceride. Geeignete (mineralische) Füllstoffe sind beispielsweise Calciumcarbonat, Titandioxid, Siliciumdioxid, Talkum, Aluminiumoxid, Dicalciumphosphat, Tricalciumphosphat, Magnesiumhydroxid und deren Mischungen. Geeignete Weichmacher bzw. Mittel zur Verhinderung des Verklebens (detackifier) sind beispielsweise Lanolin, Stearinsäure, Natriumstearat, Ethylacetat, Diacetin (Gylcerindiacetat), Triacetin (Gylcerintriacetat), Triethylcitrat. Geeignete Wachse sind beispielsweise Paraffin Wachse, Candelilla Wachs, Carnauba Wachs, mikrokristalline Wachse und Polyethylen Wachse. Geeignete Emulgatoren sind beispielsweise Phosphatide wie Lecithin, Mono- und Diglyceride von Fettsäuren, z.B. Glycerinmonostearat

Bevorzugte erfindungsgemäße pharmazeutische Zubereitungen im Sinne der Erfindung sind orale Zubereitungen, die z.B. in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen und als verschreibungspflichtige, apothekenpflichtige oder sonstige Arzneimittel oder als Nahrungsergänzungsmittel verwendet werden.

Erfindungsgemäße kosmetische Zubereitungen können z. B. in einer der folgenden Formen vorliegen: Seife, Syndet, flüssiges Wasch-, Dusch-, oder Badepräparat, Emulsion (als Lösung, Dispersion, Suspension, Creme, Lotion oder Milch je nach Herstellungsverfahren und Inhaltsstoffen vom Typ "Wasser-in-Öl" (W/O), "Öl-in-Wasser" (O/W) oder multiple Emulsion, PIT Emulsion, Emulsionsschaum, Mikroemulsion, Nanoemulsion, Pickering Emulsion), Salbe, Paste, Gel (inklusive Hydro-, Hydrodispersions-, Oleogel), Öl, Toner, Balsam, Serum, Puder, Eau de Toilette, Toilette, Eau de Cologne, Perfum, Wachs, als Stift, Roll-On, (Pump-)Spray, Aerosol (schäumend, nicht schäumend oder nach-schäumend), Fusspflegemittel (inklusive Keratolytika, Desodorant), Bartreinigungs- oder -pflegemittel, Insekten abwehrendes Mittel, Sonnenschutzmittel, Aftersun-Präparat, Rasiermittel (z.B. Rasierschäume, -seifen oder - gele) oder After-Shave (Balm, Lotion), Haarentfernungsmittel, Haarpflegemittel wie z.B. Shampoo (z.B. 2-in-1 Shampoo, Anti-Schuppen-Shampoo, Babyshampoo, Shampoo für trockene Kopfhaut, Shampookonzentrat), Conditioner, Haarkur, Haarwasser, Haarspülung, Frisiercreme, Pomade, Dauerwell- und Fixierungsmittel, Haarglättungsmittel (Entkräuselungsmittel, Relaxer), Haarfestiger, Styling-Hilfe (z.B. Gel oder Wachs), Blondiermittel, Haaraufheller, Haarconditioner, Haarmousse, Haartönung, Haarfärbemittel (z.B. temporäre, direktziehende, semipermanente, permanente Haarfärbemittel), Nagelpflegemittel wie z.B. Nagellack und Nagellackentferner, Deodorant und / oder Antitranspirant, Mundwasser, Munddusche, Make-Up, Make-Up-Entferner, Augenpflege, Lippenkosmetika, Lippenpflegemittel, dekorative Kosmetik (z.B. Puder, Lidschatten, Kajalstift, Lippenstift), Badeartikel (z.B. Kapsel), oder Maske.

Erfindungsgemäße Zubereitungen, die erfindungsgemäße Verbindungen, oder eine erfindungsgemäße Mischung umfassen, werden vorzugsweise hergestellt, indem die Verbindung, oder die Mischung, z. B. eine Mischung, die neben einer erfindungsgemäßen Verbindung einen festen oder flüssigen Trägerstoff umfasst, in eine Basis-Zubereitung eingearbeitet wird. Vorteilhafterweise werden zunächst als Lösung vorliegende erfindungsgemäße Mischungen, die eine erfindungsgemäße Verbindung umfassen, durch Sprühtrocknung in eine feste Zubereitung überführt.

Erfindungsgemäße Zubereitungen können gemäß einer alternativen bevorzugten Ausführungsform hergestellt werden, indem die erfindungsgemäßen Verbindungen bzw. Mischungen, gegebenenfalls mit weiteren Bestandteilen der erfindungsgemäßen Zubereitung, zunächst in Emulsionen, in Liposomen, z. B. ausgehend von Phosphatidylcholin, in Microsphären, in Nanosphären oder auch in Kapseln, Granulaten oder Extrudaten aus einer für Lebens- und Genussmittel oder kosmetische Zubereitungen geeigneten Matrix, z.B. aus Stärke, Stärkederivaten (z.B. modifizierte Stärke), Cellulose oder Cellulosederivaten (z.B. Hydroxypropylcellulose), anderen Polysacchariden (z.B. Dextrin, Alginat, Curdlan, Carageenan, Chitin, Chitosan, Pullulan), natürlichen Fetten, natürlichen Wachsen (z.B. Bienenwachs, Carnaubawachs), aus Proteinen, z.B. Gelatine oder sonstigen Naturprodukten (z.B. Schellack) oder nichtnatürlichen Matrixmaterialien (wie Polyharnstoff) eingearbeitet werden. Dabei können je nach Matrix die Produkte durch Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren und ggfs. einer geeigneter Kombination der vorgenannten Verfahren behandelt werden.

In einem weiteren bevorzugten Herstellungsverfahren werden die erfindungsgemäßen Verbindungen bzw. Mischungen zunächst mit einem oder mehreren geeigneten Komplexbildnern, beispielsweise mit Cyclodextrinen oder Cyclodextrinderivaten, bevorzugt alpha-, beta- oder gamma-Cyclodextrin, komplexiert und in dieser komplexierten Form eingesetzt.

Besonders bevorzugt ist eine erfindungsgemäße Zubereitung, bei der die Matrix so gewählt ist, dass die erfindungsgemäßen Verbindungen oder Mischungen, insbesondere Mischungen, die weitere Kühlwirkstoffe und/oder Aromen umfassen, verzögert von der Matrix freigegeben werden, so dass eine langanhaltende Kühlwirkung erreicht wird.

Als weitere Bestandteile für erfindungsgemäße, der Ernährung oder dem Genuss dienende Zubereitungen können übliche Grund-, Hilfs- und Zusatzstoffe für Nahrungs-oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder - pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Mannitol, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutahthion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Kombination einer erfindungsgemäßen Verbindung oder einer erfindungsgemäßen Mischung mit Zuckeralkoholen, insbesondere mit Xylitol, Sorbitol, Mannitol und/oder Erythritol zeigten eine verstärkte, zum Teil synergistisch verstärkte, Kühlwirkung.

Ein weiterer Aspekt der vorliegenden Erfindung betrifft die Verwendung einer (a) Verbindung der Formel (I) oder (b) Mischung umfassend eine, zwei oder mehr Verbindungen der Formel (I) oder (c) Mischung bestehend aus zwei oder mehr Verbindungen der Formel (I) 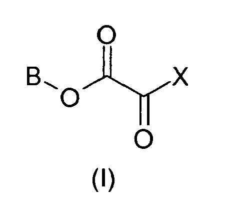 wobei jeweils gilt:
B bedeutet einen Kohlenwasserstoffrest ausgewählt aus der folgenden Gruppe: 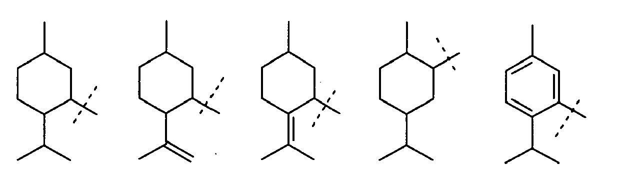 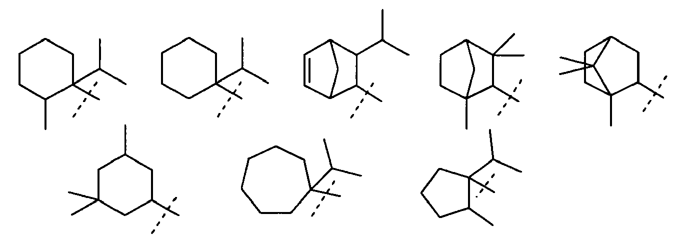
worin die
gestrichelte 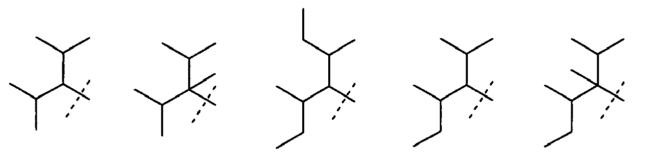 Linie die Bindung markiert, welche den Kohlenwasserstoffrest B mit dem in Formel (I) benachbarten Sauerstoffatom verknüpft,
X bedeutet NR¹R² oder SR³,
wobei jeweils unabhängig voneinander
in NR¹R² die Reste R¹ und R² ausgewählt sind und
in SR³ der Rest R³ ausgewählt ist
aus der Gruppe bestehend aus:
Wasserstoff
und
organischer Rest mit
1 bis 12 C-Atomen der
ausgewählt ist aus der Gruppe bestehend aus substituiertem oder nicht substituiertem
Alkyl, Heteroalkyl, Cycloalkyl, Heterocycloalkyl, Cycloalkylalkyl, Alkenyl, Cycloalkenyl, Cycloalkenylalkyl, Alkinyl, Cycloalkylalkinyl, Aryl, Heteroaryl, Arylalkyl, Cycloalkylaryl, Cycloalkenylaryl, Cycloalkylheteroaryl, Heterocycloalkylaryl, Heterocycloalkenylaryl, Heterocycloalkenylheteroaryl und Heteroarylalkyl,
wobei gegebenenfalls in NR¹R² die Reste R¹ und R² unter Fortfall je eines Wasserstoffatomes und unter Ausbildung eines Ringes kovalent miteinander verbunden sind
zum Erzeugen einer Kühlwirkung auf der Haut oder einer Schleimhaut zu anderen als therapeutischen Zwecken.

Ein weiterer Aspekt das vorliegenden Erfindung ist in Anspruch 11 wiedergegehen.

Hinsichtlich bevorzugt zu verwendender (a) Verbindungen, (b) Mischungen umfassend eine, zwei oder mehr Verbindungen der Formel (I) oder (c) Mischung bestehend aus zwei oder mehr Verbindungen der Formel (I) gilt das oben zu den erfindungsgemäßen Mischungen Gesagte entsprechend.

Bevorzugt werden die erfindungsgemäßen Verbindungen (a) und/oder Mischungen (b), (c) zum Herstellen eines Arzneimittels, das der Bekämpfung oder der Linderung von Husten-Schnupfen-, Mund-, Nasen-, Hals- oder Rachenentzündungs-, Halsschmerz-oder Heiserkeitssymptomen dient, verwendet.

Ein weiterer Aspekt der vorliegenden Erfindung betrifft ein nicht-therapeutisches Verfahren zum Erreichen einer physiologischen Kühlwirkung auf der Haut und/oder einer Schleimhaut, mit folgendem Schritt:
- Applizieren einer zum Erreichen einer physiologischen Kühlwirkung ausreichenden Menge
   (1) einer Verbindung oder einer Mischung wie oben beschrieben
   (2) einer Zubereitung wie oben beschrieben auf die Haut und/oder eine Schleimhaut.

Ein weiterer Aspekt der Erfindung betrifft die Verwendung erfindungsgemäßer Zubereitungen, enthaltend eine erfindungsgemäße Verbindung oder eine erfindungsgemäße Mischung, vorzugsweise eine erfindungsgemäße Mischung, welche einen oder mehrere Aromastoffe und/oder einen oder mehrere weitere Kühlwirkstoffe (Kühlwirkstoffe, bei denen es sich nicht um eine Verbindung der allgemeinen Formel (I) handelt) umfasst, als Halbfertigwaren (sogenannte Aromamischungen) zur Aromatisierung von unter Verwendung der Halbfertigwaren gefertigten Fertigwaren.

Weitere Aspekte der vorliegenden Erfindung ergeben sich aus den nachfolgenden Beispielen sowie den beigefügten Patentansprüchen.

### Beispiele

Die Beispiele dienen nur zur Verdeutlichung der Erfindung, ohne diese damit einzuschränken. Sofern nicht anders angegeben, beziehen sich alle Angaben auf das Gewicht.

### Beispiel 1: Synthese von L-Menthylmethyloxalat

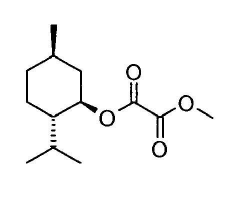

156 g L-Menthol wurden mit 116 g Triethylamin in 500 ml Dichlormethan vorgelegt und Monomethyloxalylchlorid bei 0°C innerhalb von 30 Minuten zugetropft. Es wurde eine Stunde bei Raumtemperatur nachgerührt und anschließend mit Wasser gewaschen. Nach Entfernen des Lösungsmittels wurden 240 g der Verbindung erhalten.
¹H-NMR (400 MHz, CDCl₃, TMS): δ= 0.78 (d, J = 7.0 Hz, 3 H), 0.86-0.96 (m, 1 H), 0.91 (d, J = 7.0 Hz, 3 H), 0.93 (d, J = 6.5 Hz, 3 H) 1.02-1.19 (m, 2 H), 1.46-1.58 (m, 2 H), 1.67-1.76 (m, 2 H), 1.89 (dhept, J = 2.8 Hz, J = 7.0 Hz, 1 H), 2.02-2.08 (m, 1 H), 3.90 (s, 3 H), 4.87 (dt, J = 11 Hz, J = 4.5 Hz, 1 H) ppm.
¹³C-NMR (100 MHz, CDCl₃, TMS): δ = 16.18 (CH₃), 20.65 (CH₃), 21.92 (CH₃), 23.35 (CH₂), 26.17 (CH), 31.46 (CH), 34.02 (CH₂), 40.28 (CH₂), 46.72 (CH), 53.46 (CH₃), 77.89 (CH), 157.33 (CO), 158.59 (CO) ppm.
MS (EI): *mlz* = 242 (1, M⁺), 227 (1), 183 (1), 155 (1), 139 (22), 123 (7), 95 (30), 83 (100), 69 (40), 55 (55).

### Beispiel 2: Synthese von L-Menthyl-N-methyloxamat

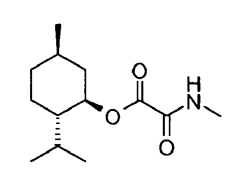

120 g L-Menthylmethyloxalat wurden in 1000 mL Diethylether vorgelegt und langsam und unter konstantem Kühlen (0°C) 1,1 Äquivalente Methylamin zugetropft. Es wurde 30 Minuten nachgerührt, mit Wasser gewaschen und nach Trocknen und Einengen 116 g Rohprodukt aus 290 g Heptan umkristallisiert. Es wurden so 98,1 g L-Menthyl-N-Methyloxamat erhalten.
¹H-NMR (400 MHz, CDCl₃, TMS): δ = 0.75 (d, J = 7.0 Hz, 3 H), 0.85-0.96 (m, 1 H), 0.90 (d, J = 7.0 Hz, 3 H), 0.92 (d, J = 6.6 Hz, 3 H), 1.01-1.22 (m, 2 H), 1.44-1.64 (m, 2 H), 1.67-1.75 (m, 2 H), 1.90 (dhept, J = 7.0 Hz, J = 2.8 Hz, 1 H), 1.97-2.03 (m, 1 H), 2.93 (d, J = 5.2 Hz, 3 H), 4.83 (dt, J = 11 Hz, J = 4.5 Hz, 1 H), 7.14 (s, br., NH) ppm.
¹³C-NMR (100 MHz, CDCl₃, TMS): δ = 16.09 (CH₃), 20.65 (CH₃), 21.94 (CH₃), 23.26 (CH₂), 26.08 (CH), 26.46 (CH₃), 31.50 (CH), 34.03 (CH₂), 40.29 (CH₂), 46.57 (CH), 77.77 (CH), 157.46 (CO), 160.26 (CO) ppm.
MS (EI): *m*/*z* = 241 (1, M⁺), 183 (1), 155 (1), 139 (20), 123 (5), 97 (19), 83 (100), 69 (37), 55 (52).

### Beispiel 3: Synthese von L-Menthyl-N-ethyloxamat

Analog zur Synthese von L-Menthyl-N-methyloxamat wurde ausgehend von L-Menthylmethyloxalat das L-Menthyl-N-ethyloxamat erhalten. 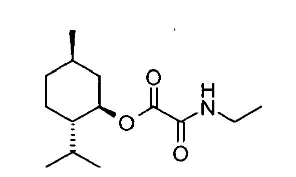 ¹H-NMR (400 MHz, CDCl₃, TMS): δ = 0.76 (d, J = 7.0 Hz, 3 H), 0.85-0.96 (m, 1 H), 0.90 (d, J = 7.0 Hz, 3 H), 0.92 (d, J = 6.6 Hz, 3 H), 1.02-1.22 (m, 2 H), 1.22 (t, J = 7.3 Hz, 3 H), 1.44-1.64 (m, 2 H), 1.66-1.76 (m, 2 H), 1.91 (dhept, J = 7.0 Hz, J = 2.7 Hz, 1 H), 1.97-2.04 (m, 1 H), 3.35-3.43 (m, 2 H), 4.83 (dt, J = 11.0 Hz, J = 4.5 Hz, 1 H), 7.17 (s, br., 1 NH) ppm.
¹³C-NMR (100 MHz, CDCl₃, TMS): δ = 14.39 (CH₃), 16.10 (CH₃), 20.65 (CH₃), 21.94 (CH₃), 23.27 (CH₂), 26.05 (CH), 31.50 (CH), 34.03 (CH₂), 34.81 (CH₂), 40.28 (CH₂), 46.55 (CH), 77.70 (CH), 156.65 (CO), 160.48 (CO) ppm.
MS (EI): *m*/*z* = 255 (1, M⁺), 168 (1), 155 (1), 139 (20), 123 (5), 97 (20), 83 (100), 69 (37), 55 (50).

### Beispiel 4: Synthese von Neomenthyl-N-methyloxamat

Analog zur Synthese von L-Menthyl-N-methyloxamat wurde ausgehend von Neomenthylmethyloxalat das Neomenthyl-N-methyloxamat erhalten. 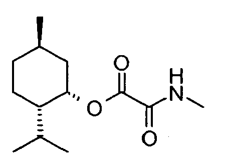 ¹H-NMR (400 MHz, CDCl₃, TMS): δ = 0.84-1.16 (m, 4 H), 0.85 (d, J = 6.6 Hz, 3 H), 0.86 (d, J = 6.7 Hz, 3 H), 0.90 (d, J = 6.7 Hz, 3 H), 1.43-1.57 (m, 2 H), 1.63-1.83 (m, 2 H), 1.94-2.01 (m, 1 H), 2.92 (d, J = 5.2 Hz, 3 H), 5.34 (d, br., J = 2.1 Hz, 1 H), 7.05 (s, br., 1 NH) ppm.
¹³C-NMR (100 MHz, CDCl₃, TMS): δ = 20.77 (CH₃), 20.84 (CH₃), 22.04 (CH₃), 24.86 (CH₂), 26.39 (CH), 26.41 (CH₃), 29.05 (CH), 34.60 (CH₂), 38.84 (CH₂), 46.79 (CH), 74.90 (CH), 157.48 (CO), 160.09 (CO) ppm.
MS (EI): *m*/*z* = 242 (1, MH⁺), 182 (1), 155 (1), 139 (25), 123 (5), 95 (20), 83 (100), 69 (45), 55 (55).

### Beispiel 5: Synthese von Neomenthyl-N-cyclopropyloxamat

Analog zur Synthese von L-Menthyl-*N*-methyloxamat wurde ausgehend von Neomenthylmethyloxalat das *N*eomenthyl-*N*-cyclopropyloxamat erhalten. 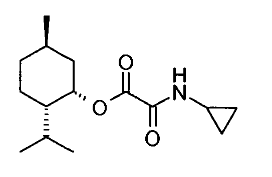 ¹H-NMR (400 MHz, CDCl₃, TMS): δ = 0.60-0.65 (m, 2 H), 0.82-1.15 (m, 5 H), 0.85 (d, J = 6.5 Hz, 3 H), 0.86 (d, J = 6.7 Hz, 3 H), 0.90 (d, J = 6.7 Hz, 3 H), 1.44-1.57 (m, 2 H), 1.62-1.82 (m, 3 H), 1.92-2.00 (m, 1 H), 2.77-2.84 (m, 1 H), 5.32 (d, br., J = 2.1 Hz, 1 H), 7.08 (s, br., 1 H) ppm.
¹³C-NMR (100 MHz, CDCl₃, TMS): δ = 6.53 (2 × CH₂), 20.79 (CH₃), 20.83 (CH₃), 22.04 (CH₃), 22.81 (CH), 24.80 (CH₂), 26.35 (CH), 29.00 (CH), 34.61 (CH₂), 38.83 (CH₂), 46.83 (CH), 74.97 (CH), 157.95 (CO), 160.25 (CO) ppm.
MS (EI): *m*/*z* = 267 (1, M⁺), 155 (1), 139 (25), 129 (22), 95 (20), 83 (100), 69 (30), 55 (45).

### Beispiel 6: Synthese von L-Menthylmorpholinooxamat (Morpholin-4-yl-oxo-essigsäure-(1 R,2S,5R)-2-isopropyl-5-methyl-cyclohexylester)

Analog zur Synthese von L-Menthyl-N-methyloxamat wurde ausgehend von L-Menthylmethyloxalat das L-Menthylmorpholinooxamat erhalten. 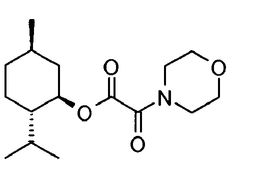 ¹H-NMR (400 MHz, CDCl₃, TMS): δ = 0.79 (d, J = 6.9 Hz, 3 H), 0.85-0.95 (m, 1 H), 0.91 (d, J = 7.0 Hz, 3 H), 0.93 (d, J = 7.0 Hz, 3 H), 1.02-1.16 (m, 2 H). 1.46-1.58 (m, 2 H), 1.67-1.76 (m, 2 H), 1.92 (dhept, J = 7.0 Hz, J = 2.8 Hz, 1 H), 2.03-2.10 (m, 1 H), 3.42-3.46 (m, 2 H), 3.58-3.77 (m, 6 H), 4.86 (dd, J = 10.9 Hz, J = 4.4 Hz, 1 H) ppm.
¹³C-NMR (100 MHz, CDCl₃, TMS): δ = 16.08 (CH₃), 20.68 (CH₃), 21.93 (CH₃), 23.26 (CH₂), 26.12 (CH), 31.48 (CH), 34.01 (CH₂), 40.47 (CH₂), 41.60 (CH₂), 46.33 (CH₂), 46.74 (CH), 66.40 (CH₂), 66.62 (CH₂), 76.79 (CH) 160.49 (CO), 162.30 (CO) ppm.

MS (EI): *m*/*z* = 297 (2, M⁺), 160 (1), 139 (15), 114 (14), 97 (20), 83 (100), 69 (30), 55 (35).

### Beispiel 7: Synthese von L-Menthyloxamat

Analog zur Synthese von L-Menthyl-N-methyloxamat wurde ausgehend von L-Menthylmethyloxalat das L-Menthyloxamat erhalten. 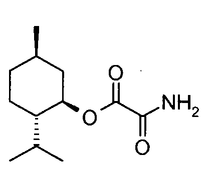 ¹H-NMR (400 MHz, CDCl₃, TMS): δ = 0.77 (d, J = 7.1 Hz, 3 H), 0.90 (d, J = 7.1 Hz, 3 H), 0.85-0.97 (m, 1 H), 0.93 (d, J = 6.7 Hz, 3 H), 1.02-1.22 (m, 2 H), 1.46-1.63 (m, 2 H), 1.67-1.76 (m, 2 H), 1.89 (dhept, J = 7.0 Hz, J = 2.7 Hz, 1 H), 1.99-2.05 (m, 1 H), 4.85 (dt, J = 11.0 Hz, J = 4.5 Hz, 1 H), 6.23 (s, br. NH), 6.98 (s, br. NH) ppm.
¹³C-NMR (100 MHz, CDCl₃, TMS): δ = 16.10 (CH₃), 20.65 (CH₃), 21.92 (CH₃), 23.25 (CH₂), 26.13 (CH), 31.50 (CH), 34.00 (CH₂), 40.28 (CH₂), 46.60 (CH), 78.08 (CH), 158.73 (CO), 159.67 (CO) ppm.
MS (EI): *m*/*z =* 155 (1), 139 (20), 123 (5), 97 (17), 83 (100), 69 (32), 55 (55), 41 (25).

### Beispiel 8: Synthese von Bornyl-N-ethyloxamat

Analog zur Synthese von L-Menthyl-*N*-methyloxamat wurde ausgehend von Bornylmethyloxalat das Bornyl-*N*-ethyloxamat erhalten. 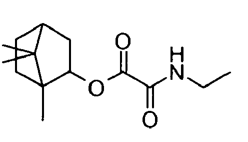 ¹H-NMR (400 MHz, CDCl₃, TMS): δ = 0.88 (s, 3 H), 0.9 (s, 3 H), 0.93 (s, 3 H), 1.11 (dd, J = 13.8 Hz, J = 3.4 Hz, 1 H), 1.22 (t, J = 7.3 Hz, 3 H), 1.28-1.41 (m, 2 H), 1.70-1.81 (m, 2 H), 2.04-2.13 (m, 1 H), 2.35-2.44 (m, 1 H), 3.34-3.43 (m, 2 H), 5.01 (ddd, J = 10 Hz, J = 3.5 Hz, J = 2.1 Hz, 1 H), 7.06 (s, br., 1 NH) ppm.
¹³C-NMR (100 MHz, CDCl₃, TMS): δ = 13.49 (CH₃), 14.42(CH₃), 18.84 (CH₃), 19.68 (CH₃), 27.02 (CH₂), 27.91 (CH₂), 34.77 (CH₂), 36.27 (CH₂), 44.85 (CH), 48.07 (C), 49.17 (C), 83.18 (CH), 156.58 (CO), 161.26 (CO) ppm.
MS (EI): *mlz* = 253 (2, M⁺), 225 (3), 197 (10), 181 (10), 153 (52), 137 (70), 121 (23), 109 (21), 95 (98), 81 (100), 41 (25).

### Beispiel 9: Synthese von D-Menthyl-N,N-dimethyloxamat

Analog zur Synthese von L-Menthyl-N-methyloxamat wurde ausgehend von *D*-Menthylmethyloxalat das *D*-Menthyl-*N,N*-dimethyloxamat erhalten. 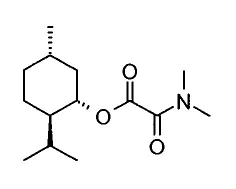 ¹H-NMR (400 MHz, CDCl₃, TMS): δ = 0.80 (d, J = 7.0 Hz, 3 H), 0.87-0.95 (m, 1 H), 0.91 (d, J = 7.0 Hz, 3 H), 0.93 (d, J = 6.5 Hz, 3 H), 1.03-1.17 (m, 2 H), 1.45-1.59 (m, 2 H), 1.67-1.76 (m, 2 H), 1.96 (dhept, J = 2.8 Hz, J = 7.0 Hz, 1 H), 2.06-2.12 (m, 1 H), 2.98 (s, 3 H), 3.01 (s, 3 H), 4.87 (dt, J = 11 Hz, J = 4.4 Hz, 1 H) ppm.
¹³C-NMR (100 MHz, CDCl₃, TMS): δ = 16.03 (CH₃), 20.70 (CH₃), 21.93 (CH₃), 23.23 (CH₂), 26.02 (CH), 31.46 (CH), 33.89 (CH₃), 34.04 (CH₂), 36.98 (CH₃), 40.44 (CH₂), 46.75 (CH), 76.39 (CH), 162.06 (CO), 162.94 (CO) ppm.
MS (EI): *mlz* = 255 (1, M⁺), 139 (17), 123 (5), 97 (20), 83 (100), 69 (36), 55 (49).

### Beispiel 10: Synthese von Fenchyl-N-methyloxamat

Analog zur Synthese von L-Menthyl-N-methyloxamat wurde ausgehend von Fenchylmethyloxalat das Fenchyl-N-methyloxamat erhalten. 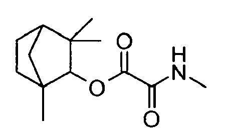 ¹H-NMR (400 MHz, CDCl₃, TMS): δ = 0.82 (s, 3 H), 1.08 (s, 3 H), 1.12 (s, 3 H), 1.17 (m, 1 H), 1.25 (dd, J = 10.4 Hz, J = 1.6 Hz, 1 H), 1.49 (m, 1 H), 1.62 (m, 1 H), 1.74-1.76 (m, 2 H), 1.90 (m, 1 H), 2.93 (d, J = 5.2 Hz, 3 H), 4.48 (d, J = 2.0 Hz, 1 H), 7.05 (s, br. 1 H) ppm.
¹³C-NMR (100 MHz, CDCl₃, TMS): δ = 19.34 (CH₃), 20.20 (CH₃), 25.79 (CH₂), 26.42 (CH₃), 26.53 (CH₂), 29.78 (CH₃), 39.77 (C), 41.46 (CH₂), 48.28 (CH), 48.53 (C), 89.30 (CH), 157.28 (CO), 161.02 (CO) ppm.
MS (EI): *m*/*z =* 240 (1, MH⁺), 211 (1), 183 (1), 153 (100), 137 (30), 81 (90), 58 (20).

### Beispiel 11: Synthese von 1-Isopropyl-2-methylpropyl-N-ethyloxamat

Analog zur Synthese von L-Menthyl-*N*-methyloxamat wurde ausgehend von (1-Isopropyl-2-methyl)propyl-methyloxalat das 1-Isopropyl-2-methylpropyl-*N*-ethyloxamat erhalten. 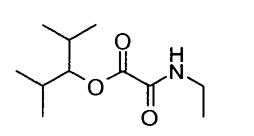 ¹H-NMR (400 MHz, CDCl₃, TMS): δ = 0.91 (d, J = 6.9 Hz, 6 H), 0.92 (d, J = 6.7 Hz, 6 H), 1.22 (t, J = 7.2 Hz, 3 H), 2.03 (m, 2 H), 3.39 (m, 2 H), 4.70 (t, J = 6.2 Hz, 1 H), 7.06 (s, br., NH) ppm.
¹³C-NMR (100 MHz, CDCl₃, TMS): δ = 14.43 (CH₃), 17.23 (2 × CH₃), 19.43 (2 × CH₃), 29.36 (2 × CH), 34.80 (CH₂), 86.78 (CH), 156.55 (CO), 160.96 (CO) ppm.
MS (EI): *m*/*z* = 216 (1, MH⁺), 172 (1), 128 (1), 144 (1), 115 (1), 99 (20), 73 (30), 57 (100), 43 (40).

### Beispiel 12: Synthese von L-Menthyl-N-2-methoxyethyloxamat

Analog zur Synthese von L-Menthyl-N-methyloxamat wurde ausgehend von L-Menthylmethyloxalat das L-Menthyl-N-2-methoxyethyloxamat erhalten. 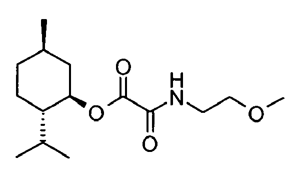 ¹H-NMR (400 MHz, CDCl₃, TMS): δ = 0.76 (d, J = 7.0 Hz, 3 H), 0.85-0.96 (m, 1 H), 0.90 (d, J = 7.0 Hz, 3 H), 0.93 (d, J = 6.5 Hz, 3 H), 1.02-1.22 (m, 2 H), 1.45-1.64 (m, 2 H), 1.66-1.76 (m, 2 H), 1.90 (dhept, J = 2.8 Hz, J = 7.0 Hz, 1 H), 1.98-2.05 (m, 1 H), 3.38 (s, 3 H), 3.48-3.57 (m, 4 H), 4.83 (dt, J = 11.0 Hz, J = 4.5 Hz, 1 H), 7.42 (s, br., NH) ppm.
¹³C-NMR (100 MHz, CDCl₃, TMS): δ = 16.15 (CH₃), 20.64 (CH₃), 21.94 (CH₃), 23.30 (CH₂), 26.10 (CH), 31.49 (CH), 34.03 (CH₂), 39.64 (CH₂), 40.27 (CH₂), 46.56 (CH), 58.85 (CH₃), 70.49 (CH₂), 77.79 (CH), 156.87 (CO), 160.17 (CO) ppm.
MS (EI): *m*/*z =* 286 (1, MH⁺), 253 (1), 158 (1), 147 (15), 139 (17), 97 (17), 83 (100), 69 (30), 55 (43).

### Anwendungsbeispiel 1: Kühlwirkung

Die Verbindungen gemäß den Beispielen 1 bis 12 wurden auf ihre sensorischen Eigenschaften, insbesondere ihre Kühlwirkung getestet. Dafür wurden sie jeweils in einer bestimmten Endkonzentration in einer aus Sucrose (Saccharose) und Wasser bereiteten Masse (Konditorenfondant, Lieferant Nordzucker AG, Nordstemmen) gelöst und in einer Runde von Experten bewertet. Die sensorischen Eindrücke wurden notiert und die Kühlwirkung anhand einer Skala von 1 (keine Kühlwirkung) bis 9 (extrem starke Kühlwirkung) bewertet. Die Ergebnisse waren in jedem Falle überzeugend.

Das Profil des von L-Menthyl-*N*-methyloxamats (Beispiel 2) bei einer Konzentration von 0,05 Gew.-%, bezogen auf die Gesamtzubereitung wurde wie folgt beschrieben: sehr schwach bitter, Kühlwirkung 6.

### Anwendungsbeispiel 2: Aromamischung zur Erreichung einer Kühlwirkung

| **Inhaltsstoff** | **Anteil in Gew-%** |
|---|---|
| L-Menthyl-*N*-methyloxamat (Beispiel 2) | 25 |
| L-Menthyllactat (Frescolat ML, Symrise) | 50 |
| L-Menthyl-3-oxobutyrat | 15 |
| O-L-Menthyl-O'-(2-hydroxyethyl)carbonat (Frescolat MGC, Symrise) | 10 |

Durch Mischung der Komponenten wird eine stark kühlende, jedoch nahezu geschmacks- und geruchslose, bei Raumtemperatur (20 °C) flüssige Aromamischung erhalten.

### Anwendungsbeispiel 2A: Aromamischung zur Erreichung einer Kühlwirkung

| **Inhaltsstoff** | **Anteil in Gew-%** |
|---|---|
| L-Menthyl-N-methyloxamat (Beispiel 2) | 14 |
| Menthancarbonsäure-N-(4-cyanomethylphenyl)amid | 1 |
| Pfefferminzöl | 30 |
| I-Menthol | 30 |
| L-Menthyllactat (Frescolat ML, Symrise) | 15 |
| Propylenglycol | 10 |

Durch Mischung der Komponenten wird eine stark kühlende, jedoch nahezu geschmacks- und geruchslose Aromamischung erhalten.

### Anwendungsbeispiel 3: Aromamischung zum Erreichen einer Kühlwirkung

| **Inhaltsstoff** | **Anteil in Gew-%** |
|---|---|
| L-Menthyl-N-methyloxamat (Beispiel 2) | 7,5 |
| L-Menthancarbonsäure-N-ethylamid (WS3, z.B. Millenium) | 5 |
| L-Menthyllactat (Frescolat ML, Symrise) | 32,5 |
| O-L-Menthyl-O'-(2-hydroxyethyl)carbonat (Frescolat MGC, Symrise) | 5 |
| Propylenglycol | 50 |

Durch Mischung der Komponenten wird eine stark kühlende, nahezu geschmacks- und geruchslose, bei Raumtemperatur (20 °C) flüssige Aromamischung erhalten.

### Anwendungsbeispiel 4: Aromamischung zum Erreichen einer Aroma- und Kühlwirkung

| **Inhaltsstoff** | **Anteil in Gew-%** |
|---|---|
| L-Menthyl-N-methyloxamat (Beispiel 2) | 15 |
| Pfefferminzöl | 10 |
| 1-Menthol | 40 |
| L-Menthyllactat (Frescolat ML, Symrise) | 25 |
| O-L-Menthyl-O'-(2-hydroxyethyl)carbonat (Frescolat MGC, Symrise) | 10 |

Durch Mischung der Komponenten wird eine stark kühlende, stark nach Pfefferminz riechende Aromamischung erhalten.

### Anwendungsbeispiel 5: Aromamischung zum Erreichen einer Kühlwirkung mit gleichzeitigem Tingling-Effekt

| **Inhaltsstoff** | **Anteil in Gew-%** |
|---|---|
| L-Menthyl-N-methyloxamat (Beispiel 2) | 15 |
| Lösung aus 10 Gew.-% trans-Pellitorin in Propylenglycol/Pfefferminzöl 1:1 | 10 |
| L-Menthyllactat (Frescolat ML, Symrise) | 65 |
| O-L-Menthyl-O'-(2-hydroxyethyl)carbonat (Frescolat MGC, Symrise) | 10 |

Durch Mischung der Komponenten wird eine stark kühlende Aromamischung erhalten, welche speichelanregend ist und einen Tingling-Effekt verursacht.

### Anwendungsbeispiel 6: Verwendung in Form einer Aromamischung in einer Zahnpasta

| **Teil** | **Inhaltsstoff** | **I (Gew.-%)** | **II (Gew.-%)** |
|---|---|---|---|
| A | demineralisiertes Wasser | 22,00 | 22,00 |
| | Sorbitol (70%) | 45,00 | 45,00 |
| | Solbrol^{®} M, Natriumsalz (Bayer AG, p-Hydroxybenzoesäurealkylester) | 0,15 | 0,15 |
| | Trinatriumphosphat | 0,10 | 0,10 |
| | Saccharin, 450 fach | 0,20 | 0,20 |
| | Natriummonofluorphosphat | 1,12 | 1,12 |
| | Polyethylenglycol 1500 | 5,00 | 5,00 |
| B | Sident 9 (abrasives Siliciumdioxid) | 10,00 | 10,00 |
| | Sident 22 S (verdickendes Siliciumdioxid) | 8,00 | 8,00 |
| | Natriumcarboxymethylcellulose | 0,90 | 0,90 |
| | Titandioxid | 0,50 | 0,50 |
| C | demineralisertes Wasser | 4,53 | 4,53 |
| | Natriumlaurylsulfat | 1,50 | 1,50 |
| D | Aromamischung aus Anwendungsbeispiel 2 | 1,00 | - |
| | Aromamischung aus Anwendungsbeispiel 2A | - | 1,00 |

Die Inhaltsstoffe der Teile A und B wurden jeweils für sich vorgemischt und zusammen unter Vakuum bei 25 - 30°C jeweils 30 min lang gut verrührt. Teil C wurde vorgemischt und zu A und B gegeben; D wurde hinzugefügt und die Mischung unter Vakuum bei 25 - 30°C weitere 30 min gut verrührt. Nach Entspannung war die Zahnpasta fertig und konnte abgefüllt werden.
Bei der Verwendung der so hergestellten Zahnpasten konnte eine deutliche Kühlwirkung festgestellt werden.

### Anwendungsbeispiel 7: Verwendung als Kühlwirkstoff in einem zuckerfreien Kaugummi

| **Teil** | **Inhaltsstoff** | **I (Gew.-%)** | **II (Gew.-%)** |
|---|---|---|---|
| A | Kaugummibase, Company "Jagum T" | 30,00 | 30,00 |
| B | Sorbit, pulverisiert | 39,00 | 38,80 |
| | Isomalt^{®} (Palatinit GmbH) | 9,50 | 9,50 |
| | Xylitol | 2,00 | 2,00 |
| | Mannitol | 3,00 | 3,00 |
| | Aspartam^{®} | 0,10 | 0,10 |
| | Acesulfam^{®} K | 0,10 | 0,10 |
| | Emulgum^{®} (Colloides Naturels, Inc.) | 0,30 | 0,30 |
| C | Sorbitol, 70% | 14,00 | 14,00 |
| | Glycerin | 1,00 | 1,00 |
| D | Spearmint-Pfefferminz-Eucalyptus-Aroma, enthaltend 5 Gew.-% L-Menthyl-*N*-methyloxamat (Beispiel 2) | 1,00 | - |
| | Aromamischung aus Anwendungsbeispiel 2A | - | 1,20 |

Teile A bis D wurden gemischt und intensiv geknetet. Die Rohmasse wurde z.B. in Form von dünnen Streifen zu verzehrsfertigen Kaugummis verarbeitet.
Bei der Verwendung des so hergestellten Kaugummis konnte eine deutliche Kühlwirkung festgestellt werden.

### Anwendungsbeispiel 8: Verwendung als Kühlwirkstoff in einem Mundwasser

| **Teil** | **Inhaltsstoff** | **Einsatz in Gew.-%** |
|---|---|---|
| A | Ethanol | 10,00 |
| | Cremophor^{®} CO 40 (BASF, Detergenz) | 1,00 |
| | Benzoesäure | 0,12 |
| | Pfefferminz-Zitronenmelisse-Aroma, enthaltend 0,4 Gew.-% Pellitorin und 10 Gew.-% L-Menthyl-N-methyloxamat (Beispiel 2) | 0,25 |
| B | demineralisiertes Wasser | 83,46 |
| | Sorbitol, 70% | 5,00 |
| | Natriumsaccharin 450 | 0,07 |
| | L-Blue 5000 e.c., 1% in Wasser (Farbstoff) | 0,10 |

Die Inhaltsstoffe der Teile A und B wurden jeweils für sich gemischt. Teil B wurde langsam in Teil A eingerührt, bis die Mischung homogen war.
Bei der Verwendung des so hergestellten Mundwassers konnte eine deutliche Kühlwirkung festgestellt werden.

### Anwendungsbeispiel 9: Halsbonbons mit flüssig-viskoser Kernfüllung (centrefilled hard candy)

| | I (Gew.-%) | II (Gew.-%) |
|---|---|---|
| **Mischung A (Hülle) (80% der Bonbons)** | | |
| Zucker (Saccharose) | 58,12 | 49,37 |
| Glucosesirup (Feststoffgehalt 80%) | 41,51 | 49,37 |
| Aromamischung aus Anwendungsbeispiel 5 | 0,17 | 0,25 |
| I-Menthol | 0,10 | - |
| Citronenöl | 0,10 | 0,10 |
| Citronensäure | - | 0,91 |
| Total: | 100 | 100 |

| **Mischung B (Kern) (20% der Bonbons)** | | |
|---|---|---|
| High Fructose Maissirup (Gehalt an festen Zuckern 85%, knapp 15% Wasser) | 84,38 | 84,36 |
| Glycerin | 15,0 | 15,0 |
| Lecithin | 0,02 | 0,02 |
| Zimtöl | - | 0,32 |
| Spearmintöl | 0,28 | - |
| Capsaicin | 0,05 | - |
| Vanillylalkohol-n-butylether | - | 0,10 |
| Roter Farbstoff, als 5%ige wässrige Lösung | 0,20 | 0,20 |
| Vanillin | 0,07 | - |
| Total | 100 | 100 |

In Anlehnung an die in US 6,432,441 (dort Beispiel 1) sowie die in US 5,458,894 bzw. US 5,002,791 beschriebenen Verfahren wurden Bonbons mit flüssig-viskosem Kern hergestellt. Beide Mischungen A und B wurden separat zu Basen für Hülle (Mischung A) bzw. Kern (Mischung B) verarbeitet. Die mittels Co-Extrusion erhaltenen gefüllten Halsbonbons wirkten bei betroffenen Personen beim Verzehr gegen Husten, Halsschmerzen und Heiserkeit.

### Anwendungsbeispiel 10: Kaugummi

Die Kaugummibase K2 bestand aus folgenden Zutaten: 28,5% Terpenharz, 33,9% Polyvinylacetat (MW = 14,000), 16,25% hydriertes Pflanzenöl, 5,5% Mono- und Diglyceride, 0,5% Polyisobuten (MW 75,000), 2,0% Butyl Rubber (Isobuten-Isopren-Copolymer), 4,6% amorphes Siliciumdioxid (Wassergehalt ca. 2,5%), 0,05% Antioxidans tert.-Butylhydroxytoluol (BHT), 0,2% Lecitihin, und 8,5% Calciumcarbonat. Die Herstellung der Kaugummibase K2 und der Kaugummis erfolgte analog zu US 6,986,907.

| | I (Gew.-%) | II (Gew.-%) | III (Gew.-%) |
|---|---|---|---|
| Kaugummibase K2 | 25,30 | 27,30 | 26,30 |
| Sorbitol | 61,48 | 59,48 | 61,80 |
| Glycerin | 2,40 | 2,40 | 2,40 |
| Lecithin | 7,00 | 7,00 | 7,00 |
| Aspartam | 0,14 | 0,14 | 0,14 |
| Verkapseltes Aspartam | 0,68 | 0,68 | 0,68 |
| Menthol, sprühgetrocknet | 0,50 | - | - |
| Kirscharoma, sprühgetrocknet | - | 1,20 | - |
| Aromamischung aus Anwendungsbeispiel 4, sprühgetrocknet | 1,50 | 1,80 | - |
| Aromamischung aus Anwendungsbeispiel 3 | 1,00 | - | 1,68 |

Die Kaugummis der Rezeptur (I) und (II) wurden als Streifen, die der Rezeptur (III) als Pellets ausgeformt.
Bei der Verwendung des so hergestellten Kaugummis konnte eine deutliche Kühlwirkung festgestellt werden.

### Anwendungsbeispiel 11: Gelatinekapsel zum Direktverzehr

| | I (Gew.-%) | II (Gew.-%) | III (Gew.-%) |
|---|---|---|---|
| Gelatinehülle: | | | |
| Glycerin | 2,014 | 2,014 | 2,014 |
| Gelatine 240 Bloom | 7,91 | 7,91 | 7,91 |
| Sucralose | 0,065 | 0,065 | 0,065 |
| Allura Rot | 0,006 | 0,006 | 0,006 |
| Brillantblau | 0,005 | 0,005 | 0,005 |
| | | | |

| Kernzusammensetzung: | | | |
|---|---|---|---|
| Pflanzenöl-Triglycerid (Kokosöl - Fraktion) | 79,39 | 68,40 | 58,25 |
| Zimt-Anis-Aroma | 10,00 | 20,90 | - |
| Aroma X | - | - | 29,95 |
| Neotam und Aspartam | 0,01 | 0,05 | - |
| Sucralose | 0,22 | 0,30 | 0,70 |
| Aromamischung aus Anwendungsbeispiel 5 | 0,33 | - | - |
| Aromamischung aus Anwendungsbeispiel 3 | - | 0,20 | 0,60 |
| L-Menthyl-*N*-methyloxamat (Beispiel 2) | - | 0,05 | - |
| (-)-Menthonglycerinacetal (Frescolat MGA) | - | 0,10 | 0,40 |
| Vanillin | 0,05 | - | 0,10 |

Aroma X hatte dabei folgende Zusammensetzung (Angaben jeweils in Gew.-%): 0,1% Neotam Pulver, 0,05% Aspartam, 29,3% Pfefferminzöl arvensis, 29,3% Pfefferminz piperita Öl Willamette, 2,97% Sucralose, 2,28% Triacetin, 5,4% Diethyltartrat, 12,1% Pfefferminzöl yakima, 0,7% Ethanol, 3,36% 2-Hydroxyethylmenthylcarbonat, 3,0% 2-Hydroxypropylmenthylcarbonat, 5,77% D-Limonen, 5,67% L-Menthylacetat.
Die zum Direktverzehr geeigneten Gelatinekapseln I, II, III wurden gemäß WO 2004/050069 hergestellt und hatten jeweils einen Durchmesser von 5 mm, das Gewichtsverhältnis von Kernmaterial zu Hüllmaterial lag bei 90 : 10. Die Kapseln öffneten sich jeweils im Mund innerhalb von weniger als 10 Sekunden und lösten sich vollständig innerhalb von weniger als 50 Sekunden auf. Beim Kauen und Verzehr der so hergestellten Gelatinekapseln konnte eine deutliche Kühlwirkung festgestellt werden.

### Anwendungsbeispiel 12: Kaubonbon

| | | Gew-% |
|---|---|---|
| Wasser | | 7,80 % |
| Zucker | Raffinade C4 | 42,10 % |
| Glucose Sirup | Dextrose 40 | 37,30 % |
| gehärtetes Pflanzenfett | Schmelzpunkt 32-36°C | 6,60 % |
| Lecithin | Emulgator (Sojalecithin) | 0,30 % |
| Gelatine | Schweinegelatine | 0,80 % |
| Fondant | Typ - S30 | 4,80 % |
| Himbeeraroma | | 0,22 % |
| Aromamischung aus Anwendungsbeispiel 3 | | 0,08 % |

| | | |
|---|---|---|
| Herstellungshinweise: a) Gelatine mit Wasser (1,8 fache Menge der Gelatine) bei 70°C 2 Stunden quellen lassen; b) Zucker, Sirup, Wasser, Fett und Lecithin auf 123°C kochen; c) Gelatinelösung langsam mit dem Kochansatz vermischen; d) Aroma aus Beispiel 2 und optional Farbe unterrühren; e) resultierende Masse auf einem Kühltisch auf ca. 70°C temperieren, danach Fondant zugeben und auf einer Ziehmaschine ca. 3 Minuten belüften; f) Kaubonbonmasse anschließend schneiden und verpacken. | | |

Beim Verzehr der Kaubonbons wird während des Kauens ein frischer, kühlender Himbeergeschmack wahrgenommen.

### Anwendungsbeispiel 13: Extrudat

| | | |
|---|---|---|
| Glukosesirup, sprühgetrocknet (DE-Wert: 31-34) | Glucidex IT33W (Firma Roquette) | 62,0 % |
| Maltodextrin (DE-Wert: 17-20) | (Firma Cerestar) | 28,4 % |
| Emulgator Monomuls | Emulgator auf der Basis von gehärtetem Palmöl; Schmelzpunkt: 64°C, (Firma Grünau) | 1,8 % |
| Dextrosemonohydrat (DE-Wert: 99,5) | Dextrose, kristallwasserhaltig (Firma Cerestar) | 1,8 % |
| Wasser | | 2,0 % |
| Orangen-Vanillearoma | | 3,2 % |
| Aromamischung aus Anwendungsbeispiel 4 | | 0,8% |

| | | |
|---|---|---|
| Herstellungshinweis (siehe auch WO 03/092412): Alle Bestandteile wurden gemischt und per Einpunktdosierung in einen Doppelschneckenextruder gefördert. Die Extrusionstemperaturen lagen zwischen 100 und 120°C, der spezifische Energieeintrag lag bei 0,2 kWh/kg. Die aus der mit 1 mm Bohrungen versehene Düsenplatte des Extruders austretenden Stränge wurden unmittelbar nach Austritt aus den Düsen durch rotierende Messer auf Partikel mit ca. 1 mm Durchmesser geschnitten. | | |

### Anwendungsbeispiel 14: Wirbelschichtgranulate

In einer Granulierapparatur des in EP 163 836 dargestellten Typs (mit den folgenden Merkmalen: Durchmesser Anströmboden: 225 mm, Sprühdüse: Zweistoffdüse; Sichtender Austrag: Zick-Zack-Sichter; Filter: internes Schlauchfilter) wird eine Lösung bestehend aus 44 Gew.-% Wasser, 8 Gew.% Citronenaroma, 3 Gew.-% Aromamischung aus Anwendungsbeispiel 4, 13 Gew.-% Gummi arabicum und 32 Gew.-% hydrolysierter Stärke (Maltodextrin DE 15-19) sowie etwas grünem Farbstoff granuliert. Die Lösung wird bei einer Temperatur von 32°C in den Wirbelschichtgranulator gesprüht. Zur Fluidisierung des Bettinhaltes wird Stickstoff in einer Menge von 140 kg/h eingeblasen. Die Eintrittstemperatur des Fluidisiergases beträgt 140°C. Die Temperatur des Abgases beträgt 76°C. Als Sichtgas wird ebenfalls Stickstoff in einer Menge von 15 kg/h mit einer Temperatur von 50°C zugeführt. Der Inhalt des Wirbelbettes beträgt ca. 500 g. Die Granulierleistung beträgt ca. 2,5 kg pro Stunde. Man erhält ein frei fließendes Granulat mit einem mittleren Teilchendurchmesser von 360 Mikrometern. Die Granulate sind rund und weisen eine glatte Oberfläche auf. Aufgrund des konstanten Druckverlustes des Filters und des ebenfalls konstant bleibenden Bettinhalts ist von stationären Bedingungen hinsichtlich des Granulationsprozesses auszugehen.

### Anwendungsbeispiel 15: Teebeutel mit Rooibos bzw. schwarzem Tee und Extrudaten aus Auswendungsbeispiel 13 bzw. Granulaten aus Anwendungsbeispiel 14

Jeweils 800 g Rotbuschtee (Rooibos-Tee) wurden einmal mit 33 g der Extrudate aus Anwendungsbeispiel 13 und einmal mit 30 g Granulaten aus Anwendungsbeispiel 14 gemischt, portioniert und anschließend in Teebeutel abgefüllt.

Jeweils 800 g schwarzer Tee (Blattgrad Fannings) wurden einmal mit 33 g der Extrudate aus Anwendungsbeispiel 13 und einmal mit 30 g Granulaten aus Anwendungsbeispiel 14 gemischt, portioniert und anschließend in Teebeutel abgefüllt.

Die in den Anwendungsbeispiel gefundenen Effekte lassen sich sich - gegebenenfalls durch vom Fachmann unschwer vorzunehmende Modifikationen - auf alle Produkte der jeweiligen Produktgruppe, d.h. insbesondere auf Zahnpasten, Kaugummis, Mundwässer, Halsbonbons, Gelatinekapseln, Kaubonbons und Tee in Beuteln übertragen. Dabei ist es für den Fachmann aufgrund der vorliegenden Beschreibung unschwer erkennbar, dass ohne großen Aufwand die erfindungsgemäßen Verbindungen und Mischungen - eventuell unter geringfügigen Modifikationen - untereinander austauschbar sind. Das bedeutet, dass die in den Produkten der Anwendungsbeispiele verwendete erfindungsgemäße Verbindung als Platzhalter auch für die anderen erfindungsgemäßen Verbindungen und Mischungen aufgefasst werden muss. Auch die Konzentration der verwendeten erfindungsgemäßen Verbindung oder Mischung ist für den Fachmann leicht erkenntlich variierbar. Außerdem sind die produktspezifischen weiteren Bestandteile in dem jeweiligen Anwendungsbeispiel für den Fachmann leicht nachvollziehbar ebenfalls gegen weitere produkttypische Bestandteile austauschbar bzw. durch solche ergänzbar. Eine Vielzahl solcher produktspezifischen Bestandteile sind in der oben stehenden Beschreibung offenbart.

### Anwendungsbeispiel 16: Gebrauchsfertiges Mundwasser mit Fluorid und Wirksamkeit gegen Mundgeruch

| | I (%) | II (%) | III (%) |
|---|---|---|---|
| Ethanol | 7,00 | 7,00 | 7,00 |
| Glycerin | 12,00 | 12,00 | 12,00 |
| Na-fluorid | 0,05 | 0,05 | 0,05 |
| Pluronic F-127^{®} (BASF, oberflächenaktive Substanz) | 1,40 | 1,40 | 1,40 |
| Na-phosphatpuffer pH 7,0 | 1,10 | 1,10 | 1,10 |
| Sorbinsäure | 0,20 | 0,20 | 0,20 |
| Na-saccharinat | 0,10 | 0,10 | 0,10 |
| Pfefferminz-Aroma | 0,15 | 0,15 | 0,10 |
| beta-Homocyclocitral | 0,01 | 0,02 | 0,03 |
| Aromamischung aus Anwendungsbeispiel 3 | 0,05 | 0,10 | 0,15 |
| Farbstoff | 0,01 | 0,01 | 0,01 |
| Wasser dest. | Ad 100,00 | Ad 100,00 | Ad 100,00 |

### Anwendungsbeispiel 17: Gel-Zahncreme mit Wirksamkeit gegen Mundgeruch

| | I (%) | II (%) | III (%) |
|---|---|---|---|
| Na-carboxymethylcellulose | 0,40 | 0,40 | 0,40 |
| Sorbitol 70 %, in Wasser | 72,00 | 72,00 | 72,00 |
| Polyethylenglykol (PEG) 1500 | 3,00 | 3,00 | 3,00 |
| Na-saccharinat | 0,07 | 0,07 | 0,07 |
| Na-fluorid | 0,24 | 0,24 | 0,24 |
| p-Hydroxybenzoesäure (PHB)- Ethylester | 0,15 | 0,15 | 0,15 |
| Aroma X (siehe Anwendungsbeispiel 11, oben) | 0,90 | 0,80 | 0,55 |
| Aroma Z | 0,05 | 0,10 | 0,10 |
| Abrasivkieselsäure | 11,00 | 11,00 | 11,00 |
| Verdickungskieselsäure | 6,00 | 6,00 | 6,00 |
| Natriumdodecylsulfat (SDS) | 1,40 | 1,40 | 1,40 |
| Aromamischung aus Anwendungsbeispiel 5 | 0,10 | 0,25 | 0,30 |
| Blaue Mikrokapseln gemäß US 6 506 368 | - | 0,15 | 0,50 |
| Wasser dest. | Ad 100,00 | Ad 100,00 | Ad 100,00 |

Aroma Z: Eugenolacetat, beta-Homocyclocitral, Isoeugenol-methylether, Farnesol (50 : 1 : 30 : 10 (w/w))

### Anwendungsbeispiel 18: Zahncreme gegen Plaque mit Wirksamkeit gegen Mundgeruch

| | I (%) | II (%) | III (%) |
|---|---|---|---|
| Na-carboxymethylcellu lose | 1,00 | 1,00 | 1,00 |
| Glycerin | 12,50 | 12,50 | 12,50 |
| Sorbitol 70 %, in Wasser | 29,00 | 29,00 | 29,00 |
| Na-saccharinat | 0,20 | 0,20 | 0,20 |
| Na-fluorid | 0,22 | 0,22 | 0,22 |
| Azacycloheptan-2,2-diphosphosäure, Di-natriumsalz | 1,00 | 1,00 | 1,00 |
| Bromchlorophen | 0,10 | 0,10 | 0,10 |
| Pfefferminz-Aroma | 0,90 | 1,00 | 1,00 |
| Aromamischung aus Anwendungsbeispiel 4 | 0,10 | 0,20 | 0,30 |
| Petersiliensamenöl | 0,15 | 0,10 | 0,05 |
| Abrasivkieselsäure | 15,00 | 15,00 | 15,00 |
| Verdickungskieselsäure | 5,00 | 5,00 | 5,00 |
| Natriumdodecylsulfat (SDS) | 1,50 | 1,50 | 1,50 |
| Wasser dest. | Ad 100,00 | Ad 100,00 | Ad 100,00 |

### Anwendungsbeispiel 19: Zahncreme gegen empfindliche Zähne

| | I (%) | II (%) | III (%) |
|---|---|---|---|
| Na-carboxymethylcellu lose | 0,70 | 0,70 | 0,70 |
| Xanthan Gum | 0,50 | 0,50 | 0,50 |
| Glycerin | 15,00 | 15,00 | 15,00 |
| Sorbitol 70 %, in Wasser | 12,00 | 12,00 | 12,00 |
| K-nitrat | 5,00 | 5,00 | 5,00 |
| Na-monofluorphosphat | 0,80 | 0,80 | 0,80 |
| PHB-Methylester | 0,15 | 0,15 | 0,15 |
| PHB-Propylester | 0,05 | 0,05 | 0,05 |
| Na-saccharinat | 0,20 | 0,20 | 0,20 |
| Aroma X (siehe Anwendungsbeispiel 11, oben) | 1,00 | 0,90 | 0,65 |
| Aromamischung aus Anwendungsbeispiel 3 | 0,10 | 0,25 | 0,45 |
| Ca-carbonat | 35,00 | 35,00 | 35,00 |
| Siliciumdioxid | 1,00 | 1,00 | 1,00 |
| Natriumdodecylsulfat (SDS) | 1,50 | 1,50 | 1,50 |
| Wasser dest. | Ad 100,00 | Ad 100,00 | Ad 100,00 |

### Anwendungsbeispiel 20: Non-stick Kaugummi

Die Kaugummibase K1 bestand aus 2,0% Butyl Rubber (Isobuten-Isopren-Copolymer, MW 400000), 6,0% Polyisobuten (MW = 43800), 43,5% Polyvinylacetat (MW = 12000), 31,5% Polyvinylacetat (MW = 47000), 6,75% Triacetin und 10,25% Calciumcarbonat. Die Herstellung der Kaugummibase K1 und der Kaugummis kann analog zu US 5,601,858 erfolgen.

| | I (Gew.-%) | II (Gew.-%) | III (Gew.-%) |
|---|---|---|---|
| Kaugummibase K1 | 26,00 | 26,00 | 26,00 |
| Triacetin | 0,25 | 0,25 | 0,25 |
| Lecithin | 0,50 | 0,50 | 0,50 |
| Sorbitol, kristallin | Ad 100 | Ad 100 | Ad 100 |
| Mannitol | 15,30 | 15,20 | 15,10 |
| Glycerin | 12,10 | 12,00 | 11,80 |
| Aspartam | 0,17 | 0,17 | 0,17 |
| Verkapseltes Aspartam | 1,08 | 1,08 | 1,08 |
| Amorphes Silica | 1,00 | 1,00 | 1,00 |
| Baumwollsamenöl | 0,50 | 0,50 | 0,50 |
| Polyoxyethylen-sorbitan-monolaurat (E-432) | 1,00 | 1,00 | 1,00 |
| Verkapseltes Spearmint- Aroma (enthält I-Carvon) | 0,20 | 0,10 | 0,20 |
| Verkapseltes Wintergrün-Aroma (enthält Methylsalicylat) | - | 0,20 | - |
| Aromamischung aus Anwendungsbeispiel 3 | 1,00 | 1,40 | 1,70 |

### Anwendungsbeispiel 21: Aromamischung zur Erreichung einer Kühlwirkung

| **Inhaltsstoff** | I (Gew.-%) | II (Gew.-%) |
|---|---|---|
| L-Menthyl-N-methyloxamat (Beispiel 2) | 75 | 50 |
| Menthancarbonsäure-N-(4-methoxyphenyl)amid [WS12] | 25 | 50 |

Die beiden obigen Kühlwirkstoff-Bausteine (I) und (II) wurden in Dosierung von 0,30 Gew.-%, 0,50 Gew.-% und 0,70 Gew.-% in Kaugummis, zuckerfreie Kaugummis, Zahncremes, Zahngele und Mundwasserkonzentrate eingearbeitet.

### Anwendungsbeispiel 22: Zahncreme und Mundwasser als 2-in-1 Produkt

| | I (wt. %) | II (wt.%) |
|---|---|---|
| Sorbitol | 40,00 | 45,00 |
| Glycerin | 20,00 | 20,00 |
| Ethanol | 5,00 | - |
| Wasser | Ad 100 | Ad 100 |
| Solbrol M, Na-Salz (Methylparaben, Na-Salz) | 0,15 | 0,15 |
| Na-monofluorphosphat | 0,75 | 0,75 |
| Saccharin | 0,20 | 0,20 |
| Sident 9 (abrasives Siliciumdioxid) | 20,00 | 20,00 |
| Sident 22 S (verdickendes Siliciumdioxid) | 2,00 | 2.00 |
| Natriumcarboxymethylcellulose | 0,30 | 0,30 |
| Natrium lauryl sulfat (SDS) | 1,20 | 1,20 |
| Farbe (1% in Wasser) | 0,50 | 0,30 |
| Aroma X aus Anwendungsbeispiel 11 | 0,90 | |
| Aromamischung aus Anwendungsbeispiel 2 | 0,30 | - |
| Aromamischung aus Anwendungsbeispiel 4 | - | 1,00 |

## Patentansprüche

1. (a) Verbindung der Formel (I) oder (b) Mischung umfassend eine, zwei oder mehr Verbindungen der Formel (I) oder (c) Mischung bestehend aus zwei oder mehr Verbindungen der Formel (I) 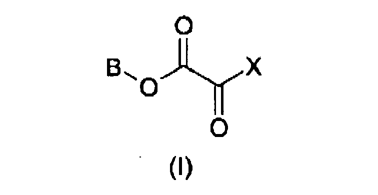 wobei jeweils gilt:
B bedeutet einen Kohlenwasserstoffrest ausgewählt aus der folgenden Gruppe: 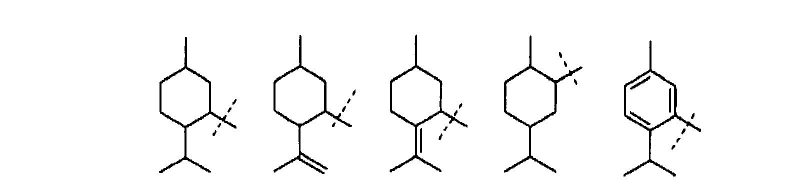 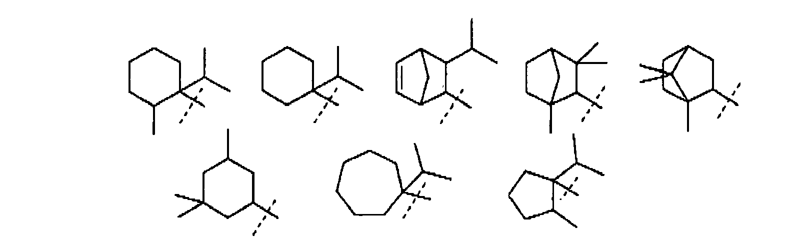 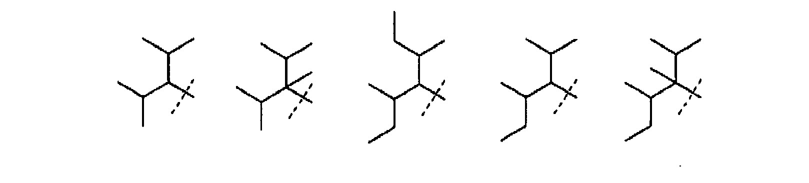 worin die gestrichelte Linie die Bindung markiert, welche den Kohlenwasserstoffrest B mit dem in Formel (I) benachbarten Sauerstoffatom verknüpft,
X bedeutet NR¹R² oder SR³,
wobei jeweils unabhängig voneinander
in NR¹R² die Reste R¹ und R² ausgewählt sind und
in SR³ der Rest R³ ausgewählt ist
aus der Gruppe bestehend aus:
Wasserstoff
und
organischer Rest mit
1 bis 12 C-Atomen
wobei gegebenenfalls in NR¹R² die Reste R¹ und R² unter Fortfall je eines Wasserstoffatomes und unter Ausbildung eines Ringes kovalent miteinander verbunden sind,
mit der Maßgabe, dass im obigen Falle (a) die Verbindung der Formel (I) und im Falle (b) zumindest eine Verbindung der Formel (I) nicht ausgewählt ist aus der Gruppe bestehend aus: 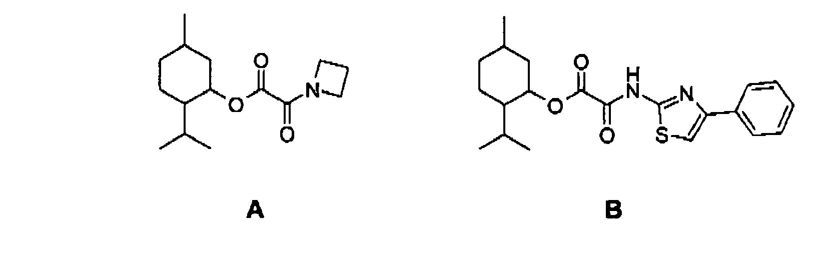 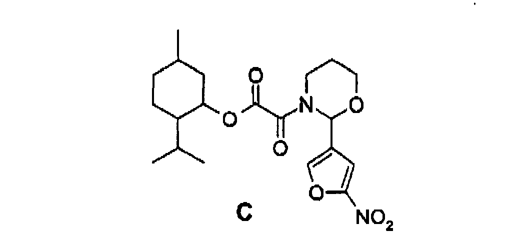
und D (+)-Menthyloxamid.

2. (a) Verbindung der Formel (I) oder (b) Mischung umfassend eine, zwei oder mehr Verbindungen der Formel (I) oder (c) Mischung bestehend aus zwei oder mehr Verbindungen der Formel (I) nach Anspruch 1, wobei im Falle (a) die Verbindung bzw. im Falle (b) oder (c) zumindest eine der Verbindungen in der Mischung ausgewählt ist aus der Gruppe bestehend aus Verbindungen der Formel (I), wobei gilt:
X bedeutet NR¹R² oder SR³,
wobei jeweils unabhängig voneinander
in NR¹R² die Reste R¹ und R² ausgewählt sind und
in SR³ der Rest R³ ausgewählt ist
aus der Gruppe bestehend aus:
Wasserstoff
und
organischer Rest mit 1 bis 12 C-Atomen und gegebenenfalls bis zu 3 Heteroatomen, die jeweils unabhängig voneinander ausgewählt sind aus der Gruppe bestehend aus N, S und O, wobei gegebenenfalls vorhandene Heteroatome aus der Gruppe bestehend aus O und S keine kovalente Bindung zu anderen Heteroatomen aus dieser Gruppe besitzen,
wobei gegebenenfalls eine Anzahl von an C gebundenen Wasserstoffatomen durch Fluor ersetzt ist,
wobei der organische Rest jeweils inklusive aller gegebenenfalls vorhandenen Substituenten nicht mehr Heteroatome aus der Gruppe bestehend aus N, S und O als C-Atome enthält und ausgewählt ist aus der Gruppe bestehend aus substituiertem oder nicht substituiertem
C₁-C₁₀-Alkyl, C₁-C₁₀-Heteroalkyl, C₂-C₁₀-Heterocycloalkyl, C₃-C₁₀-Cycloalkyl, C₄-C₁₀-Cycloalkylatkyl, C₂-C₁₀-Alkenyl, C₃-C₁₀-Cycloalkenyl, C₄-C₁₀-Cycloalkenylalkyl, C₂-C₁₀-Alkinyl, C₅-C₁₀-Cycloalkylalkinyl, C₃-C₁₀-Aryl, C₂-C₁₀-Heteroaryl, C₄-C₁₀-Arylalkyl, C₈-C₁₀-Cycloalkylaryl, C₈-C₁₀-Cycloalkenylaryl, C₅-C₁₀-Cycloalkylheteroaryl, C₈-C₁₀-Heterocycloalkylaryl, C₈-C₁₀-Heterocycloalkenylaryl, C₈-C₁₀-Heterocycloalkenylheteroaryl und C₃-C₁₀₋Heteroarytalkyl,
und bevorzugt ausgewählt ist aus der Gruppe bestehend aus substituiertem oder nicht substituiertem C₁-C₈-Alkyl, C₂-C₈-Heteroalkyl, C₁-C₈-Heterocycloalkyl, C₃-C₈-Cycloalkyl C₄-C₈-Cycloalkylalkyl, C₂-C₈-Alkenyl, C₃-C₈-Cycloalkenyl, C₄-C₈-Cycloalkenylalkyl, C₃-C₆-Aryl, C₂-C₆-Heteroaryl, C₄-C₈-Arylalkyl,
wobei gegebenenfalls in NR¹R² die Reste R¹ und R² unter Fortfall je eines Wasserstoffatomes und unter Ausbildung eines Ringes kovalent miteinander verbunden sind.

3. (a) Verbindung der Formel (I) oder (b) Mischung umfassend eine, zwei oder mehr Verbindungen der Formel (I) oder (c) Mischung bestehend aus zwei oder mehr Verbindungen der Formel (I) nach einem der vorhergehenden Ansprüche, wobei im Falle (a) die Verbindung bzw. im Falle (b) oder (c) zumindest eine der Verbindungen in der Mischung ausgewählt ist aus der Gruppe bestehend aus Verbindungen der Formel (I), wobei gilt:
X bedeutet NHR¹
wobei R¹ ausgewählt ist aus der Gruppe bestehend aus
organischer Rest mit 1 bis 12 C-Atomen und gegebenenfalls bis zu 3 Heteroatomen, die jeweils unabhängig voneinander ausgewählt sind aus der Gruppe bestehend aus N, S und O, wobei gegebenenfalls vorhandene Heteroatome aus der Gruppe bestehend aus O und S keine kovalente Bindung zu anderen Heteroatomen aus dieser Gruppe besitzen, wobei gegebenenfalls eine Anzahl von an C gebundenen Wasserstoffatomen durch Fluor ersetzt ist,
wobei der organische Rest jeweils inklusive aller gegebenenfalls vorhandenen Substituenten nicht mehr Heteroatome aus der Gruppe bestehend aus N, S und O als C-Atome enthält und ausgewählt ist aus der Gruppe bestehend aus substituiertem oder nicht substituiertem
C₁-C₁₀-Alkyl, C₁-C₁₀-Heteroalkyl, C₂-C₁₀-Heterocycloalkyl, C₃-C₁₀-Cycloalkyl, C₄-C₁₀-Cycloalkylalkyl, C₂-C₁₀-Alkenyl, C₃-C₁₀-Cycloalkenyl, C₄-C₁₀-Cycloalkenylalkyl, C₂-C₁₀-Alkinyl, C₅-C₁₀-Cycloalkylalkinyl, C₃-C₁₀-Aryl, C₂-C₁₀-Heteroaryl, C₄-C₁₀-Arylalkyl, C₈-C₁₀-Cycloalkylaryl, C₆-C₁₀-Cycloalkenylaryl, C₅-C₁₀-Cycloalkytheteroaryl, C₈-C₁₀-Heterocycloalkylaryl, C₈-C₁₀-Heterocycloalkenylaryl, C₆-C₁₀-Heterocyctoalkenylheteroaryl und C₃-C₁₀-Heteroarylalkyl,
und bevorzugt ausgewählt ist aus der Gruppe bestehend aus substituiertem oder nicht substituiertem C₁-C₈-Alkyl, C₂-C₈-Heteroalkyl, C₁-C₈-Heterocycloalkyl, C₃-C₈-Cycloalkyl, C₄-C₈-Cycloalkylalkyl, C₂-C₈-Alkenyl, C₃-C₈-Cycloalkenyl, C₄-C₈-Cycloalkenylalkyl, C₃-C₆-Aryl, C₂-C₆-Heteroaryl, C₄-C₈-Arylalkyl.

4. (a) Verbindung der Formel (I) oder (b) Mischung umfassend eine, zwei oder mehr Verbindungen der Formel (I) oder (c) Mischung bestehend aus zwei oder mehr Verbindungen der Formel (I) nach einem der vorhergehenden Ansprüche, wobei im Falle (a) die Verbindung bzw. im Falle (b) oder (c) zumindest eine der Verbindungen in der Mischung ausgewählt ist aus der Gruppe bestehend aus Verbindungen der Formel (I), wobei gilt:
B bedeutet einen Kohlenwasserstoffrest ausgewählt aus der Gruppe bestehend aus:

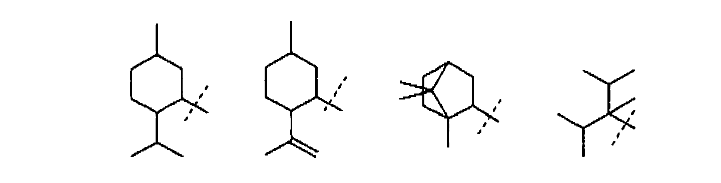

5. (a) Verbindung der Formel (I) oder (b) Mischung umfassend eine, zwei oder mehr Verbindungen der Formel (I) oder (c) Mischung bestehend aus zwei oder mehr Verbindungen der Formel (I) nach einem der vorhergehenden Ansprüche, wobei im Falle (a) die Verbindung bzw. im Falle (b) oder (c) zumindest eine der Verbindungen in der Mischung ausgewählt ist aus der Gruppe bestehend aus Verbindungen der Formel (I), wobei gilt:
B bedeutet einen Kohlenwasserstoffrest ausgewählt aus der Gruppe bestehend aus:
X bedeutet NR¹R²
wobei jeweils unabhängig voneinander
die Reste R¹ und R² ausgewählt sind aus der Gruppe bestehend aus:
Wasserstoff
und
organischer Rest mit 1 bis 12 C-Atomen und gegebenenfalls bis zu 3 Heteroatomen, die jeweils unabhängig voneinander ausgewählt sind aus der Gruppe bestehend aus N, S und O, wobei gegebenenfalls vorhandene Heteroatome aus der Gruppe bestehend aus O und S keine kovalente Bindung zu anderen Heteroatomen aus dieser Gruppe besitzen,
wobei gegebenenfalls eine Anzahl von an C gebundenen Wasserstoffatomen durch Fluor ersetzt ist,
wobei der organische Rest jeweils inklusive aller gegebenenfalls vorhandenen Substituenten nicht mehr Heteroatome aus der Gruppe bestehend aus N, S und O als C-Atome enthält und ausgewählt ist aus der Gruppe bestehend aus substituiertem oder nicht substituiertem C₁-C₄-Alkyl, C₂-C₅-Heteroalkyl, C₃-C₆-Cycloalkyl, C₃-C₈-Aryl, C2-C₆-Heteroaryl, C₄-C₈-Arylalkyl
wobei in NR¹R² gegebenenfalls die Reste R¹ und R² unter Fortfall je eines Wasserstoffatomes kovalent miteinander verbunden sind, wobei der dadurch ausgebildete Ring maximal 7-gliedrig ist,
wobei die gegebenenfalls vorhandenen Substituenten der gegebenenfalls vorhandenen Reste R¹, R² und R³ unabhängig voneinander ausgewählt sind aus der Gruppe bestehend aus:
Methyl, Ethyl, n-Propyl, iso-Propyl, n-Butyl, iso- Butyl, tert.- Butyl, Cyclopropyl, Cyclopentyl, Cyclohexyl, Cyclooctyl, Ethenyl, Propenyl, Ethinyl, Propinyl, Trifluormethyl, Methoxy, Ethoxy, iso-Propoxy, n-Butoxy, iso-Butoxy, tert.-Butoxy, C₃-Cycloalkoxy, C₅-Cycloalkoxy, C₆-Cycloalkoxy, C₈-Cycloalkoxy, -[-O-CH₂-CH₂]ᵥ-Q oder -[-O-CH₂-CHMe-]ᵥ-Q, wobei Q = OH oder CH₃ ist und wobei v = 1 oder 2 bedeuten kann, Acetyl, CO₂Me, CO₂Et, CO₂iso-Pr, CO₂tert.-Bu, Acetyloxy.

6. (a) Verbindung der Formel (I) oder (b) Mischung umfassend eine, zwei oder mehr Verbindungen der Formel (I) oder (c) Mischung bestehend aus zwei oder mehr Verbindungen der Formel (I) nach einem der vorhergehenden Ansprüche, wobei im Falle (a) die Verbindung bzw. im Falle (b) oder (c) zumindest eine der Verbindungen in der Mischung ausgewählt ist aus der Gruppe bestehend aus
Menthyloxamat
Menthyl-N-methyloxamat
Menthyl-N,N-dimethyloxamat
Menthyl-N-ethyloxamat
Menthyl-N,N-diethyloxamat
Menthyl-N-propyloxamat
Menthyt-N,N-dipropytoxamat
Menthyl-N-isopropyloxamat
Menthyl-N,N-diisopropyloxamat
Menthyl-N-cyclopropyloxamat
Menthyl-N-butyloxamat
Morpholin-4-yl-oxo-essigsäure-(1R,2S,5R)-2-isopropyl-5-methyl-cyclohexylester Menthyl-N-(2-methoxyethyl)-oxamat
Menthyl-N-(3-methoxypropyl)-oxamat
Menthyl-N-(2-hydroxyethyl)-oxamat
Menthyl-N-(3-hydroxypropyl)-oxamat.

7. Mischung (b) nach einem der vorangehenden Ansprüche, bestehend aus oder umfassend
(1) eine, zwei oder mehr Verbindungen der Formel (I)
sowie
(2) einen oder mehrere weitere Stoffe mit physiologischer Kühlwirkung, wobei der weitere Stoff bzw. einer, mehrere oder sämtliche der weiteren Stoffe (i) einen geschmacklichen Effekt verursachen oder (ii) keinen geschmacklichen Effekt verursachen, und/oder
(3) einen oder mehrere Aromastoffe ohne physiologische Kühlwirkung und/oder
(4) einen oder mehrere trigeminal oder mundwässernd wirksame Stoffe ohne physiologische Kühlwirkung.

8. Mischung nach Anspruch 7, umfassend
im Bestandteil (2) einen oder mehrere weitere Stoffe mit physiologischer Kühlwirkung ohne geschmacklichen Effekt
und/oder
im Bestandteil (3) einen oder mehrere Aromastoffe ohne physiologische Kühlwirkung und/oder
eine oder mehrere Verbindungen, der oder die unabhängig von einander oder gemeinsam zusätzlich einen geschmacksmodulierenden Effekt und/oder einen trigeminalen und/oder einen mundwässernden Reiz verursachen.

9. Der Ernährung, der Mundhygiene oder dem Genuss dienende Zubereitung oder pharmazeutische oder kosmetische Zubereitung, umfassend eine zum Erreichen einer physiologischen Kühlwirkung auf der Haut und/oder Schleimhaut ausreichende Menge
(i) einer Verbindung oder einer Mischung nach einem der Ansprüche 1 bis 6 oder
(ii) eine Mischung nach einem der Ansprüche 7 bis 8.

10. Verwendung
einer (a) Verbindung der Formel (I) oder (b) Mischung umfassend eine, zwei oder mehr Verbindungen der Formel (I) oder (c) Mischung bestehend aus zwei oder mehr Verbindungen der Formel (I) 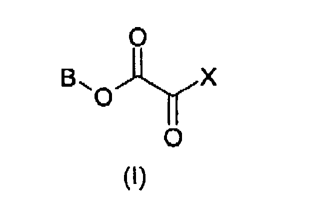 wobei jeweils gilt:
B bedeutet einen Kohlenwasserstoffrest ausgewählt aus der folgenden Gruppe: 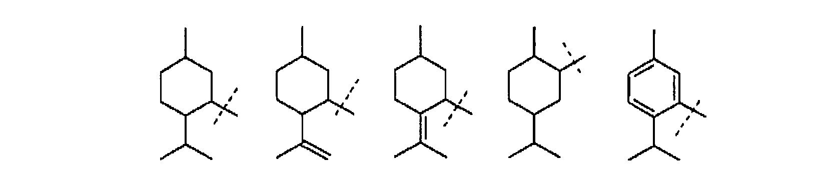 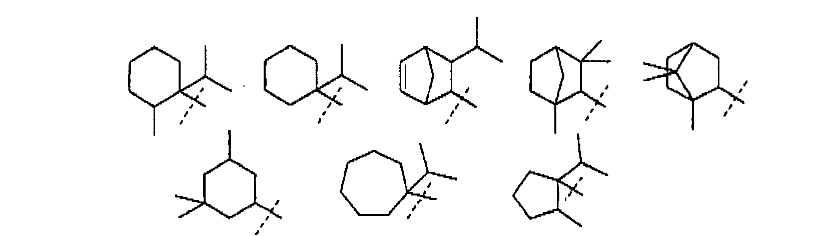 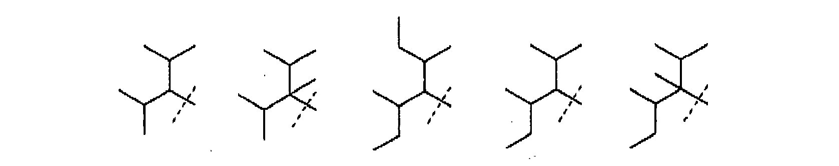 worin die gestrichelte Linie die Bindung markiert, welche den Kohlenwasserstoffrest B mit dem in Formel (I) benachbarten Sauerstoffatom verknüpft,
X bedeutet NR¹R² oder SR³,
wobei jeweils unabhängig voneinander
in NR¹R² die Reste R¹ und R² ausgewählt sind und
in SR³ der Rest R³ ausgewählt ist
aus der Gruppe bestehend aus:
Wasserstoff
und
organischer Rest mit 1 bis 12 C-Atomen der
ausgewählt ist aus der Gruppe bestehend aus substituiertem oder nicht substituiertem Alkyl, Heteroalkyl, Cycloalkyl, Heterocycloalkyl, Cycloalkylalkyl, Alkenyl, Cycloalkenyl, Cycloalkenylalkyl, Alkinyl, Cycloalkylalkinyl, Aryl, Heteroaryl, Arylalkyl, Cycloalkylaryl, Cycloalkenylaryl, Cycloalkylheteroaryl, Heterocycloalkylaryl, Heterocycloalkenylaryl, Heterocycloalkenylheteroaryl und Heteroarylalkyl,
wobei gegebenenfalls in NR¹R² die Reste R¹ und R² unter Fortfall je eines Wasserstoffatomes und unter Ausbildung eines Ringes kovalent miteinander verbunden sind
zum Erzeugen einer Kühlwirkung auf der Haut oder einer Schleimhaut zu anderen als therapeutischen Zwecken.

11. (a) Verbindung der Formel (I) oder (b) Mischung umfassend eine, zwei oder mehr Verbindungen der Formel (I) oder (c) Mischung bestehend aus zwei oder mehr Verbindungen der Formel (I) 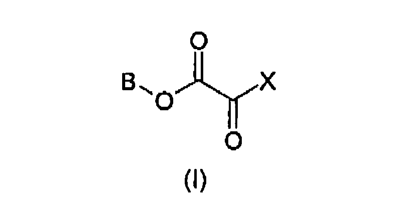 wobei jeweils gilt:
B bedeutet einen Kohlenwasserstoffrest ausgewählt aus der folgenden Gruppe: 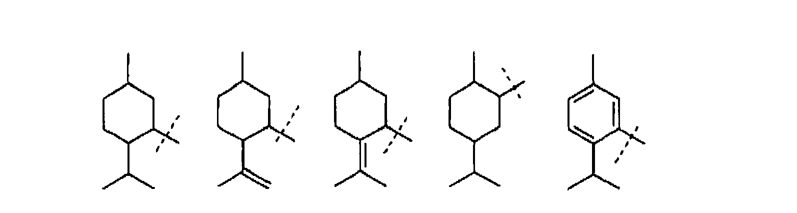 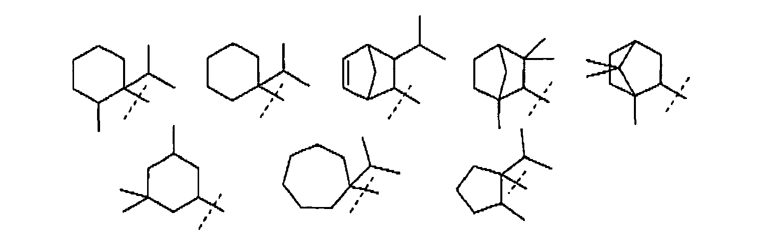 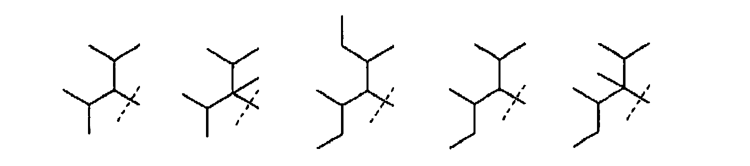
worin die gestrichelte Linie die Bindung markiert, welche den Kohlenwasserstoffrest B mit dem in Formel (I) benachbarten Sauerstoffatom verknüpft,
X bedeutet NR¹R² oder SR³,
wobei jeweils unabhängig voneinander
in NR¹R² die Reste R¹ und R² ausgewählt sind und
in SR³ der Rest R³ ausgewählt ist
aus der Gruppe bestehend aus:
Wasserstoff
und
organischer Rest mit 1 bis 12 Atomen der
ausgewählt ist aus der Gruppe bestehend aus substituiertem oder nicht substituiertem Alkyl, Heteroalkyl, Cycloalkyl, Heterocycloalkyl, Cycloalkylalkyl, Alkenyl, Cycloalkenyl, Cycloalkenylalkyl, Alkinyl, Cycloalkylalkinyl, Aryl, Heteroaryl, Arylalkyl, Cycloalkylaryl, Cycloalkenylaryl, Cycloalkylheteroaryl, Heterocycloalkylaryl, Heterocycloalkenylaryl, Heterocycloalkenylheteroaryl und Heteroarylalkyl,
wobei gegebenenfalls in NR¹R² die Reste R¹ und R² unter Fortfall je eines Wasserstoffatomes und unter Ausbildung eines Ringes kovalent miteinander verbunden sind
Zur Verwendung als Arzneimittel.

12. (a) Verbindung der Formel (I) oder (b) Mischung umfassend eine, zwei oder mehr Verbindungen der Formel (I) oder (c) Mischung bestehend aus zwei oder mehr Verbindungen der Formel (I) nach Anspruch 11 zur Bekämpfung oder Linderung von Husten-, Schnupfen-, Entzündungs-, Halsschmerz- oder Heiserkeitssymptomen.

13. Nicht-therapeutisches Verfahren zum Erreichen einer physiologischen Kühlwirkung auf der Haut und/oder einer Schleimhaut, mit folgendem Schritt:
- Applizieren einer zum Erreichen einer physiologischen Kühlwirkung ausreichenden Menge
(1) einer Verbindung oder einer Mischung nach einem der vorangehenden Ansprüche 1 bis 6 oder
(2) einer Mischung nach einem der Ansprüche 7 oder 8 oder
(3) einer Zubereitung nach Anspruch 9 auf die Haut und/oder eine Schleimhaut.

14. Verfahren zur Herstellung einer Verbindung nach einem der Ansprüche 1 bis 6, umfassend die Schritte:
(1) Bereitstellen eines gemischten Oxalsäureesters der Formel 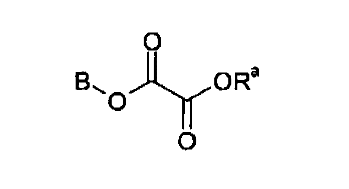 wobei R^{a} einen Alkylrest, vorzugsweise einen C₁-C₄-Alkylrest, bedeutet, oder Oxalsäurehalogenids der Formel 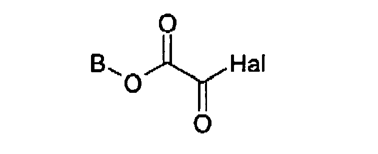 wobei Hal Halogenid bedeutet,
(2) Bereitstellen eines Amins HNR¹R² oder Thiols HSR³ oder eines entsprechenden Salzes,
(3) Umsetzen der bereitgestellten Verbindungen miteinander, so dass eine Verbindung nach einem der Ansprüche 1 bis 6 entsteht.

## Claims

1. (a) Compound of formula (I) or (b) mixture comprising one, two or more compounds of formula (I) or (c) mixture consisting of two or more compounds of formula (I), 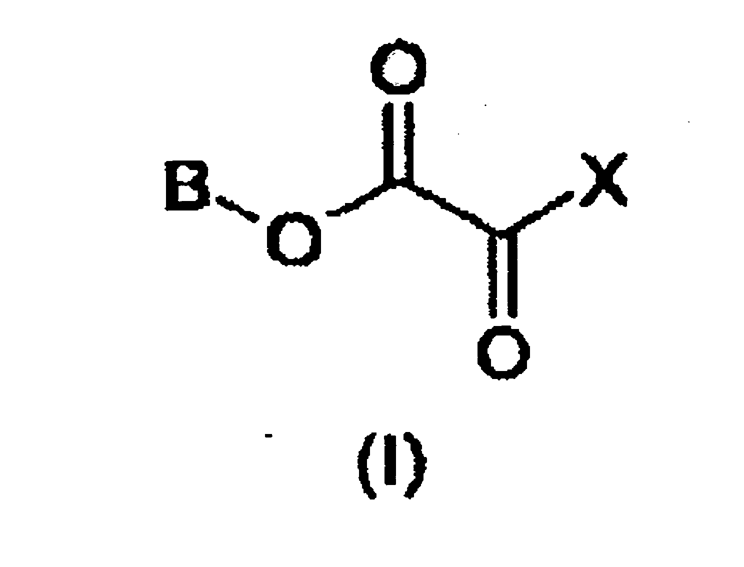 wherein in each case the following applies:
B denotes a hydrocarbon radical selected from the following group: 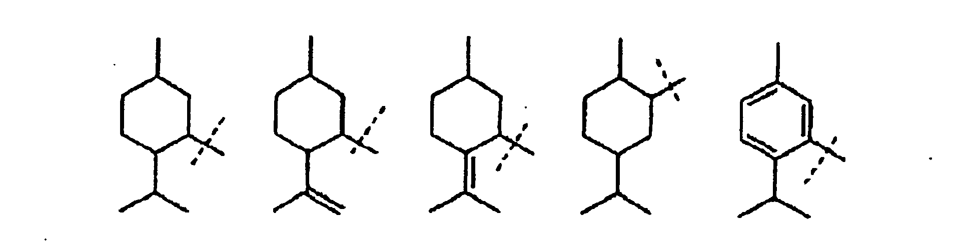 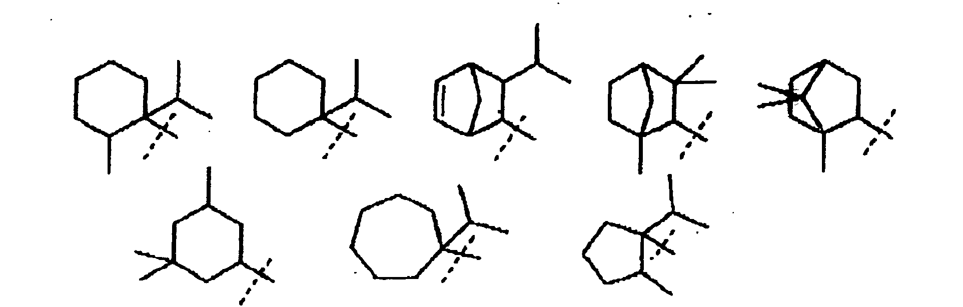 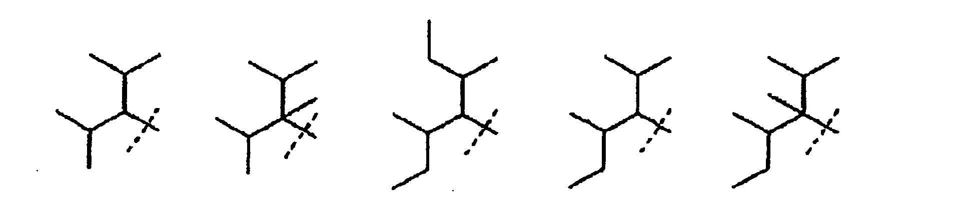 wherein the dashed line indicates the bond which binds the hydrocarbon radical B to the adjacent oxygen atom in formula (I),
X denotes NR¹R² or SR³,
wherein in each case independently of one another
in NR¹R² the radicals R¹ and R² are selected and
in SR³ the radical R³ is selected
from the group consisting of:
hydrogen
and
an organic radical having
1 to 12 C atoms,
wherein optionally in NR¹R² the radicals R¹ and R² by dropping a hydrogen atom in each case and forming a ring are covalently joined to one another, with the proviso that in the above case (a) the compound of formula (I) and in the case (b) at least one compound of formula (I) is not selected from the group consisting of: 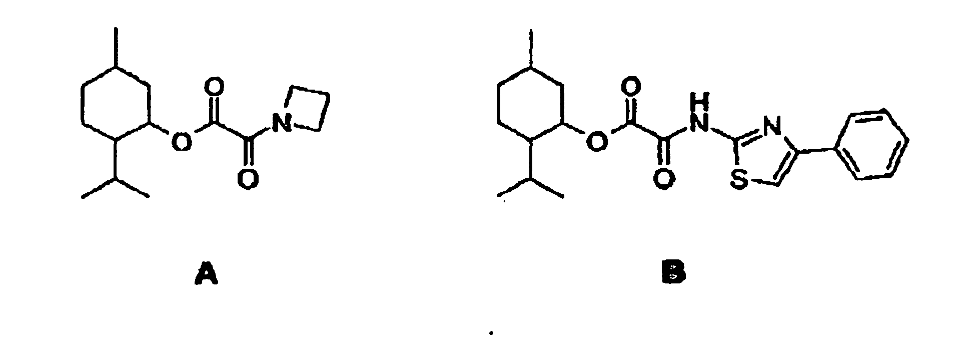 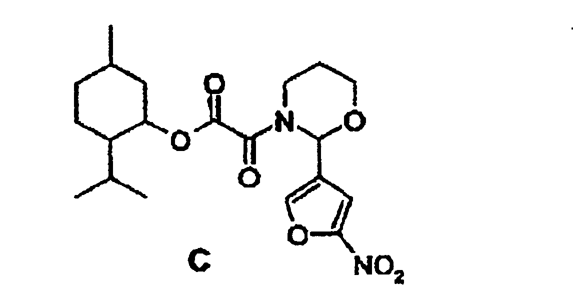
and D (+)•menthyl oxamide.

2. (a) Compound of formula (I) or (b) mixture comprising one, two or more compounds of formula (I) or (c) mixture consisting of two or more compounds of formula (I) according to Claim 1, wherein in the case (a) the compound or in the case (b) or (c) at least one of the compounds in the mixture is selected from the group consisting of compounds of formula (I), wherein the following applies:
X denotes NR¹R² or SR³,
wherein in each case independently of one another
in NR¹R² the radicals R¹ and R² are selected and
in SR³ the radical R³ is selected
from the group consisting of:
hydrogen
and
an organic radical having 1 to 12 C atoms and optionally up to 3 heteroatoms,
which in each case are selected independently of one another from the group consisting of N, S and O, wherein optionally present heteroatoms from the group consisting of O and S do not have a covalent bond to other heteroatoms from this group,
wherein optionally a number of hydrogen atoms bound to C is replaced by fluorine,
wherein the organic radical in each case inclusive of all optionally present substituents contains no more heteroatoms from the group consisting of N, S and O than C atoms and is selected from the group consisting of substituted or
non-substituted
C₁-C₁₀-alkyl, C₁-C₁₀-heteroalkyl, C₂-C₁₀-heterocycloalkyl, C₃-C₁₀-cycloalkyl, C₄-C₁₀-cycloalkylalkyl, C₂-C₁₀-alkenyl, C₃-C₁₀-cycloalkenyl, C₄-C₁₀-cycloalkenylalkyl, C₂-C₁₀-alkinyl, C₅-C₁₀-cycloalkylalkinyl, C₃-C₁₀-aryl, C₂-C₁₀-heteroaryl, C₄-C₁₀-arylalkyl, C₈-C₁₀-cycloalkylaryl, C₈-C₁₀-cycloalkenylaryl, C₅-C₁₀-cycloalkylheteroaryl, C₈-C₁₀-heterocycloalkylaryl, C₈-C₁₀-heterocycloalkenylaryl, C₈C₁₀-heterocycloalkenylheteroaryl and C₃-C₁₀-heteroarylalkyl,
and is preferably selected from the group consisting of substituted or non-substituted C₁-C₈-alkyl, C₂-C₈-heteroalkyl, C₁-C₈-heterocycloalkyl, C₃-C₈-cycloalkyl, C₄-C₈-cycloalkylalkyl, C₂-C₈-alkenyl, C₃-C₈-cycloalkenyl, C₄-C₈-cycloalkenylalkyl, C₃-C₆-aryl, C₂-C₆-heteroaryl, C₄-C₈-arylalkyl,
wherein optionally in NR¹R² the radicals R¹ and R² by dropping a hydrogen atom in each case and forming a ring are covalently joined to one another.

3. (a) Compound of formula (I) or (b) mixture comprising one, two or more compounds of formula (I) or (c) mixture consisting of two or more compounds of formula (I) according to any one of the preceding claims, wherein in the case (a) the compound or in the case (b) or (c) at least one of the compounds in the mixture is selected from the group consisting of compounds of formula (I), wherein the following applies:
X denotes NHR¹,
wherein R¹ is selected from the group consisting of
an organic radical having 1 to 12 C atoms and optionally up to 3 heteroatoms,
which in each case are selected independently of one another from the group consisting of N, S and O, wherein optionally present heteroatoms from the group consisting of O and S do not have a covalent bond to other heteroatoms from this group,
wherein optionally a number of hydrogen atoms bound to C is replaced by fluorine,
wherein the organic radical in each case inclusive of all optionally present substituents contains no more heteroatoms from the group consisting of N, S and O than C atoms and is selected from the group consisting of substituted or non-substituted
C₁-C₁₀-alkyl, C₁-C₁₀-heteroalkyl, C₂-C₁₀-heterocycloalkyl, C₃-C₁₀-cycloalkyl, C₄₋ C₁₀-cycloalkylalkyl, C₂-C₁₀-alkenyl, C₃-C₁₀-cycloalkenyl, C₄-C₁₀-cycloalkenylalkyl, C₂-C₁₀-alkinyl, C₅-C₁₀-cycloalkylalkinyl, C₃-C₁₀-aryl, C₂-C₁₀-heteroaryl, C₄-C₁₀-arylalkyl, C₈-C₁₀-cycloalkylaryl, C₈-C₁₀-cycloalkenylaryl, C₅-C₁₀-cycloalkylheteroaryl, C₈-C₁₀-heterocycloalkylaryl, C₈-C₁₀-heterocycloalkenylaryl, C₈-C₁₀-heterocycloalkenylheteroaryl and C₃-C₁₀-heteroarylalkyl,
and is preferably selected from the group consisting of substituted or non-substituted C₁-C₈-alkyl, C₂-C₈-heteroalkyl, C₁-C₈-heterocycloalkyl, C₃-C₈-cycloalkyl, C₄-C₈-cycloalkylalkyl, C₂-C₈-alkenyl, C₃-C₈-cydoalkenyl, C₄-C₈-cycloalkenylalkyl, C₃-C₆-aryl, C₂-C₆-heteroaryl, C₄-C₈-arylalkyl

4. (a) Compound of formula (I) or (b) mixture comprising one, two or more compounds of formula (I) or (c) mixture consisting of two or more compounds of formula (I) according to any one of the preceding claims, wherein in the case (a) the compound or in the case (b) or (c) at least one of the compounds in the mixture is selected from the group consisting of compounds of formula (I), wherein the following applies:
B denotes a hydrocarbon radical selected from the group consisting of:

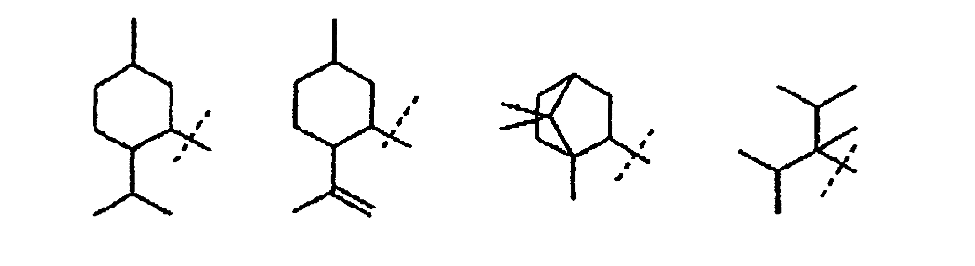

5. (a) Compound of formula (I) or (b) mixture comprising one, two or more compounds of formula (I) or (c) mixture consisting of two or more compounds of formula (I) according to any one of the preceding claims, wherein in the case (a) the compound or in the case (b) or (c) at least one of the compounds in the mixture is selected from the group consisting of compounds of formula (I), wherein the following applies:
B denotes a hydrocarbon radical selected from the group consisting of:
L-menthyl, D-menthyl or rac-menthyl,
X denotes NR¹R²,
wherein in each case independently of one another
the radicals R¹ and R² are selected from the group consisting of:
hydrogen
and
an organic radical having 1 to 12 C atoms and optionally up to 3 heteroatoms,
which in each case are selected independently of one another from the group consisting of N, S and O, wherein optionally present heteroatoms from the group consisting of O and S do not have a covalent bond to other heteroatoms from this group,
wherein optionally a number of hydrogen atoms bound to C is replaced by fluorine,
wherein the organic radical in each case inclusive of all optionally present substituents contains no more heteroatoms from the group consisting of N, S and O than C atoms and is selected from the group consisting of substituted or non-substituted C₁-C₄-alkyl, C₂-C₅-heteroalkyl, C₃-C₈-cycloalkyl, C₃-C₈-aryl, C₂-C₆-heteroaryl, C₄-C₈-arylalkyl,
wherein in NR¹R² optionally the radicals R¹ and R² by dropping a hydrogen atom in each case are covalently joined to one another, wherein the ring formed in this way is at most a seven-membered ring,
wherein the optionally present substituents or optionally present radicals R¹, R² and R³ independently of one another are selected from the group consisting of:
methyl, ethyl, n-propyl, iso-propyl, n-butyl, iso-butyl, tert.-butyl, cyclopropyl, cyclopentyl, cyclohexyl, cyclooctyl, ethenyl, propenyl, ethinyl, propinyl, trifluoromethyl, methoxy, ethoxy, iso-propoxy, n-butoxy, iso-butoxy, tert.-butoxy, C₃-cycloalkoxy, C₅-cycloalkoxy, C₆-cydoalkoxy, C₈-cycloalkoxy, -[O-CH₂-CH₂-]v-Q or -[O-CH₂-CHMe-]v-Q, wherein Q is = OH or CH₃, and wherein v can denote = 1 or 2, acetyl, CO₂Me, CO₂Et, CO₂iso-Pr, CO₂tert.-Bu, acetyloxy.

6. (a) Compound of formula (I) or (b) mixture comprising one, two or more compounds of formula (I) or (c) mixture consisting of two or more compounds of formula (I) according to any one of the preceding claims, wherein in the case (a) the compound or in the case (b) or (c) at least one of the compounds in the mixture is selected from the group consisting of:
menthyl oxamate
menthyl-N-methyloxamate
menthyl-N, N-dimethyloxamate
menthyl-N-ethyloxamate
menthyl-N,N-diethyloxamate
menthyl-N-propyloxamate
menthyl-N,N-dipropyloxamate
menthyl-N-isopropyloxamate
menthyl-N,N-diisopropyloxamate
menthyl-N-cyclopropyloxamate
menthyl-N-butyloxamate
morpholine-4-yl-oxoacetic acid-(1R,2S,5R)-2-isopropyl-5-methylcyclohexylester
menthyl-N-(2-methoxyethyl)-oxamate
menthyl-N-(3-methoxypropyl)-oxamate
menthyl-N-(2-hydroxyethyl)-oxamate
menthyl-N-(3-hydroxypropyl)-oxamate.

7. Mixture (b) according to any one of the preceding claims, consisting of or comprising
(1) one, two or more compounds of formula (I)
and
(2) one or more further substances with a physiological cooling effect, wherein the further substance or one, a plurality of or all of the further substances (i) produce a flavourful effect or (ii) do not produce any flavourful effect
and/or
(3) one or a plurality of flavouring substances with no physiological cooling effect
and/or
(4) one or a plurality of trigeminally or mouthwateringly effective substances with no physiological cooling effect.

8. Mixture according to Claim 7, comprising
in element (2) one or a plurality of further substances with a physiological cooling effect and no flavourful effect
and/or
in element (3) one or a plurality of flavouring substances with no physiological cooling effect
and/or
one or a plurality of compounds which independently of one another or together additionally produce a taste-modulating effect and/or a trigeminal and/or a mouthwatering stimulus.

9. Preparation for a nutritional purpose, an oral hygiene purpose or to be enjoyed, or pharmaceutical or cosmetic preparation, comprising a quantity, sufficient to obtain a physiological cooling effect on the skin and/or on a mucuous membrane,
(i) of a compound or of a mixture according to any one of Claims 1 to 6 or
(ii) a mixture according to either of Claims 7 and 8,

10. Use of a (a) compound of formula (I) or (b) mixture comprising one, two or more compounds of formula (I) or (c) mixture consisting of two or more compounds of formula (I), 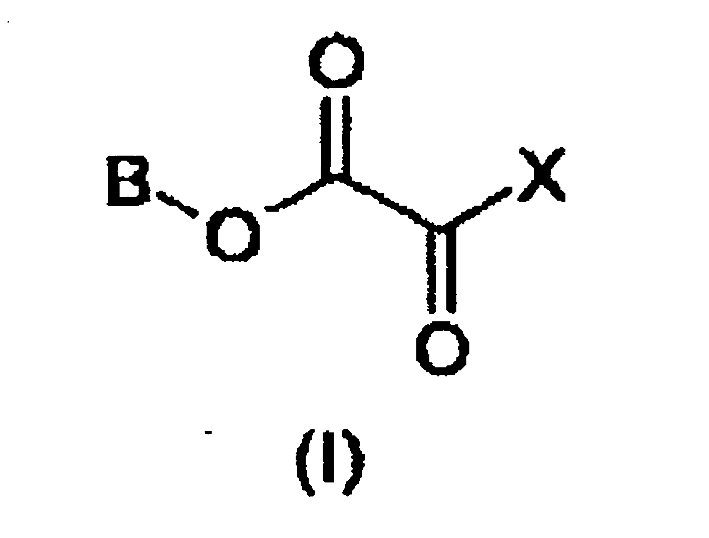 wherein in each case the following applies:
B denotes a hydrocarbon radical selected from the following group: 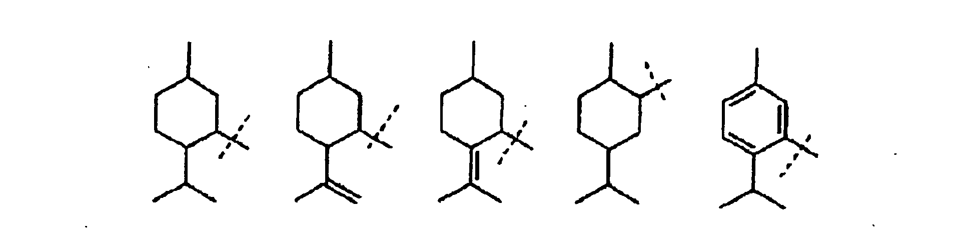 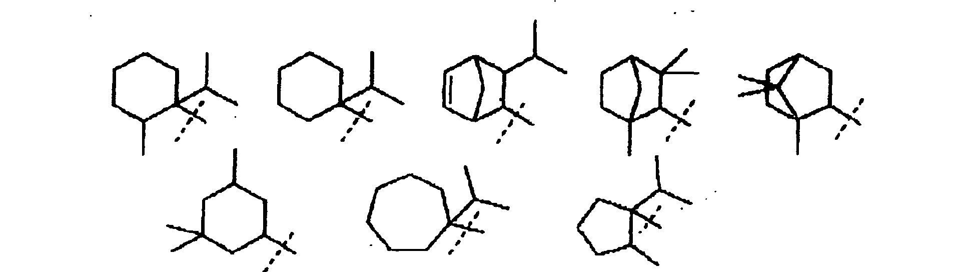 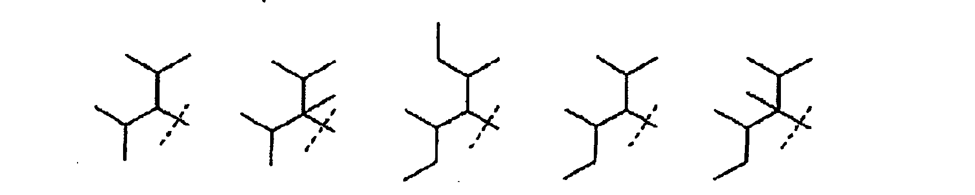 wherein the dashed line indicates the bond which binds the hydrocarbon radical B to the adjacent oxygen atom in formula (I),
X denotes NR¹R² or SR³,
wherein in each case independently of one another
in NR¹R² the radicals R¹ and R² are selected and
in SR³ the radical R³ is selected
from the group consisting of:
hydrogen
and
an organic radical having 1 to 12 C atoms which
is selected from the group consisting of substituted or non-substituted alkyl, heteroalkyl, cycloalkyl, heterocycloalkyl, cycloalkylalkyl, alkenyl, cycloalkenyl, cycloalkenylalkyl, alkinyl, cycloalkylalkinyl, aryl, heteroaryl, arylalkyl, cycloalkylaryl, cycloalkenylaryl, cycloalkylheteroaryl, heterocycloalkylaryl, heterocycloalkenylaryl, heterocycloalkenylheteroaryl and heteroarylalkyl,
wherein optionally in NR¹R² the radicals R¹ and R² by dropping a hydrogen atom in each case and forming a ring are covalently joined to one another to produce a cooling effect on the skin or on a mucuous membrane for purposes other than therapeutic.

11. (a) Compound of formula (I) or (b) mixture comprising one, two or more compounds of formula (I) or (c) mixture consisting of two or more compounds of formula (I), 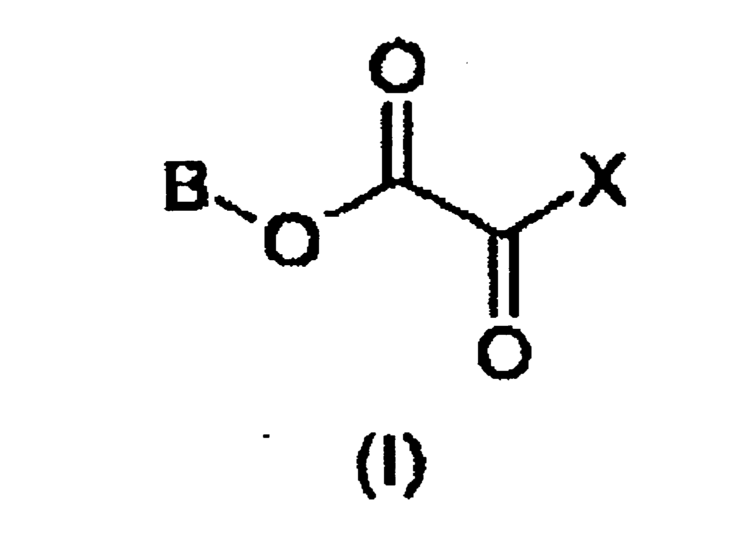 wherein in each case the following applies:
B denotes a hydrocarbon radical selected from the following group: 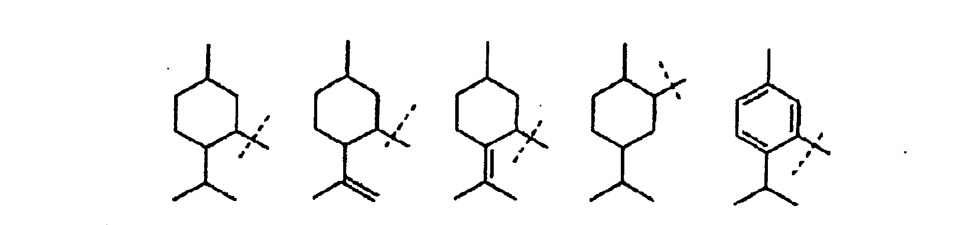 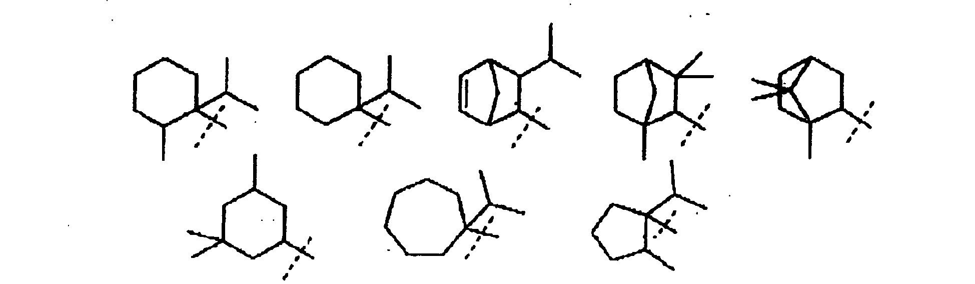 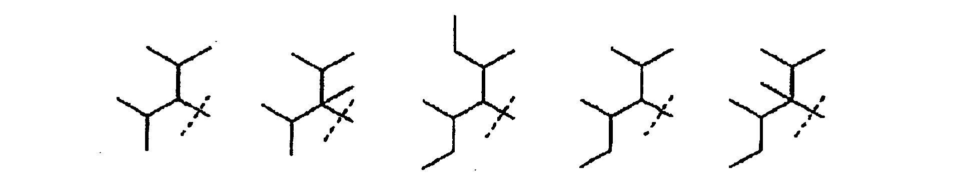 wherein the dashed line indicates the bond which binds the hydrocarbon radical B to the adjacent oxygen atom in formula (I),
X denotes NR¹R² or SR³,
wherein in each case independently of one another
in NR¹R² the radicals R¹ and R² are selected and
in SR³ the radical R³ is selected
from the group consisting of:
hydrogen
and
an organic radical having 1 to 12 C atoms which
is selected from the group consisting of substituted or non-substituted alkyl, heteroalkyl, cycloalkyl, heterocycloalkyl, cycloalkylalkyl, alkenyl, cycloalkenyl, cycloalkenylalkyl, alkinyl, cycloalkylalkinyl, aryl, heteroaryl, arylalkyl, cycloalkylaryl, cycloalkenylaryl, cycloalkylheteroaryl, heterocycloalkylaryl, heterocycloalkenylaryl, heterocycloalkenylheteroaryl and heteroarylalkyl,
wherein optionally in NR¹R² the radicals R¹ and R² by dropping a hydrogen atom in each case and forming a ring are covalently joined to one another for use as a pharmaceutical.

12. (a) Compound of formula (I) or (b) mixture comprising one, two or more compounds of formula (I) or (c) mixture consisting of two or more compounds of formula (I) according to Claim 11 for combating or relieving cough, cold, inflammation, sore throat or hoarseness symptoms.

13. Non-therapeutic method for obtaining a physiological cooling effect on the skin and/or on a mucuous membrane, having the following step:
- Applying an amount, sufficient to obtain a physiological cooling effect,
(1) of a compound or a mixture according to any one of preceding Claims 1 to 6 or
(2) of a mixture according to either of Claims 7 and 8 or
(3) of a preparation according to Claim 9
to the skin and/or to a mucuous membrane.

14. Method for producing a compound according to any one of Claims 1 to 6, comprising the steps:
(1) Providing a mixed oxalic acid ester of the formula 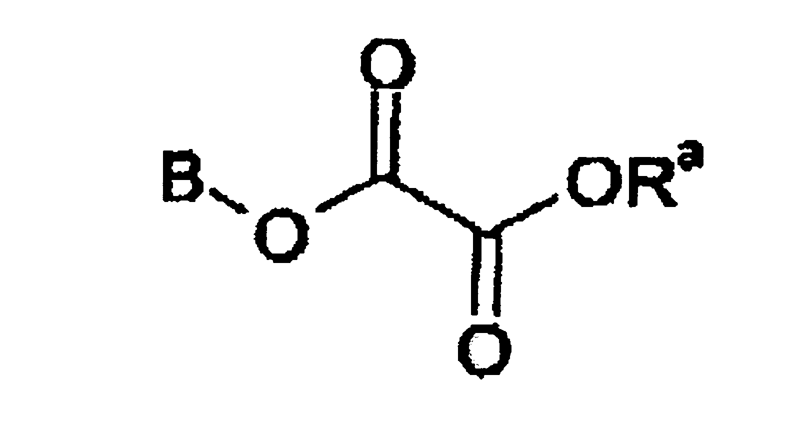 wherein R^{a} denotes an alkyl radical, preferably a C₁-C₄-alkyl radical, or an oxalic acid halide of the formula 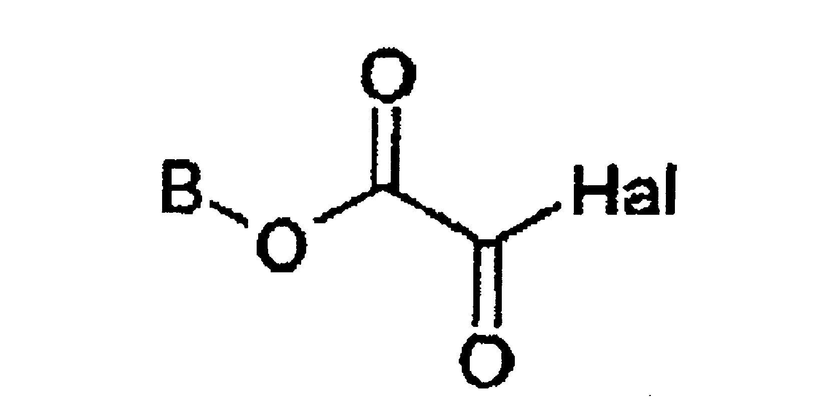 wherein Hal denotes halide,
(2) Providing an amine HNR¹R² or a thiol HSR³ or a corresponding salt,
(3) Reacting the compounds provided with one another, so that a compound according to any one of Claims 1 to 6 is formed.

## Revendications

1. (a) Composé de formule (I) ou (b) mélange comprenant un, deux, ou plusieurs composés de formule (I) ou (c) mélange consistant en deux composés ou plus de formule (I) 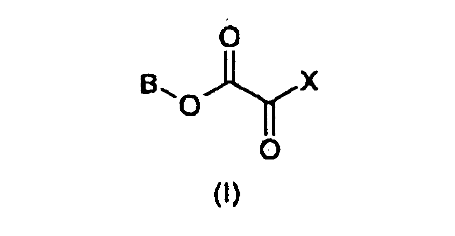 dans lequel, respectivement :
B représente un radical hydrocarbure choisi dans le groupe suivant : 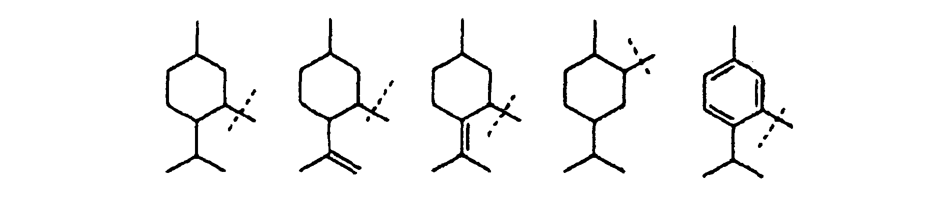 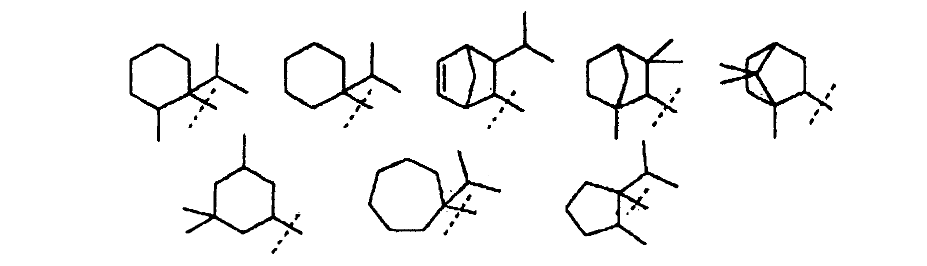 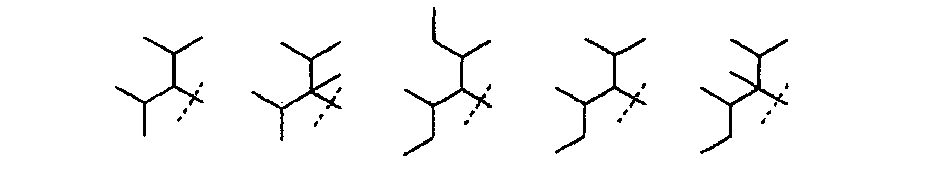 la ligne pointillée marquant la liaison qui relie le radical hydrocarbure B à l'atome d'oxygène voisin du composé de formule (I),
X représente NR¹R² ou SR³,
dans lequel respectivement, indépendamment les uns des autres,
dans NR¹R², les radicaux R¹ et R² sont choisis, et dans SR³, le radical R³ est choisi
dans le groupe comprenant :
l'hydrogène,
et
un radical organique comportant
1 à 12 atomes de carbone
dans lequel éventuellement, dans NR¹R², les radicaux R¹ et R², quand ils ne sont pas l'hydrogène, sont liés de façon covalente pour former un cycle,
à condition que, dans le cas ci-dessus (a) le composé de formule (I), et dans le cas ci-dessus (b) au moins un composé de formule (I), ne soit pas choisi dans le groupe comprenant : 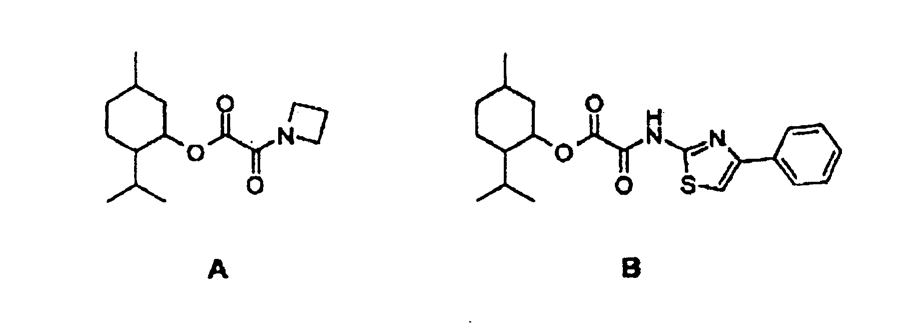 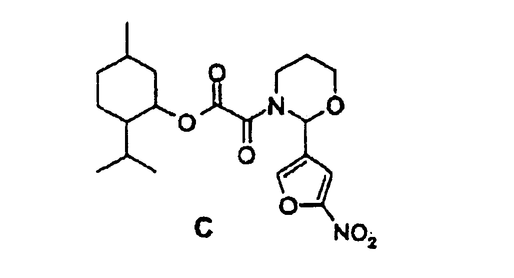
et la D(+)-menthyloxamide.

2. (a) Composé de formule (I) ou (b) mélange comprenant un, deux composés ou plus de formules (I) ou (c) mélange consistant en deux composés ou plus de formule (I) selon la revendication 1, dans lequel, dans le cas (a), le composé, ou, dans le cas (b) ou (c), au moins l'un des composés du mélange, est choisi dans le groupe comprenant des composés de formule (I), dans lequel :
X représente NR¹R² ou SR³,
dans lequel respectivement, indépendamment les uns des autres,
dans NR¹R², les radicaux R¹ et R² sont choisis, et
dans SR³, le radical R³ est choisi
dans le groupe comprenant :
l'hydrogène
et
un radical organique comportant 1 à 12 atomes de carbone et éventuellement jusqu'à 3 hétéroatomes qui sont choisis respectivement, indépendamment les uns des autres, dans le groupe comprenant N, S et O, dans lequel les hétéroatomes éventuellement présents du groupe constitué de O et S ne possèdent pas de liaison covalente avec d'autres hétéroatomes de ce groupe,
dans lequel éventuellement, un nombre d'atomes d'hydrogène liés à C peut être remplacé par un atome de fluor,
dans lequel le radical organique comprend respectivement de façon inclusive tous les substituants éventuellement présents, ne contient pas plus d'hétéroatomes du groupe comprenant N, S et O que les atomes de carbone et est choisi dans le groupe comprenant les groupes substitués ou non substitués :
alkyle en C₁ à C₁₀, hétéroalkyle en C₁ à C₁₀, hétérocycloalkyle en C₂ à C₁₀, cycloalkyle en C₃ à C₁₀, cycloalkylalkyle en C₄ à C₁₀, alcényle en C₂ à C₁₀, cycloalcényle en C₃ à C₁₀, cycloalcénylalkyle en C₉ à C₁₀, alcinyle en C₂ à C₁₀, cycloalkylalcinyle en C₅ à C₁₀, aryle en C₃ à C₁₀, hétéroaryle en C₂ à C₁₀, arylalkyle en C₄ à C₁₀, cycloalkylaryle en C₈ à C₁₀, cycloalcénylaryle en C₈ à C₁₀, cycloalkylhétéroaryle en C₅ à C₁₀, hétérocycloalkylaryle en C₈ à C₁₀, hétérocycloalcénylaryle en C₈ à C₁₀, hétérocycloalcénylhétéroaryle en C₈ à C₁₀ et hétéroarylalkyle en C₃ à C₁₀,
et de préférence, choisi dans le groupe comprenant les groupes substitués ou non substitués : alkyle en C₁ à C₈, hétéroalkyle en C₂ à C₈, hétérocycloalkyle en C₁ à C₈, cycloalkyle en C₃ à C₈, cycloalkylalkyle en C₄ à C₈, alcényle en C₂ à C₈, cycloalcényle en C₃ à C₈, cycloalcénylalkyle en C₉ à C₈, aryle en C₃ à C₆, hétéroaryle en C₂ à C₆, arylalkyle en C₉ à C₈,
dans lequel éventuellement, dans NR¹R², les radicaux R¹ et R², quand ils ne sont pas l'hydrogène, sont liés de façon covalente pour former un cycle.

3. (a) Composé de formule (I) ou (b) mélange comprenant un, deux, ou plusieurs composés de formule (I) ou (c) mélange consistant en deux composés ou plus de formule (I) selon l'une quelconque des revendications précédentes, dans lequel, dans le cas (a), le composé ou dans le cas (b) ou (c) au moins l'un des composés du mélange est choisi dans le groupe comprenant des composés de formule (I), dans lequel :
X représente NHR¹,
dans lequel R¹ est choisi dans le groupe comprenant
un radical organique comportant 1 à 12 atomes de carbone et éventuellement jusqu'à 3 hétéroatomes qui sont choisis respectivement, indépendamment les uns des autres, dans le groupe comprenant N, S et O, dans lequel les hétéroatomes éventuellement présents du groupe constitué de O et S ne possèdent pas de liaison covalente avec d'autres hétéroatomes de ce groupe,
dans lequel éventuellement, un nombre d'atomes d'hydrogène liés à C peut être remplacé par un atome de fluor,
dans lequel le radical organique comprend respectivement tous les substituants éventuellement présents, ne contient pas plus d'hétéroatomes du groupe comprenant N, S et 0 que d'atomes de carbone et est choisi dans le groupe comprenant les groupes substitués ou non substitués :
alkyle en C₁ à C₁₀, hétéroalkyle en C₁ à C₁₀,
hétérocycloalkyle en C₂ à C₁₀, cycloalkyle en C₃ à C₁₀, cycloalkylalkyle en C₄ à C₁₀, alcényle en C₂ à C₁₀, cycloalcényle en C₃ à C₁₀, cycloalcénylalkyle en C₄ à C₁₀, alcinyle en C₂ à C₁₀, cycloalkylalcinyle en C₅ à C₁₀, aryle en C₃ à C₁₀, hétéroaryle en C₂ à C₁₀, arylalkyle en C₉ à C₁₀, cycloalkylaryle en C₈ à C₁₀, cycloalcénylaryle en C₈ à C₁₀, cycloalkylhétéroaryle en C₅ à C₁₀, hétérocycloalkylaryle en C₈ à C₁₀, hétérocycloalcénylaryle en C₈ à C₁₀, hétérocycloalcénylhétéroaryle en C₈ à C₁₀ et hétéroarylalkyle en C₃ à C₁₀,
et de préférence, choisi dans le groupe comprenant les groupes substitués ou non substitués : alkyle en C₁ à C₈, hétéroalkyle en C₂ à C₈, hétérocycloalkyle en C₁ à C₈, cycloalkyle en C₃ à C₈, cycloalkylalkyle en C₉ à C₈, alcényle en C₂ à C₈, cycloalcényle en C₃ à C₈, cycloalcénylalkyle en C₄ à C₈, aryle en C₃ à C₆, hétéroaryle en C₂ à C₆, arylalkyle en C₄ à C₈.

4. (a) Composé de formule (I) ou (b) mélange comprenant un, deux composés ou plus de formule (I) ou (c) mélange consistant en deux composés ou plus de formule (I) selon l'une quelconque des revendications précédentes, dans lequel, dans le cas (a), le composé ou dans le cas (b) ou (c) au moins l'un des composés du mélange est choisi dans le groupe comprenant des composés de formule (I), dans lequel :
B représente un radical hydrocarbure choisi dans le groupe comprenant :

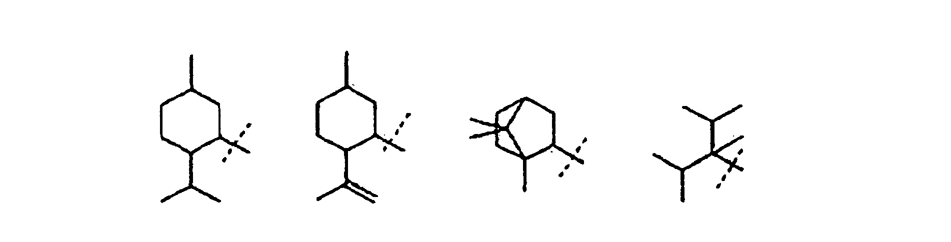

5. (a) Composé de formule (I) ou (b) mélange comprenant un, deux, ou plusieurs composés de formule (I) ou (c) mélange consistant en deux composés ou plus de formule (I) selon l'une quelconque des revendications précédentes, dans lequel, dans le cas (a), le composé ou dans le cas (b) ou (c) au moins l'un des composés du mélange est choisi dans le groupe comprenant des composés de formule (I), dans lequel :
B représente un radical hydrocarbure choisi dans le groupe comprenant :
un groupe L-menthyle, D-menthyle ou rac-menthyle,
X représente NR¹R²,
dans lequel respectivement, indépendamment les uns des autres,
les radicaux R¹ et R² sont choisis dans le groupe comprenant :
l'hydrogène
et
un radical organique comportant 1 à 12 atomes de carbone et éventuellement jusqu'à 3 hétéroatomes qui sont choisis respectivement, indépendamment les uns des autres, dans le groupe comprenant N, S et O, dans lequel les hétéroatomes éventuellement présents du groupe constitué de O et S ne possèdent pas de liaison covalente avec d'autres hétéroatomes de ce groupe,
dans lequel éventuellement, un nombre d'atomes d'hydrogène liés à C peut être remplacé par un atome de fluor,
dans lequel le radical organique comprend respectivement tous les substituants éventuellement présents, ne contient pas plus d'hétéroatomes du groupe comprenant N, S et O que d'atomes de carbone et est choisi dans le groupe comprenant les groupes substitués ou non substitués : alkyle en C₁ à C₄, hétéroalkyle en C₂ à C₅, cycloalkyle en C₃ à C₆, aryle en C₃ à C₆, hétéroaryle en C₂ à C₆, arylalkyle en C₉ à C₈,
dans lequel dans NR¹R², éventuellement les radicaux R¹ et R², quand ils ne sont pas l'hydrogène, sont liés de façon covalente pour former un cycle, dans lequel le cycle ainsi formé est au maximum à 7 chaînons,
dans lequel les substituants éventuellement présents des radicaux R¹, R² et R³ éventuellement présents sont choisis, indépendamment les uns des autres, dans le groupe comprenant les groupes :
méthyle, éthyle, n-propyle, iso-propyle, n-butyle, iso-butyle, tert-butyle, cyclopropyle, cyclopentyle, cyclohexyle, cyclooctyle, éthényle, propényle, éthinyle, propinyle, trifluorométhyle, méthoxy, éthoxy, iso-propoxy, n-butoxy, iso-butoxy, tert-butoxy, cycloalcoxy en C₃, cycloalcoxy en C₅, cycloalcoxy en C₆, cycloalcoxy en C₈, -[-O-CH₂-CH₂-]ᵥ-Q ou -[-O-CH₂-CHMe-]ᵥ-Q, dans lequel Q = OH ou CH₃ et dans lequel v peut être 1 ou 2, acétyle, CO₂Me, CO₂Et, CO₂iso-Pr, CO₂tert.-Bu, acétyloxy.

6. (a) Composé de formule (I) ou (b) mélange comprenant un, deux, ou plusieurs composés de formule (I) ou (c) mélange consistant en deux composés ou plus de formule (I) selon l'une quelconque des revendications précédentes, dans lequel, dans le cas (a), le composé ou dans le cas (b) ou (c) au moins l'un des composés du mélange est choisi dans le groupe comprenant :
le menthyloxamate
le menthyl-N-méthyloxamate
le menthyl-N,N-diméthyloxamate
le menthyl-N-éthyloxamate
le menthyl-N,N-diéthyloxamate
le menthyl-N-propyloxamate
le menthyl-N,N-dipropyloxamate
le menthyl-N-isopropyloxamate
le menthyl-N,N-diisopropyloxamate
le menthyl-N-cyclopropyloxamate
le menthyl-N-butyloxamate
l'acide morpholin-4-yloxo-acétique (1R, 2S, 5R)-2-isopropyl-5-méthyl-cyclohexylester
le menthyl-N-(2-méthoxyéthyl)-oxamate
le menthyl-N-(3-méthoxypropyl)-oxamate
le menthyl-N-(2-hydroxyéthyl)-oxamate
le menthyl-N-(3-hydroxypropyl)-oxamate.

7. Mélange (b) selon l'une quelconque des revendications précédentes, consistant en ou comprenant
(1) un, deux, ou plusieurs composés de formule (I) et
(2) une ou plusieurs autres substances ayant un effet refroidissant physiologique, dans lequel l'autre substance ou une, plusieurs ou toutes les autres substances (i) entraînent un effet gustatif ou (ii) n'entraînent pas d'effet gustatif,
et/ou
(3) une ou plusieurs substances aromatiques sans effet refroidissant physiologique
et/ou
(4) une ou plusieurs substances actives efficaces sur le plant trigéminé ou salivaire sans effet refroidissant physiologique.

8. Mélange selon la revendication 7, comprenant dans le composant (2), une ou plusieurs autres substances ayant un effet refroidissant physiologique sans effet gustatif
et/ou
dans le composant (3) une ou plusieurs substances aromatiques sans effet refroidissant physiologique
et/ou
un ou plusieurs composés qui entraîne(nt), indépendamment les uns des autres ou conjointement, en outre, un effet modulateur gustatif et/ou une excitation trigéminée et/ou salivaire.

9. Préparation servant à l'alimentation, à l'hygiène bucco-dentaire ou élément gustatif ou préparation pharmaceutique ou cosmétique comprenant une quantité suffisante pour atteindre un effet refroidissant physiologique sur la peau et/ou les muqueuses,
(i) d'un composé ou d'un mélange selon l'une quelconque des revendications 1 à 6 ou
(ii) d'un mélange selon l'une quelconque des revendications 7 à 8.

10. Utilisation
de (a) un composé de formule (1) ou (b) un mélange comprenant un, deux, ou plusieurs composés de formule (I) ou (c) un mélange consistant en deux composés ou plus de formule (I) 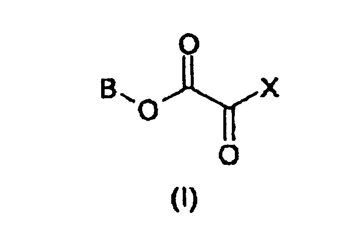 dans lequel respectivement :
B représente un radical hydrocarbure choisi dans le groupe suivant : 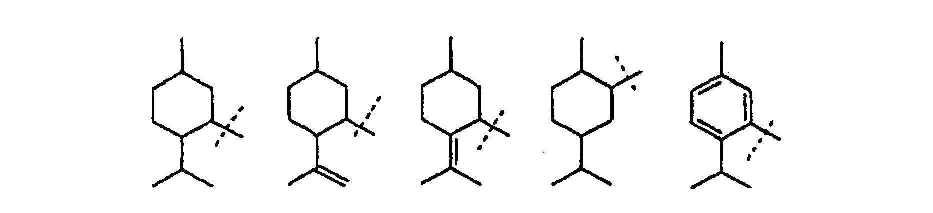 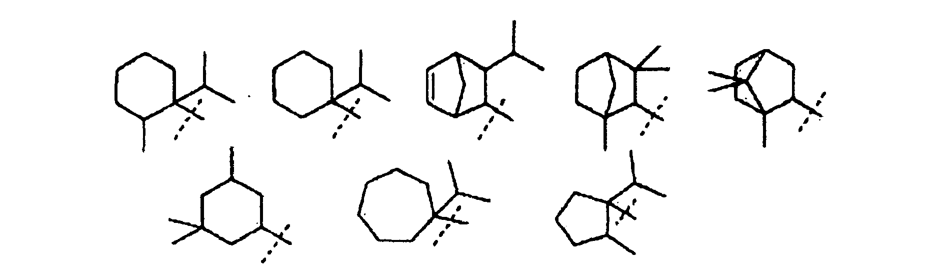 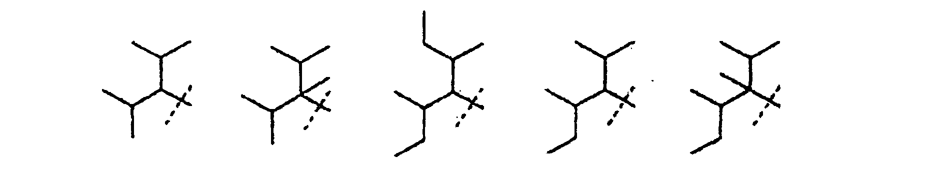 la ligne pointillée marquant la liaison qui relie le radical hydrocarbure B à l'atome d'oxygène voisin du composé de formule (I),
X représente NR¹R² ou SR³,
dans lequel respectivement, indépendamment les uns des autres,
dans NR¹R², les radicaux R¹ et R² sont choisis, et dans SR³, le radical R³ est choisi
dans le groupe comprenant :
l'hydrogène,
et
un radical organique comportant 1 à 12 atomes de C
qui est choisi dans le groupe comprenant les groupes substitués ou non substitués :
alkyle, hétéroalkyle, cycloalkyle, hétérocycloalkyle, cycloalkylalkyle, alcényle, cycloalcényle, cycloalcénylalkyle, alcinyle, cycloalkylalcinyle, aryle, hétéroaryle, arylalkyle, cycloalkylaryle, cycloalcénylaryle, cycloalkylhétéroaryle, hétéocycloalkylaryle, hétérocycloalcénylaryle, hétérocycloalcénylhétéroaryle et hétéroarylalkyle,
dans lequel éventuellement, dans NR¹R², les radicaux R¹ et R², quand ils ne sont pas l'hydrogène, sont liés de façon covalente pour former un cycle,
pour produire un effet refroidissant sur la peau ou les muqueuses à des fins autres que thérapeutiques.

11. (a) Composé de formule (I) ou (b) mélange comprenant un, deux, ou plusieurs composés de formule (I) ou (c) mélange consistant en deux composés ou plus de formule (I) 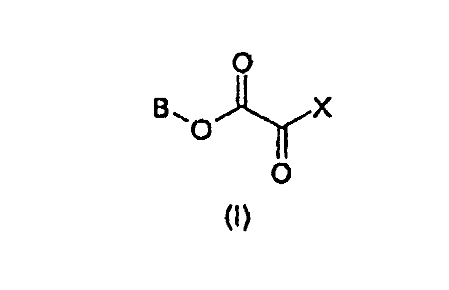 dans lequel respectivement :
B représente un radical hydrocarbure choisi dans le groupe suivant : 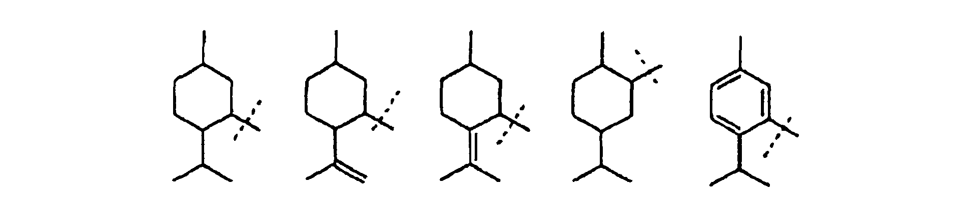 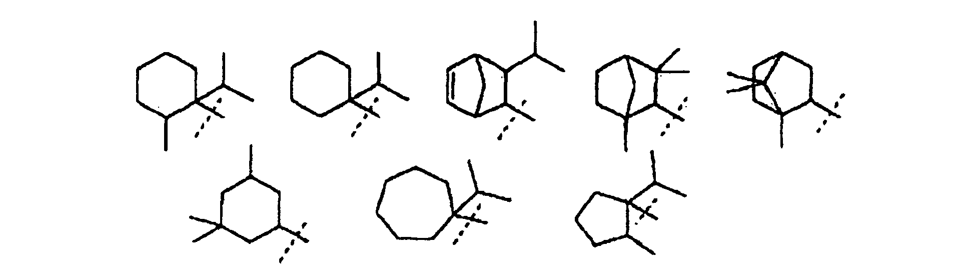 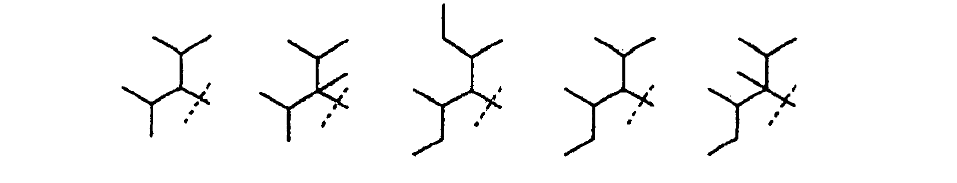 la ligne pointillée marquant la liaison qui relie le radical hydrocarbure B à l'atome d'oxygène voisin du composé de formule (I),
X représente NR¹R² ou SR³,
dans lequel respectivement, indépendamment les uns des autres,
dans NR¹R², les radicaux R¹ et R² sont choisis, et dans SR³, le radical R³ est choisi
dans le groupe comprenant :
l'hydrogène,
et
un radical organique comportant 1 à 12 atomes de carbone
qui est choisi dans le groupe comprenant les groupes substitués ou non substitués :
alkyle, hétéroalkyle, cycloalkyle, hétérocycloalkyle, cycloalkylalkyle, alcényle, cycloalcényle, cycloalcénylalkyle, alcinyle, cycloalkylalcinyle, aryle, hétéroaryle, arylalkyle, cycloalkylaryle, cycloalcénylaryle, cycloalkylhétéroaryle, hétéocycloalkylaryle, hétérocycloalcénylaryle, hétérocycloalcénylhétéroaryle et hétéroarylalkyle,
dans lequel éventuellement, dans NR¹R² les radicaux R¹ et R², quand ils ne sont pas l' hydrogène, sont liés de façon covalente pour former un cycle,
pour son utilisation en tant que médicament.

12. (a) Composé de formule (I) ou (b) mélange comprenant deux composés ou plus de formule (I) ou (c) mélange consistant en deux composés ou plus de formule (I) selon la revendication 11 pour lutter contre ou atténuer les symptômes de la toux, du rhume, de l'inflammation, des maux de gorge ou de l'enrouement.

13. Procédé non thérapeutique pour obtenir un effet refroidissant physiologique sur la peau et/ou les muqueuses, comprenant l'étape suivante :
- application d'une quantité suffisante pour obtenir un effet refroidissant physiologique
(1) d'un composé ou d'un mélange selon l'une quelconque des revendications 1 à 6 précédentes ou
(2) d'un mélange selon l'une quelconque des revendications 7 ou 8
(3) d'une préparation selon la revendication 9 sur la peau et/ou les muqueuses.

14. Procédé de production d'un composé selon l'une quelconque des revendications 1 à 6, comprenant les étapes consistant à :
(1) mettre à disposition un ester d'acide oxalique mélangé de formule 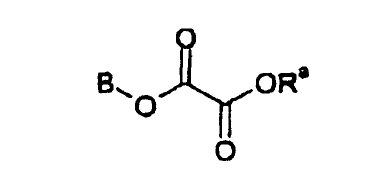 dans lequel R^{a} représente un radical alkyle, de préférence, un radical alkyle en C₁ à C₄,
ou un halogénure d'acide oxalique de formule 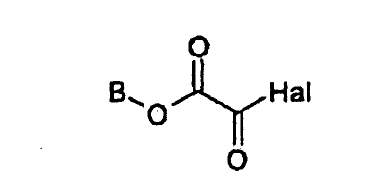 dans lequel Hal représente un halogénure,
(2) mettre à disposition d'une amine HNR¹R² ou d'un thiol HSR³ ou d'un sel correspondant,
(3) convertir les composés mis à disposition conjointement, de sorte qu'il se forme un composé selon l'une quelconque des revendications 1 à 6.
